(19) Europäisches Patentamt
European Patent Office
Office européen des brevets

(11) **EP 3 563 867 A1**

(12) **EUROPEAN PATENT APPLICATION**
published in accordance with Art. 153(4) EPC

(43) Date of publication:
06.11.2019  Bulletin 2019/45

(51) Int Cl.:
*A61K 39/395* *(2006.01)*      *A61P 37/00* *(2006.01)*

(21) Application number: 17885720.7

(86) International application number:
**PCT/RU2017/050133**

(22) Date of filing: **29.12.2017**

(87) International publication number:
**WO 2018/124948 (05.07.2018 Gazette 2018/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(30) Priority:  **30.12.2016  RU 2016152691**

(71) Applicant: **Joint Stock Company "Biocad"
St.Petersburg 198515 (RU)**

(72) Inventors:
• **LOMKOVA, Ekaterina Aleksandrovna
St.Petersburg 196066 (RU)**
• **IAKOVLEV, Aleksandr Olegovich
Moscow 109651 (RU)**
• **SHITIKOVA, Viktoriya Olegovna
Tver
Tverskaya obl. 170021 (RU)**
• **RYAKHOVSKAYA, Anastasiya Mikhajlovna
Kashinskij r-n
Tverskaya obl. 171640 (RU)**

(74) Representative: **De Anna, Pier Luigi
DEANNA-PATENT
c/o Meissner Bolte
Widenmayerstrasse 47
80538 München (DE)**

(54) **AQUEOUS PHARMACEUTICAL COMPOSITION OF A RECOMBINANT MONOCLONAL ANTIBODY TO TNF ALFA**

(57)    The invention relates to improved aqueous pharmaceutical compositions of a recombinant monoclonal antibody to TNFa, and to a method for producing same. The present invention also relates to the use of improved aqueous pharmaceutical compositions of a recombinant monoclonal antibody to TNFa to treat TNFa-mediated diseases. The proposed invention allows prevention of physical-chemical instability expressed in the formation of aggregates and fragments of proteins or in the modification of proteins in a solution, and also prevents instability during freezing/thawing, agitating and shaking.

Fig. 1

**Description**

Technical field of the invention

The invention refers to medical pharmacology and relates particularly to an aqueous pharmaceutical composition of human antibodies that specifically bind or neutralize TNFα, and using thereof.

Background of the invention

The tumour necrosis factor alpha (TNFα) is a naturally occurring mammalian cytokine produced by various types of cells, including monocytes and macrophages, in response to endotoxin or other stimuli. TNFα is a major mediator of inflammatory, immunological, and pathophysiological reactions (Grell, M., et al. (1995) Cell, 83: 793-802).

Soluble TNFα is formed by the cleavage of a precursor transmembrane protein (Kriegler, et al. (1988) Cell 53: 45-53), and secreted polypeptides of 17 kilodaltons (kDa) aggregate to form soluble homotrimeric complexes (Smith, et al. (1987), J. Biol. Chem. 262: 6951-6954; Butler, et al. (1986), Nature 320:584; Old (1986), Science 230: 630). These complexes then bind to receptors found on a variety of cells. Binding produces a number of pro-inflammatory effects, including (i) the release of other pro-inflammatory cytokines such as interleukin (IL) -6, IL-8, and IL-1, (ii) release of matrix metalloproteinases, and (iii) up-regulation of endothelial adhesion molecule expression, further amplifying inflammatory and immune cascades by attracting leukocytes into extravascular tissues.

There are many disorders associated with increased TNFα levels. For example, TNFα expression has been shown to be upregulated in a number of human diseases, including chronic diseases such as rheumatoid arthritis (RA), inflammatory bowel disorders including Crohn's disease and ulcerative colitis, sepsis, congestive heart failure, asthma bronchiale, and multiple sclerosis. TNFα is also referred to as a pro-inflammatory cytokine.

Physiologically, TNFα is also associated with protection from particular infections (Cerami. et al. (1988), Immunol. Today 9:28). TNFα is released by macrophages that have been activated by lipopolysaccharides of Gram-negative bacteria. As such, TNFα appears to be a very important endogenous mediator involved in the development and pathogenesis of endotoxic shock associated with bacterial sepsis.

Adalimumab (Humira®, AbbVie, Inc.) is a human recombinant monoclonal IgG1 antibody specific for human TNF. This antibody is also known as D2E7. Adalimumab consists of 1,330 amino acids, is described and patented in US 6090382 (WO9729131), the description of adalimumab is hereby incorporated by reference in its entirety. Adalimumab is typically produced by recombinant DNA technology in a mammalian cell expression system, such as *e.g.,* Chinese hamster cells. Adalimumab binds specifically to TNF and neutralizes TNF biological functions by blocking its interaction with TNF receptors p55 and p75 on a cell surface.

Multiple adalimumab formulations are known in the art (see *e.g.,* WO2004016286 and WO2012065072).

WO2004016286 describes a liquid composition for the stabilization of antibodies used to treat TNFα mediated diseases comprising an antibody, such as adalimumab, a buffer system, polysorbate and solution isotonicity agents, *e.g.* mannitol and sodium chloride. The composition uses a citrate-phosphate buffer (Humira formulation 1). The above formulation has a number of disadvantages: 1) insufficient colloid stability of the antibody in high concentrations, 2) insufficient stability under heat stress conditions, and 3) aggregation during long-term storage.

WO2012065072 describes a liquid composition for the stabilization of antibodies used to treat TNFα mediated diseases comprising an antibody, such as adalimumab, as well as mannitol, polysorbate 80 (Humira formulation 2). The above formulation has a number of disadvantages: 1) marked freezing and thawing instability, and 2) low aggregation temperature.

Thus, there is a need to provide a new medicament comprising an antibody binding TNFα, such as adalimumab.

The invention relates to stable aqueous compositions comprising adalimumab that enable long-term storage thereof, and also have a good tolerance to freeze-thawing and thermal stress.

Summary of the Invention

In one aspect, the present invention relates to an aqueous pharmaceutical composition for intravenous, subcutaneous or intramuscular administration comprising a recombinant monoclonal anti-TNFa antibody, an acetate ion based buffer agent (or buffer system), trehalose or proline, or a combination thereof or trehalose and arginine, polysorbate 20, polysorbate 80 or poloxamer 188, or a combination thereof.

In some embodiments of the composition, the recombinant monoclonal anti-TNFa antibody is adalimumab.

In some embodiments of the composition, the monoclonal antibody concentration is from 50 to 200 mg/mL.

In some embodiments of the composition, the acetate buffer solution concentration is from 1 to 100 mM.

In some embodiments, pH of the composition is from 4 to 7.

In some embodiments of the composition, the trehalose concentration is from 25 to 150 mg/mL.

In some embodiments of the composition, the proline concentration is from 5 to 40 mg/mL.

In some embodiments of the composition, the arginine concentration is from 0.05 to 1.0 mg/mL.

In some embodiments of the composition, the polysorbate 20 concentration is from 0.05 mg/mL to 10 mg/mL.

In some embodiments of the composition, the polysorbate 80 concentration is from 0.05 mg/mL to 10 mg/mL.

In some embodiments of the composition, the poloxamer 188 concentration is from 0.05 mg/mL to 10 mg/mL.

In some embodiments of the composition, the recombinant monoclonal anti-TNFa antibody is adalimumab.

In some embodiments, the composition comprises from 50 to 150 mg/mL of adalimumab, 0.436 mg/mL of sodium acetate trihydrate, 80 mg/mL of trehalose dihydrate, glacial acetic acid until pH 5.5, 0.5 mg/mL of polysorbate 80.

In some embodiments, the composition comprises from 50 to 150 mg/mL of adalimumab, 0.436 mg/mL of sodium acetate trihydrate, 80 mg/mL of trehalose dihydrate, 0.1 mg/mL of arginine hydrochloride, glacial acetic acid until pH 5.5, 0.5 mg/mL of polysorbate 80.

In some embodiments, the composition comprises from 50 to 150 mg/mL of adalimumab, 0.436 mg/mL of sodium acetate trihydrate, 27 mg/mL of proline, glacial acetic acid until pH 5.5, 0.5 mg/mL of Polysorbate 80.

In some embodiments, the composition comprises from 50 to 150 mg/mL of adalimumab, 0.436 mg/mL of sodium acetate trihydrate, 40 mg/mL of trehalose dihydrate, 13.5 mg/mL of proline, glacial acetic acid until pH 5.5, 0.5 mg/mL of polysorbate 80.

In some embodiments, the composition comprises from 50 to 200 mg/mL of the recombinant monoclonal anti-TNFa antibody, from 0.4 to 1.2 mg/mL of sodium acetate trihydrate, from 25 to 150 mg/mL of trehalose and/or from 5 to 40 mg/mL of proline or from 25 to 150 mg/mL of trehalose, and from 0.05 to 1.0 mg/mL of arginine, glacial acetic acid until pH 5.0 to 6.0, from 0.1 mg/mL to 1 mg/mL of polysorbate 80.

In some embodiments, the composition comprises from 50 to 200 mg/mL of the recombinant monoclonal anti-TNFa antibody, from 0.4 to 1.2 mg/mL of sodium acetate trihydrate, from 25 to 150 mg/mL of trehalose and/or from 5 to 40 mg/mL of proline or from 25 to 150 mg/mL of trehalose, and from 0.05 to 1.0 mg/mL of arginine, glacial acetic acid until pH 5.5, from 0.1 mg/mL to 1 mg/mL of polysorbate 20.

In some embodiments of the composition, the recombinant monoclonal anti-TNF$\alpha$ antibody is adalimumab.

In some embodiments, the composition comprises from 50 to 150 mg/mL of adalimumab, 0.436 mg/mL of sodium acetate trihydrate, 80 mg/mL of trehalose dihydrate, glacial acetic acid until pH 5.5, 0.5 mg/mL of polysorbate 20.

In some embodiments, the composition comprises from 50 to 150 mg/mL of adalimumab, 0.436 mg/mL of sodium acetate trihydrate, 80 mg/mL of trehalose dihydrate, 0.1 mg/mL of arginine hydrochloride, glacial acetic acid until pH 5.5, 0.5 mg/mL of polysorbate 20.

In some embodiments, the composition comprises from 50 to 150 mg/mL of adalimumab, 0.436 mg/mL of sodium acetate trihydrate, 27 mg/mL of proline, glacial acetic acid until pH 5.5, 0.5 mg/mL of polysorbate 20.

In some embodiments, the composition comprises from 50 to 150 mg/mL of adalimumab, 0.436 mg/mL of sodium acetate trihydrate, 40 mg/mL of trehalose dihydrate, 13.5 mg/mL of proline, glacial acetic acid until pH 5.5, 0.5 mg/mL of polysorbate 20.

In some embodiments, the composition comprises from 50 to 200 mg/mL of the recombinant monoclonal anti-TNF$\alpha$ antibody, from 0.4 to 1.2 mg/mL of sodium acetate trihydrate, from 25 to 150 mg/mL of trehalose and/or 5 to 40 mg/mL of proline or from 25 to 150 mg/mL of trehalose, and from 0.05 to 1.0 mg/mL of arginine, glacial acetic acid until pH 5.0 to 6.0, from 0.1 mg/mL to 1 mg/mL of poloxamer 188.

In some embodiments of the composition, the recombinant monoclonal anti-TNFa antibody is adalimumab.

In some embodiments, the composition comprises from 50 to 150 mg/mL of adalimumab, 0.436 mg/mL of sodium acetate trihydrate, 80 mg/mL of trehalose dihydrate, glacial acetic acid until pH 5.5, 1 mg/mL of poloxamer 188.

In some embodiments, the composition comprises from 50 to 150 mg/mL of adalimumab, 0.436 mg/mL of sodium acetate trihydrate, 80 mg/mL of trehalose dihydrate, 0.1 mg/mL of arginine hydrochloride, glacial acetic acid until pH 5.5, 1 mg/mL of poloxamer 188.

In some embodiments, the composition comprises from 50 to 150 mg/mL of adalimumab, 0.436 mg/mL of sodium acetate trihydrate, 27 mg/mL of proline, glacial acetic acid until pH 5.5, 1 mg/mL of poloxamer 188.

In some embodiments, the composition comprises from 50 to 150 mg/mL of adalimumab, 0.436 mg/mL of sodium acetate trihydrate, 40 mg/mL of trehalose dihydrate, 13.5 mg/mL of proline, glacial acetic acid until pH 5.5, 1 mg/mL of poloxamer 188.

In one aspect, the present invention relates to a method for treating TNF$\alpha$ mediated diseases comprising administering an effective amount of the compositions described above.

In some embodiments of the method for treating, a TNF$\alpha$ mediated disease is selected from active (moderate to severe) rheumatoid arthritis, active psoriatic arthritis, active ankylosing spondylitis, chronic (moderate to severe) plaque psoriasis, (moderate to severe) ulcerative colitis, axial spondylarthritis, active hidradenitis suppurativa, juvenile idiopathic arthritis, (moderate or severe) Crohn's disease, uveitis, active enthesitis-associated arthritis.

In one aspect, the present invention relates to use of the above compositions for treating TNF$\alpha$ mediated diseases.

In some embodiments of use, a disease is selected from active (moderate to severe) rheumatoid arthritis, active psoriatic arthritis, active ankylosing spondylitis, chronic (moderate to severe) plaque psoriasis, (moderate to severe) ulcerative colitis, axial spondylarthritis,

active hidradenitis suppurativa, juvenile idiopathic arthritis, (moderate or severe) Crohn's disease, uveitis, active enthesitis-associated arthritis.

In one aspect, the present invention relates to a method for producing the above compositions, the method comprising the addition of acetate buffering agents to an aqueous phase followed by the addition, in any order, of the following components: an osmolyte and/or a stabilizing agent selected from the group of trehalose, proline or a combination thereof; a recombinant monoclonal anti-TNF$\alpha$ antibody; a surface-active substance selected from the group of polysorbate 20, polysorbate 80, poloxamer 188, or a combination thereof.

Brief Description of Drawings

Fig. 1. Dependence of the optical density in solutions at 400 nm on the PEG 6000 concentration after preparation.
Fig. 2. Dependence of the optical density in solutions at 400 nm on the PEG 6000 concentration after preparation.
Fig. 3. Dependence of the optical density in solutions at 400 nm on the PEG 6000 concentration after preparation.
Fig. 4. Adalimumab aggregation point in acetate buffer solution, pH 5.0. $T_{ag}$ 74.0°C. Dark grey: Z-average, nm, light grey: intensity, kcps.
Fig. 5. Adalimumab aggregation point in citrate buffer solution, pH 5.0. $T_{ag}$ 69.5°C. Dark grey: Z-average, nm, light grey: intensity, kcps.
Fig. 6. Adalimumab aggregation point in histidine buffer solution, pH 5.0. $T_{ag}$ 69.5°C. Dark grey: Z-average, nm, light grey: intensity, kcps.
Fig. 7. Adalimumab aggregation point in phosphate buffer solution, pH 5.0. $T_{ag}$ 71.0°C. Dark grey: Z-average, nm, light grey: intensity, kcps.
Fig. 8. Adalimumab aggregation point in Humira formulation 1, pH 5.0. $T_{ag}$ 69.5°C. Dark grey: Z-average, nm, light gray: intensity, kcps.

Disclosure of the Invention

*Definitions and general methods*

The terms used in this specification generally have their ordinary meanings in the art, within the context of the invention, and in the specific context where each term is used. Below, or elsewhere herein, certain terms used to describe the invention are defined to provide a practitioner with an additional guidance for describing the invention. Synonyms for some terms are provided. A recital of one or more synonyms does not exclude the use of other synonyms. The use of examples anywhere in this specification, including examples of any term described herein, is illustrative only and is not intended to limit the scope and meaning of the invention or of any exemplified subject matter. The invention is not limited to various embodiments given in this specification.

Unless otherwise defined, all technical and scientific terms used herein have the same meaning as commonly understood by one of ordinary skill in the art to which this invention pertains. In the case of conflict, the present document, including definitions, will control.

The term "adalimumab" is synonymous with the active pharmaceutical ingredient in Humira® as well as protein considered or intended as bio-similar or bio-improved variants thereof. Adalimumab is a human recombinant monoclonal IgG1 antibody specific for human TNF. Adalimumab is also known as D2E7. Adalimumab comprises two light chains each with a molecular weight of approximately 24 kDa and two IgG1 heavy chains each with a molecular weight of approximately 49 kDa. Each light chain consists of 214 amino acid residues and each heavy chain consists of 451 amino acid residues. Thus, adalimumab consists of 1330 amino acids. The term adalimumab is also intended to include so-called bio-similar or bio-improved variants of the adalimumab protein used in commercially available Humira®. For example, a variant of commercial Humira® may be acceptable to the FDA when it has essentially the same pharmacological effect as commercially available Humira®, even though it may exhibit certain physical properties, such as a glycosylation profile and an acid-base profile, that may be similar if not identical to Humira®.

For the purposes of the present application, the term "adalimumab" also includes adalimumab with minor modifications in the amino acid structure (including deletions, additions, and/or substitutions of amino acids) or in the glycosylation properties and the acid-base profile, which do not significantly affect the function of the polypeptide. The term "adalimumab" includes all forms and formulations of Humira®, including but not limited to concentrated formulations, injectable ready-to-use formulations; formulations reconstituted with water, alcohol, and/or other ingredients, and others.

The term "human TNFα," which also known as hTNFα or hTNF, or TNFα, is intended to refer to a human cytokine that exists as a 17 kDa secreted form and a 26 kDa membrane-associated form, the biologically active form of which is composed of a trimer of noncovalently bound 17 kDa molecules. The structure of TNFα is described further in, for example, Pennica, D., et al. (1984) Nature 312:724-729; Davis, J. M., et al. (1987) Biochemistry 26:1322-1326; and Jones, E. Y., et al. (1989) Nature 338:225-228. The term human rhTNFα is intended to include recombinant human TNFα, which can be prepared by standard recombinant expression methods or purchased commercially (R & D Systems, catalog. No. 210-TA, Minneapolis, Minn.).

As used herein, the term "antibody" refers to immunoglobulin-type molecules comprised of four polypeptide chains, two heavy (H) chains and two light (L) chains interconnected by internal disulphide bonds. Each heavy chain consists of a heavy chain variable region (abbreviated herein as HCVR or VH) and a heavy chain constant region. The heavy chain constant region consists of three domains, CH1, CH2, and CH3. Each light chain consists of a light chain variable region (abbreviated herein as LCVR or VL) and a light chain constant region. The light chain constant region consists of one domain, CL. The VH and VL regions can be further subdivided into regions of hypervariability, referred to as complementarity determining regions (CDRs), interspersed with regions that are more conserved, termed framework regions (FRs). Each VH and VL consists of three CDRs and four FRs, arranged from N-terminus to C-terminus in the following order: FR1, CDR1, FR2, CDR2, FR3, CDR3, FR4. In one embodiment of the invention, the formulation comprises an antibody with CDR1, CDR2, and CDR3 sequences similar to those described in U.S. Pat. Nos. 6090382; 6258562, and 8216583.

An antibody or antigen-binding portion thereof may be part of a larger immune-adhesion molecule, formed by the covalent or noncovalent association of the antibody or antibody portion with one or more other proteins or peptides. Examples of such immune-adhesion molecules include use of the streptavidin core region to make a tetrameric scFv molecule (Kipriyanov, S. M., et al. (1995) Human Antibodies and Hybridomas 6:93-101) and use of a cysteine residue, a marker peptide and a C-terminal polyhistidine tag to make bivalent and biotinylated scFv molecules (Kipriyanov, S. M., et al. (1994) Mol. Immunol. 31:1047-1058). From whole antibodies, antibody portions, such as Fab and F(ab')$_2$ fragments of whole antibodies, can be prepared using conventional methods, such as papain or pepsin digestion, respectively. Further, antibodies, antibody portions, and immune-adhesion molecules can be obtained using standard recombinant DNA techniques, as described herein.

As used herein, the term "isolated antibody" refers to an antibody that is substantially free of other antibodies having different antigenic specificities (*e.g.*, an isolated antibody that specifically binds to TNFα is substantially free of antibodies that specifically bind to antigens other than TNFα). However, an isolated antibody that specifically binds to TNFα may have cross-reactivity to other antigens, such as TNFα molecules from other species. Furthermore, an isolated antibody may be substantially free of other cellular materials and/or chemicals.

The term "anti-TNFα antibody" refers to a human anti-TNFa antibody as described herein, as well as described in US Pat. Nos. 6090382; 6258562; 6509015; 7223394 and 6509015. The term anti-TNFα antibody used in the invention is an anti-TNFa antibody or a fragment thereof, including infliximab (Remicade®, Johnson and Johnson, described in US Patent No. 5656272); CDP571 (a humanized monoclonal anti-TNF-alpha IgG4 antibody); CDP870 (a humanized monoclonal anti-TNF-alpha antibody fragment); an anti-TNF dAb (Peptech); CNTO148 (golimumab; Centocor, see WO 02/12502 and U.S. 521,206 and U.S. 7250165); and adalimumab (HUMIRA® Abbott Laboratories, a human anti-TNF mAb, described in US 6090382 as D2E7). Additional anti-TNF antibodies that may be used in the invention are described in U.S. Patent Nos. 6593458; 6498237; 6451983; and 6448380. In another embodiment, the TNFα inhibitor is a TNF fusion protein, for example, etanercept (Enbrel®, Amgen; described in WO 91/03553 and WO 09/406476). In another embodiment, the TNFα inhibitor is a recombinant TNF binding protein (r-TBP-I) (Serono).

The term "long-term storage" or "long-term stability" is to be understood to mean that a pharmaceutical composition can be stored for three months or more, for six months or more, and preferably for one year or more, most preferably a minimum stable shelf life of at least two years. In general, the terms "long-term storage" and "long-term stability" further include stable storage durations that are at least comparable to or better than the stable shelf life typically required for currently available commercial formulations of adalimumab, without losses in stability that would render the formulation unsuitable for its intended pharmaceutical application. Long-term storage is also understood to mean that the pharmaceutical composition is stored either as a liquid at 2 to 8°C., or frozen, *e.g.,* at -18°C., or lower. It is also contemplated that the composition can be frozen and thawed more than once.

The term "stable state" with respect to long-term storage is understood to mean that adalimumab contained in the pharmaceutical compositions does not lose more than 20%, or more preferably 15%, or even more preferably 10%, and most preferably 5% of its activity relative to activity of the composition at the start of storage.

The term "excipient" or "additive" is used herein to describe any ingredient other than those previously described in the present invention.

The term "sugar" refers to monosaccharides, disaccharides and polysaccharides or mixtures thereof. Examples of sugars include, but are not limited to, sucrose, trehalose, glucose, dextrose, and others.

The term "polyol" as used herein refers to an excipient having multiple hydroxyl groups and includes sugar alcohols and sugar acids. Examples of polyols include, but are not limited to, mannitol, sorbitol, and others.

The term "buffer," "buffer composition," "buffer agent", as used herein, refers to an added composition that allows a liquid antibody formulation to resist changes in pH, typically by the action of its acid-base conjugate components. When reference is made to a concentration of a buffer, it is intended that said concentration represents the total molar concentration of the free acid or free base form of the buffer. Examples of buffers that are known in the art and can be found in the literature include, but are not limited to, histidine, citrate, succinate, acetate, phosphate, phosphate-buffered saline, citrate-phosphate buffers, and tromethamine-based buffers and the like, or suitable mixtures thereof.

The terms "tonicity agent" or "tonicifier," as well as "osmolyte" or "osmotic agent" as used herein refer to an excipient that can adjust the osmotic pressure of a liquid antibody preparation. In certain embodiments, the tonicity agent can adjust the osmotic pressure of the liquid antibody preparation to isotonic so that the antibody preparation is physiologically compatible with the cells of the body tissue of the subject. In yet another embodiment, the "tonicity agent" may contribute to an improvement in stability of the antibodies described herein. An "isotonic" preparation is a preparation that has the osmotic pressure equivalent to human blood. Isotonic preparations generally have an osmotic pressure of about 250 to 350 mOsm. The term "hypotonic" describes a preparation having an osmotic pressure below that of human blood. Accordingly, the term "hypertonic" is used to describe a preparation having an osmotic pressure above that of human blood. Isotonicity can be measured using, for example, a vapor pressure or ice-freezing type osmometer. The tonicity agent may be in an enantiomeric (*e.g.,* L- or D-enantiomer) or racemic form; in the form of isomers, such as alpha or beta, including alpha, alpha; or beta, beta; or alpha, beta; or beta, alpha; in a free acid or free base form; in a salt form; in a hydrated form (for example, monohydrate) or in an anhydrous form.

The term "surface-active substance" (also referred to as surfactant or detergent, or SAS) as used herein refers to an excipient that can alter the surface tension of a liquid antibody preparation. In certain embodiments, the surface-active substance reduces the surface tension of the liquid antibody preparation. In other embodiments, the "surface-active substance" may contribute to an improvement in colloidal stability or solubility of any antibody in the preparation. The surface-active substance may reduce aggregation of the resulting antibody preparation and/or minimize the formation of particulates in the preparation and/or reduce adsorption. The surface-active substance may also improve the stability of the antibody during and after a freeze/thaw cycle and while shaking. The surface-active substances may be ionic or non-ionic. Exemplary non-ionic surface-active substances that can be included in the formulations of the present invention include, *e.g.,* alkyl poly(ethylene oxide), alkyl polyglucosides (*e.g.,* octyl glucoside and decyl maltoside), fatty alcohols such as cetyl alcohol and oleyl alcohol, Cocamide MEA, Cocamide DEA, and cocamide TEA. Specific non-ionic surface-active substances that can be included in the formulations of the present invention include, *e.g.,* Polysorbates such as Polysorbate 20 (Tween 20), Polysorbate 28, Polysorbate 40, Polysorbate 60, Polysorbate 65, Polysorbate 80 (Tween 80), Polysorbate 81, and Polysorbate 85; Poloxamers such as Poloxamer 188 (Kolliphor P188), Poloxamer 407; polyethylene-polypropylene glycol; or polyethylene glycol (PEG), ethylene- and propylene glycol copolymers (*e.g.,* pluronics PF68, etc.).

The term "lyophilized" as used herein refers to a preparation that has undergone a process known in the art as freeze-drying, involving freezing the preparation and subsequently removing the ice from the frozen content.

The term "amino acid" as used herein denotes an amino acid (a free amino acid, *i.e.* not an amino acid in a peptide or protein sequence). Amino acids as used in the present invention comprise, but are not limited to, *e.g.* arginine, glycine, lysine, histidine, glutamic acid, aspartic acid, isoleucine, leucine, alanine, phenylalanine, tryptophan, serine, cysteine, methionine, and proline.

"Pharmaceutical composition" means a composition comprising an antibody of the invention and at least one of components selected from the group consisting of pharmaceutically acceptable and pharmacologically compatible excipients, solvents, diluents, carriers, additive, distributing agents, delivery agents, such as preservatives, stabilizing agents, emulsifiers, suspending agents, thickeners, prolonged delivery regulators, the choice and ratio of which depends on the nature and method for administering and dosing. Exemplary suspending agents include ethoxylated isostearyl alcohol, polyoxyethylene, sorbitol and sorbitol ester, microcrystalline cellulose, aluminum metahydroxide, bentonite, agar-agar and tragacanth, and mixtures thereof. Protection from the microorganism action can be provided with multiple antibacterial and antifungal agents, *e.g.,* such as parabens, chlorobutanol, sorbic acid and the like. The composition may also comprise isotonic agents, *e.g.,* sugars, sodium chloride and the like. The prolonged action of the composition may be provided with agents that delay the absorption of the active agent, *e.g.* aluminum monostearate and gelatin. Exemplary appropriate carriers, solvents, diluents and delivery agents include water, ethanol, polyalcohols, and mixtures thereof, vegetable oils (such as olive oil) and injectable organic esters (such as ethyl oleate). Exemplary excipients include lactose, milk sugar, sodium citrate, calcium carbonate, calcium phosphate and the like. A pharmaceutical composition for oral, sublingual, transdermal, intramuscular, intravenous, subcutaneous, topical or rectal administration of the active agent, alone or in combination with another active agent, can be administered to animals and humans in a unit dosage form as a mixture with conventional pharmaceutical carriers. Appropriate unit dosage forms include parenteral forms, implants and transdermal systems.

A "drug (preparation)" is a substance (or a substance mixture in a form of a pharmaceutical composition) in the form of tablets, capsules, powders, lyophilizates, injections, infusions, ointments and other finished dosage forms intended to restore, correct or alter physiological functions in humans and animals, and to provide disease treatment and prevention, diagnostics, anaesthesia, contraception, cosmetology and others.

"Treat," "treating," and "treatment" refer to a method for alleviating or eliminating a biological disorder and/or at least one of its attendant symptoms. As used herein, to "alleviate" a disease, disorder or condition means to reduce the severity and/or frequency of the disease, disorder or condition symptoms. In addition, the references to "treatment" herein include references to curative, palliative and preventive therapies.

In one aspect, the subject to be treated or the patient is a mammal, preferably a human subject. The above subject may be male or female of any age.

The term "disorder" denotes any condition that can be improved by the treatment according to the invention. The definition of this term includes chronic and acute disorders or diseases including pathological conditions, which predispose the mammal to said disorder. Non-limiting examples of the diseases to be treated include benign and malignant tumours; leukemias and lymphoid malignancies, in particular, breast, ovarian, stomach, endometrium, salivary gland, lung, kidney, colon, thyroid, pancreas, prostate or bladder cancer; neural, glial, astrocytic, hypothalamus and other glandular, macrophage, epithelial, stromal and blastocoelic disorders; inflammatory, angiogenic and immunological disorders. The preferred disorder to be treated according to the invention is autoimmune diseases.

The terms "immune response," "autoimmune response," "autoimmune inflammation" refer, *e.g.* to the action of lymphocytes, antigen-presenting cells, phagocytic cells, granulocytes, and soluble macromolecules produced by above cells or liver cells (including antibodies, cytokines, and complement that result from the selective damage, destruction or elimination from the human body of invasive pathogens, cells or tissues infected with pathogens, cancer cells or, in cases of autoimmunity or pathological inflammation, normal human cells or tissues).

The term "autoimmune disease" as used herein means non-malignant disease or disorder that occurs and is directed against the self (auto) antigens and/or tissues in an individual.

This definition encompasses but is not limited to rheumatoid arthritis, arthrosis, juvenile chronic arthritis, septic arthritis, Lyme's arthrosis, psoriatic arthritis, reactive arthritis, spondyloarthropathy, systemic lupus erythematosus, Crohn's disease, ulcerative colitis, inflammatory bowel disease, diabetes mellitus, thyroiditis, asthma, allergic diseases, psoriasis, atopic dermatitis, scleroderma, graft versus host reaction, transplant rejection, acute or chronic immune diseases associated with transplantation, sarcoidosis, Kawasaki's disease, Graves' disease, nephrotic syndrome, chronic fatigue syndrome, Wegener's granulomatosis, Henoch-Schonlein's purpura, microscopic renal vasculitis, chronic active hepatitis, uvenita, septic shock, toxic shock syndrome, septic syndrome, cachexia, acquired immunodeficiency syndrome, acute transverse myelitis, Huntington's chorea, Parkinson's disease, Alzheimer's disease, stroke, primary biliary cirrhosis, hemolytic anemia, adult (acute) respiratory distress syndrome, alopecia, focal alopecia, seronegative arthropathia, arthropathy, Reiter's disease, psoriatic arthropathy, arthropathy associated with ulcerative colitis, atopic allergies, autoimmune bullous diseases, pemphigus vulgaris, pemphigus foliaceus, pemphigoid disease, linear IgAs, autoimmune hemolytic anaemia, Coombs-positive hemolytic anaemia, malignant anaemia, juvenile malignant anaemia, arthritis, primary sclerosing hepatitis A, cryptogenic autoimmune hepatitis, pulmonar fibrosing disease, cryptogenic fibrous alveolitis, postinflammatory interstitial lung diseases, interstitial pneumonitis, chronic eosinophilic pneumonia, postinfectious interstitial lung diseases, urarthritis, autoimmune hepatitis, autoimmune hepatitis type 1 (classic autoimmune hepatitis or lipoid), autoimmune hepatitis type 2, osteoarthritis, primary sclerosing cholangitis, psoriasis type 1, psoriasis type 2, idiopathic leukopenia, autoimmune neutropenia, renal NOS-diseases, glomerulonephritis, microscopic renal vasculitis, discoid lupus erythematosus, idiopathic or male NOS-fertility, [autoimmunity to sperm], all subtypes of multiple sclerosis, sympathetic ophthalmia, secondary pulmonary hypertension associated with connective tissue disease, Goodpasture's syndrome, pulmonary manifestation of polyarteritis nodosa, acute rheumatic fever, rheumatoid spondylitis, ankylosing spondylitis, Still's disease, systemic sclerosis, Sjogren's syndrome, Takayasu's disease, autoimmune thrombocytopenia, essential thrombocythemia, autoimmune thyroiditis, hyperthyroidism, Hashimoto's disease, autoimmune atrophic hypothyroidism, primary myxedema, phacogenic uveitis, primary vasculitis, vitiligo, acute liver diseases, chronic liver diseases, allergies, asthma, mental disorders (including depressive syndrome and schizophrenia), Th2-type and Th1-type mediated diseases, conjunctivitis, allergic contact dermatitis, allergic rhinitis, alpha-I antitrypsin deficiency, amyotrophic lateral sclerosis, anaemia, cystic fibrosis, diseases associated with cytokine therapy, demielinising diseases, dermatitis, iridocyclitis/uveitis/ophthalmic neuritis, ischemia reperfusion injury, ischemic stroke, juvenile rheumatoid arthritis, autoimmune enteropathy, autoimmune hearing loss, autoimmune lymphoproliferative syndrome, autoimmune myocarditis, autoimmune premature ovarian failure and blepharitis. The antibody can also treat any combination of the above-listed disorders.

As used herein, the term "disorder in which the TNFα activity is detrimental" includes diseases and other disorders in which the presence of TNFα in a subject suffering from the disorder has been shown to be or is suspected of being either responsible for the pathophysiology of the disorder or a factor that contributes to a worsening of the disorder. Accordingly, a disorder in which the TNFα activity is detrimental is a disorder in which inhibition of TNFα activity is expected to alleviate the symptoms and/or progression of the disorder. Such disorders may be detected, for example, by an increase in the concentration of TNFα in the biological fluid of a subject suffering from the disorder (*e.g.,* an increase in the concentration of TNFα in serum, plasma, synovial fluid and the like of the subject) that can be detected, for instance, using an anti-TNFa antibody as described above. There are many examples of disorders in which the TNFα activity is detrimental. The use of antibodies and antibody fragments appropriate to the disorder in the treatment of specific disorders is further discussed below:

A. Sepsis

Tumour necrosis factor plays an established role in the pathophysiology of sepsis, with biological effects that include hypotension, myocardial suppression, vascular leakage syndrome, organ necrosis, stimulation of the release of toxic secondary mediators and activation of the clotting cascade (see e.g., Moeller A., et al.(1990), Cytokine 2:162-169; U.S. Patent No. 5231024 Moeller et at.; European Patent No. 260610 B1 Moeller A.; Tracey K.J. and Cerami A. (1994) Annu. Rev. Med. 45:491-503; Russel D. and Thompson R.C. (1993) Curr. Opin. Biotech. 4:714-721). Correspondingly, the human antibodies and antibody fragments according to the invention can be used to treat sepsis in any of its clinical manifestations, including septic shock, endotoxic shock, sepsis caused by Gram-negative bacteria, and toxic shock syndrome.

Furthermore, for sepsis treatment, the anti-TNFα antibodies and antibody fragments according to the invention can be co-administered with one or more therapeutic agents that may further alleviate sepsis, such as an interleukin-1 inhibitor (described in PCT Publication Nos. WO 92/16221 and WO 92/17583), cytokine interleukin-6 (see e.g., PCT Publication No. WO 93/11793) or a platelet activating factor antagonist (see e.g., European Patent Application No. EP 374 510). Other methods of combination therapy for sepsis treatment are discussed below in subsection III.

Additionally, in a preferred embodiment, anti-TNFa antibody and antibody fragments according to the invention is administered to a human from a subgroup of sepsis patients having a serum or plasma concentration of IL-6 above 500 pg/mL and more preferably 1000 pg/mL during treatment (see PCT Publication No. WO 95/20978 Daum, L., et al.).

B. Autoimmune Diseases

Tumour necrosis factor has been shown to play a role in the pathophysiology of a variety of autoimmune diseases. For example, TNFα has also been involved in activating tissue inflammation and caused joint destruction in rheumatoid arthritis (see e.g., Moeller A. et al.(1990), Cytokine 2:162-169; US Patent No. 5231024 Moeller et al.; European Patent No. 260610 B1 Moeller A.; Tracey K.J. and Cerami A., supra; end W.P. and Dayer J.M. (1995) ath. Rheum. 38:151-160; Fava R.A. et al. (1993) Clin. Exp. Immunol. 94:261-266). TNFα has also been involved in promoting the death of islet cells and in the formation of insulin resistance in diabetes (see e.g., Tracey and Cerami, supra; PCT publication No. WO 94/08609). TNFα has also been involved in the formation of cytotoxicity to oligodendrocytes and in the induction of inflammatory plaques in multiple sclerosis (see e.g., Tracey and Cerami, supra). Chimeric and humanized murine anti-hTNFa antibodies have been clinically tested for the treatment of rheumatoid arthritis (see e.g., Elliot M.J. et al. (1994); Lancet 344:1125-1127; Elliot M.J. et al. (1994) Lancet 344:1105-1110; Rankin E.C. et al. (1995) Br. J. Rheumatol. 34:334-342).

Human antibodies and antibody fragments according to the invention can be used to treat autoimmune diseases, in particular, those associated with inflammation, including rheumatoid arthritis, rheumatoid spondylitis, osteoarthritis and gouty arthritis, allergy, multiple sclerosis, autoimmune diabetes, autoimmune uveitis and renal syndrome. Typically, the antibody or antibody fragment is administered systemically, although, for certain disorders, local administration of the antibody or antibody fragment at a site of inflammation may be beneficial (e.g., local administration into the joints in rheumatoid arthritis or topical application to diabetic ulcers, alone or in combination with a cyclohexane-ylidene derivative as described in PCT Publication No. WO 93/19751). The antibody and antibody fragment according to the invention can also be used with one or more additional therapeutic agents used in the treatment of autoimmune diseases, as further described in subsection III.

C. Infectious Diseases

Tumour necrosis factor has been involved in the production of biological effects observed in a variety of infectious diseases. For example, TNFα has been involved in the development of brain inflammation and capillary thrombosis and infarction in malaria. TNFα has also been involved in the development of brain inflammation, the induction blood-brain barrier breakdown, the development of septic shock syndrome, and the activation of venous infarction in meningitis. TNFα has also been involved in the induction of cachexia, stimulation of viral proliferation and formation of central nervous system injuries in acquired immune deficiency syndrome (AIDS). Correspondingly, the antibodies and antibody fragments according to the invention can be used in the treatment of infectious diseases, including bacterial meningitis (see e.g., European Patent Application No. EP 585 705), cerebral malaria, AIDS and AIDS-related complex (αC) (see e.g., European Patent Application No. EP 230 574), as well as cytomegalovirus infection secondary to transplantation (see *e.g.,* Fietze, E., et al. (1994) Transplantation 58:675-680). The antibodies and antibody fragments according to the invention can also be used to alleviate symptoms associated with infectious diseases, including fever and myalgias due to infection (such as influenza) and cachexia secondary to the infection (*e.g.,* secondary to AIDS or $\alpha$C).

## D. Transplantation

Tumour necrosis factor has been considered as a key mediator in allograft rejection and graft versus host disease (GVHD) and in the formation of an adverse reaction that was observed when the rat antibody OKT3, directed against the T cell receptor CD3 complex, is used to inhibit rejection of renal transplants (see *e.g.,* Eason J.D. et al. (1995) Transplantation 59:300-305; Suthanthiran M. and Strom T. B. (1994) New Engl. J. Med. 331:365-375). Correspondingly, the antibodies and antibody fragments according to the invention can be used to inhibit transplant rejection, including allograft and xenograft rejections, and to inhibit GVHD. Although the antibody or antibody fragment can be used alone, more preferably, they are used in combination with one or more other agents that inhibit the immune response against the allograft or inhibit GVHD. For example, in one embodiment, the antibody or antibody fragment of the invention is used in combination with one or more antibodies directed at other targets involved in the regulation of immune responses, such as the cell surface molecules CD25 (interleukin-2 receptor-$\beta$), CD11a (LFA-1), CD54 (ICAM-1), CD4, CD45, CD28/CTLA4, CD80 (B7-1) and/or CD86 (B7-2). In yet another embodiment, the antibody or antibody fragment according to the invention is used in combination with one or more general immunosuppressive agents, such as cyclosporin A or FK506.

## E. Malignancies

Tumour necrosis factor has been involved in the induction of cachexia, the stimulation of Tumour growth, the enhancement of metastatic potential and the development of cytotoxicity in malignancies. Correspondingly, the antibodies and antibody fragments according to the invention are used in the treatment of malignancies, to inhibit Tumour growth or metastasis and/or to alleviate cachexia secondary to malignancies. The antibody or antibody fragment may be administered systemically or topically to the Tumour site.

## F. Pulmonary Disorders

Tumour necrosis factor has been involved in the pathophysiology of adult respiratory distress syndrome ($\alpha$DS), including the stimulation of endothelial leukocyte activation, the direction of cytotoxicity to pneumocytes, and the induction of vascular leakage syndrome. Correspondingly, the antibodies and antibody fragments according to the invention can be used to treat various pulmonary disorders, including adult respiratory distress syndrome (see *e.g.,* PCT Publication No. WO 91/04054), shock lung, chronic pulmonary inflammatory disease, pulmonary sarcoidosis, pulmonary fibrosis, and silicosis. The antibody and antibody fragment according to the invention can be administered with one or more additional therapeutic agents used in the treatment of pulmonary disorders, as described in subsection III below.

## G. Intestinal Disorders

Tumour necrosis factor has been involved in the pathophysiology of inflammatory bowel disorders (see *e.g.,* Tracy K.J., et al. (1986) Science 234:470-474; Sun X-M., et al. (1988) J. Clin. Invest. 81:1328-1331; MacDonald T.T., et al. (1990) Clin. Exp. Immunol. 81: 301-305). Chimeric murine anti-TNF$\alpha$ antibodies have been clinically tested for the treatment of Crohn's disease (van Dullemen H.M. et al.(1995) Gastroenterology 109:129-135). The human antibody or antibody fragment according to the invention can also be used to treat intestinal disorders, such as idiopathic inflammatory bowel disease, which includes two syndromes, Crohn's disease, and ulcerative colitis. The antibody and antibody fragment according to the invention can also be administered with one or more additional therapeutic agents used in the treatment of intestinal disorders, as described in subsection III below.

## H. Cardiac Disorders

The antibodies or antibody fragments according to the invention can also be used to treat various cardiac disorders, including cardiac ischemia (see *e.g.,* European Patent Application No. EP 453 898) and heart failure (amyocardia) (see *e.g.,* PCT Publication No. WO 94/20139).

## I. Others

The antibodies or antibody fragments according to the invention can also be used to treat various disorders in which TNFα activity is detrimental. Examples of other diseases and disorders in which TNFα activity is involved in the pathophysiology, and which can be thus treated using the antibody or antibody fragment according to the invention, include inflammatory bone disorders and bone resorption disease (see *e.g.,* Bertolini D.R., et al. (1986) Nature 319:516-518; Konig A., et al. (1988) J. Bone Miner. Res. 3:621-627; Lerner U.H. and Ohiin A. (1993) J. Bone Miner. Res. 8:147-155; and Shanka G. and Stern P.H. (1993) Bone 14:871-876), hepatitis, including alcoholic hepatitis (see *e.g.,* McClain C.J. and Cohen D.A. (1989) Hepatology 9:349-351; Felver M.E. et al. (1990) Alcohol Clin. Exp. Res. 14:255-259; and Hansen J. et al. (1994) Hepatology 20:461-474), viral hepatitis (Sheron N. et al. (1991) J. Hepatol. 12:241-245; and Hussain M.J. et al. (1994) J. Clin. Pathol. 47:1112-1115), and fulminant hepatitis; coagulation disorders (see *e.g.*, van der Poll T. et al. (1990) N. Engl. J. Med. 322:1622-1627; van der Poll T. et al. (1991) Prog. Clin. Biol. Res. 367:55-60; burns (see *e.g.,* Giroir B.P. et al. (1994) Am. J. Physiol. 267:H118-124; Liu X.S. et al. (1994) Burns 20:40-44), reperfusion injury (see *e.g.,* Scales W.E. et al. (1994) Am. J. Physiol. 26:1122-1127; Serrick C. et al. (1994) Transplantation 58:1158-1162; Yao Y.M. et al. (1995) Resuscitation 29:157-168), keloid formation (see *e.g.,* McCauley R. L et al. (1992) J. Clin. Immunol. 12:300-308), scar tissue formation; hyperthermia; periodontal disease; obesity and radiation toxicity.

"Therapeutically effective amount" is considered as the amount of therapeutic agent administered during the treatment that will relieve to some extent one or more symptoms of the disease being treated.

The term "chronic" administration refers to the continuous (long-term) administration of the agent(s) as opposed to the acute (short-term) administration mode, so as to maintain the initial therapeutic effect (activity) for an extended period of time.

"Intermittent" administration refers to the treatment that is not performed consecutively without interruption, but rather is cyclic by nature.

In the present specification and in the following claims, unless the context requires otherwise, the words "have," "include" and "comprise," or variations thereof, such as "has," "having," "includes," "including," "comprises" or "comprising" should be understood as the inclusion of a stated integer or group of integers but not the exclusion of any other integer or group of integers.

Detailed Description of the Invention

Aqueous Composition

The present invention relates to novel, improved aqueous compositions, that can optionally be lyophilized, comprising an appropriate amount of a recombinant monoclonal anti-TNFa antibody in an appropriate buffer(s), one or more appropriate stabilizing agents and other excipients selected from appropriate surface-active substances and isotonicity agents. Said composition prevents the formation of protein (antibody) aggregates and maintains the efficacy and stability of the therapeutic compound for a desirable period of time.

Adalimumab and infliximab are examples of a recombinant monoclonal anti-TNFa antibody. Infliximab is an example of a chimeric antibody. Adalimumab is an example of a human antibody. In a preferred embodiment, the antibodies used in the composition are human antibodies. In a more preferred embodiment, the antibodies used in the composition are human anti-human TNFα antibodies. In yet another embodiment, the composition comprises D2E7 and a combination of D2E7 with other antibodies. The D2E7 antibody, known generically as adalimumab, with its high affinity for binding to TNFα, low dissociation kinetics, and high neutralizing ability. Adalimumab is available in the pharmaceutical market under the trademark Humira®. The names adalimumab and D2E7 used throughout the present specification represent the same human monoclonal antibody. In a preferred embodiment, the monoclonal antibody is adalimumab or antigen-binding portion thereof.

In some embodiments, the recombinant monoclonal anti-TNFα antibodies or antigen-binding portions thereof are generally present in a therapeutic amount up to 200 mg/mL. In a preferred embodiment, the therapeutic amount is from about 1 mg/mL to about 150 mg/mL. In a more preferred embodiment, the therapeutic amount is from about 1 mg/mL to about 100 mg/mL. In a more preferred embodiment, the therapeutic amount is from about 50 mg/mL to about 100 mg/mL. In an even more preferred embodiment, the therapeutic amount is about 100 mg/mL.

The aqueous composition in question comprises an appropriate buffer solution in combination with other pharmaceutically acceptable excipients that stabilize the pharmaceutical preparation. Appropriate buffer solutions that can be used are selected from the group that is known in the art and can be found in the literature. In one embodiment, appropriate buffer solutions comprise, but are not limited to, an acetate ion-based buffer agent (or buffer system). In a preferred embodiment, the appropriate buffer comprises an acetate buffer. In an even more preferred embodiment, the acetate buffer is selected from sodium acetate trihydrate combined with acetic acid.

Buffer solutions are generally used in concentrations from about 1 mM to about 100 mM. In a preferred embodiment, the buffer concentration is from about 1 mM to about 50 mM. In a more preferred embodiment, the buffer concentration is from about 1 mM to about 20 mM. In an even more preferred embodiment, the buffer concentration is about 5 mM.

In some embodiments, sodium acetate trihydrate is present in an amount of up to 1.2 mg/mL. In a preferred embodiment, the amount of sodium acetate trihydrate is from about 0.4 mg/mL to about 1.2 mg/mL. In a more preferred embodiment, the amount of sodium acetate trihydrate is from about 0.4 mg/mL to about 0.5 mg/mL. In a more preferred embodiment, the amount of sodium acetate trihydrate is 0.436 mg/mL.

In one embodiment, the liquid composition maintains pH in the range of from 4.0 to about 7.0, depending on the monoclonal antibody used. In a preferred embodiment, the buffer used maintains pH of the composition in the range of about 4.5 to 6.0. In a more preferred embodiment, pH is maintained at about from 5.0 to 6.0. In a more preferred embodiment, pH is maintained at about 5.5.

Said aqueous composition comprises appropriate surface-active substances that are pharmaceutically acceptable excipients used to protect protein compositions from various stress effects such as stirring, shearing, high temperature, freezing, etc. Appropriate surface-active substances include, but are not limited to, Polysorbates or Poloxamers, or a combination of Polysorbate and Poloxamer. In a preferred embodiment, the appropriate surface-active substance is Polysorbate. In a preferred embodiment, the appropriate surface-active substance is Poloxamer. In a preferred embodiment, the appropriate surface-active substance is a combination of Polysorbate and Poloxamer. In a more preferred embodiment, Polysorbate is Polysorbate 20 or Polysorbate 80 (also distributed under the trademark Tween® 20 or Tween® 80). In a more preferred embodiment, Poloxamer is Poloxamer 188 (also distributed under the trademark Kolliphor P188®). In another preferred embodiment, the appropriate surface-active substance is a combination of polysorbate 20 / polysorbate 80 and poloxamer 188.

In some embodiments, Polysorbate 20 is present in an amount of up to 10 mg/mL. In a preferred embodiment, the amount of polysorbate 20 is from about 0.05 mg/mL to about 10 mg/mL. In a more preferred embodiment, the amount of Polysorbate 20 is from about 0.1 mg/mL to about 1 mg/mL. In a more preferred embodiment, the amount of polysorbate 20 is about 0.5 mg/mL.

In some embodiments, Polysorbate 80 is present in an amount of up to 10 mg/mL. In a preferred embodiment, the amount of polysorbate 80 is from about 0.05 mg/mL to about 10 mg/mL. In a more preferred embodiment, the amount of Polysorbate 80 is from about 0.1 mg/mL to about 1 mg/mL. In a more preferred embodiment, the amount of polysorbate 80 is about 0.5 mg/mL.

In some embodiments, poloxamer 188 is present in an amount of up to 10 mg/mL. In a preferred embodiment, the amount of poloxamer 188 is from about 0.05 mg/mL to about 10 mg/mL. In a more preferred embodiment, the amount of poloxamer 188 is from about 0.1 mg/mL to about 1 mg/mL. In a more preferred embodiment, the amount of poloxamer 188 is about 0.5 mg/mL.

In some embodiments, the combination of polysorbate 20 / polysorbate 80 and poloxamer 188 is Polysorbate 80 in an amount of up to 10 mg/mL and poloxamer 188 in an amount of up to 10 mg/mL. In a preferred embodiment, the amount of poloxamer 188 is from about 0.05 mg/mL to about 10 mg/mL and the amount of Polysorbate 20 / polysorbate 80 is from about 0.05 mg/mL to about 10 mg/mL. In a more preferred embodiment, the amount of poloxamer 188 is from about 0.1 mg/mL to about 1 mg/mL and the amount of Polysorbate 20 / polysorbate 80 is from about 0.1 mg/mL to about 1 mg/mL. In a more preferred embodiment, the amount of Polysorbate 20 / polysorbate 80 is about 0.5 mg/mL and the amount of Poloxamer 188 is about 1.0 mg/mL.

The aqueous composition comprises one or more appropriate stabilizing agents that are pharmaceutically acceptable excipients and protect the pharmaceutically active ingredient from chemical and/or physical degradation during manufacture, storage, and use. In one embodiment, the stabilizing agents include, but are not limited to, amino acids, oligosaccharides, or appropriate derivatives or mixtures thereof. In another preferred embodiment, the appropriate stabilizing agent is trehalose. In another preferred embodiment, the appropriate stabilizing agent is proline. In another preferred embodiment, the appropriate stabilizing agent is a combination of trehalose and proline. In another preferred embodiment, the appropriate stabilizing agent is a combination of trehalose and arginine.

In another embodiment, oligosaccharides that can also be used as stabilizing agents include trehalose.

In some embodiments, trehalose is present in an amount of up to 150 mg/mL. In a preferred embodiment, the amount of trehalose is from about 25 mg/mL to about 150 mg/mL. In a more preferred embodiment, the amount of trehalose is from about 50 mg/mL to about 100 mg/mL. In a more preferred embodiment, the amount of trehalose dihydrate is about 80 mg/mL.

In another embodiment, the combination of trehalose and arginine is selected as the stabilizing agent. In some embodiments, trehalose is present in an amount of up to 150 mg/mL, and arginine is in an amount of up to 1.0 mg/mL. In a preferred embodiment, the amount of trehalose is from about 25 mg/mL to about 150 mg/mL, and the amount of arginine is from 0.05 to 1.0 mg/mL. In a more preferred embodiment, the amount of trehalose is from about 50 mg/mL to about 100 mg/mL, and the amount of arginine is from 0.05 to 0.2 mg/mL. In a more preferred embodiment, the amount of trehalose dihydrate is about 80 mg/mL, and the amount of arginine hydrochloride is about 0.1 mg/mL.

In another such embodiment, an amino acid that can be used as stabilizing agents is proline.

In some embodiments, proline is present in an amount of up to 40 mg/mL. In a preferred embodiment, the amount of proline is from about 5 mg/mL to about 40 mg/mL. In a more preferred embodiment, the amount of proline is from about 20 mg/mL to about 35 mg/mL. In a more preferred embodiment, the amount of proline is about 27 mg/mL.

In some embodiments, the combination of trehalose and proline is trehalose in an amount of up to 150 mg/mL and proline in an amount of up to 40 mg/mL. In a preferred embodiment, the amount of trehalose is from about 25 mg/mL to about 150 mg/mL, and the amount of proline is from about 5 mg/mL to about 40 mg/mL. In a more preferred embodiment, the amount of trehalose is from about 20 mg/mL to about 50 mg/mL, and the amount of proline is from about 10 mg/mL to about 20 mg/mL. In a more preferred embodiment, the amount of trehalose dihydrate is about 40 mg/mL, and the amount of proline is about 13.5 mg/mL.

In some embodiments, the aqueous composition comprises from 50 to 200 mg/mL of the recombinant monoclonal anti-TNF$\alpha$ antibody, from 0.4 to 1.2 mg/mL of sodium acetate trihydrate, from 25 to 150 mg/mL of trehalose and/or from 5 to 40 mg/mL of proline or from 25 to 150 mg/mL of trehalose, and from 0.05 to 0.5 mg/mL of arginine, glacial acetic acid until pH 5.0 to 6.0, from 0.1 mg/mL to 1 mg/mL of Polysorbate 20. In a preferred embodiment of the aqueous composition, the recombinant monoclonal anti-TNF$\alpha$ antibody is adalimumab. In a more preferred embodiment, the composition comprises from 50 to 150 mg/mL of adalimumab, 0.436 mg/mL of sodium acetate trihydrate, 80 mg/mL of trehalose dihydrate, glacial acetic acid until pH 5.5, 0.5 mg/mL of polysorbate 20. In a more preferred embodiment, the composition comprises from 50 to 150 mg/mL of adalimumab, 0.436 mg/mL of sodium acetate trihydrate, 80 mg/mL of trehalose dihydrate, 0.1 mg/mL of arginine hydrochloride, glacial acetic acid until pH 5.5, 0.5 mg/mL of polysorbate 20. In a more preferred embodiment, the composition comprises from 50 to 150 mg/mL of adalimumab, 0.436 mg/mL of sodium acetate trihydrate, 27 mg/mL of proline, glacial acetic acid until pH 5.5, 0.5 mg/mL of Polysorbate 20. In a more preferred embodiment, the composition comprises from 50 to 150 mg/mL of adalimumab, 0.436 mg/mL of sodium acetate trihydrate, 40 mg/mL of trehalose dihydrate, 13.5 mg/mL of proline, glacial acetic acid until pH 5.5, 0.5 mg/mL of polysorbate 20.

In some embodiments, the aqueous composition comprises from 50 to 200 mg/mL of the recombinant monoclonal anti-TNF$\alpha$ antibody, from 0.4 to 1.2 mg/mL of sodium acetate trihydrate, from 25 to 150 mg/mL of trehalose and/or from 5 to 40 mg/mL of proline or from 25 to 150 mg/mL of trehalose, and from 0.05 to 0.5 mg/mL of arginine, glacial acetic acid until pH 5.0 to 6.0, from 0.1 mg/mL to 1 mg/mL of polysorbate 80. In a preferred embodiment of the aqueous composition, the recombinant monoclonal anti-TNF$\alpha$ antibody is adalimumab. In a more preferred embodiment, the composition comprises from 50 to 150 mg/mL of adalimumab, 0.436 mg/mL of sodium acetate trihydrate, 80 mg/mL of trehalose dihydrate, glacial acetic acid until pH 5.5, 0.5 mg/mL of Polysorbate 80. In a more preferred embodiment, the composition comprises from 50 to 150 mg/mL of adalimumab, 0.436 mg/mL of sodium acetate trihydrate, 80 mg/mL of trehalose dihydrate, 0.1 mg/mL of arginine hydrochloride, glacial acetic acid until pH 5.5, 0.5 mg/mL of Polysorbate 80. In a more preferred embodiment, the composition comprises from 50 to 150 mg/mL of adalimumab, 0.436 mg/mL of sodium acetate trihydrate, 27 mg/mL of proline, glacial acetic acid until pH 5.5, 0.5 mg/mL of Polysorbate 80. In a more preferred embodiment, the composition comprises from 50 to 150 mg/mL of adalimumab, 0.436 mg/mL of sodium acetate trihydrate, 40 mg/mL of trehalose dihydrate, 13.5 mg/mL of proline, glacial acetic acid until pH 5.5, 0.5 mg/mL of polysorbate 80.

In some embodiments, the aqueous composition comprises from 50 to 200 mg/mL of the recombinant monoclonal anti-TNF$\alpha$ antibody, from 0.4 to 1.2 mg/mL of sodium acetate trihydrate, from 25 to 150 mg/mL of trehalose and/or 5 to 40 mg/mL of proline or from 25 to 150 mg/mL of trehalose, and from 0.05 to 0.5 mg/mL of arginine, glacial acetic acid until pH 5.0 to 6.0, from 0.1 mg/mL to 1 mg/mL of Poloxamer 188. In a preferred embodiment, the recombinant monoclonal anti-TNF$\alpha$ antibody is adalimumab. In a more preferred embodiment, the composition comprises from 50 to 150 mg/mL of adalimumab, 0.436 mg/mL of sodium acetate trihydrate, 80 mg/mL of trehalose dihydrate, glacial acetic acid until pH 5.5, 1 mg/mL of Poloxamer 188. In a more preferred embodiment, the composition comprises from 50 to 150 mg/mL of adalimumab, 0.436 mg/mL of sodium acetate trihydrate, 80 mg/mL of trehalose dihydrate, 0.1 mg/mL of arginine hydrochloride, glacial acetic acid until pH 5.5, 1 mg/mL of Poloxamer 188. In a more preferred embodiment, the composition comprises from 50 to 150 mg/mL of adalimumab, 0.436 mg/mL of sodium acetate trihydrate, 27 mg/mL of proline, glacial acetic acid until pH 5.5, 1.0 mg/mL of Poloxamer 188. In a more preferred embodiment, the composition comprises from 50 to 150 mg/mL of adalimumab, 0.436 mg/mL of sodium acetate trihydrate, 40 mg/mL of trehalose dihydrate, 13.5 mg/mL of proline, glacial acetic acid until pH 5.5, 1.0 mg/mL of poloxamer 188.

Further, said composition may additionally comprise one or more other appropriate excipients that are well-known to those skilled in the art.

The above compositions are suitable for intravenous, subcutaneous or intramuscular administration.

In some embodiments, the liquid composition maintains storage stability to the effect that there are no further protein aggregation processes or modifications compared to the measure of stability at the time zero.

Methods for treatment and use of an aqueous composition

In another embodiment, the invention relates to a method for treating a mammal comprising administering to a mammal a therapeutically effective amount of the pharmaceutical composition of the invention wherein the mammal may have a disease or disorder that can be effectively treated with adalimumab.

In a preferred embodiment, the mammal is a human.

Diseases or disorders that can be treated with the compositions provided include, as non-limiting examples, active (moderate to severe) rheumatoid arthritis, active psoriatic arthritis, active ankylosing spondylitis, chronic (moderate to severe) plaque psoriasis, (moderate to severe) ulcerative colitis, axial spondyloarthritis, active hidradenitis suppurativa, juvenile idiopathic arthritis, (moderate or severe) Crohn's disease, uveitis, active enthesitis-associated arthritis. Additional diseases or disorders that can be treated with the compositions of the present invention include those described in US Patent Nos. 6090382 and 8216583, the relevant portions of which are incorporated herein by reference.

The provided pharmaceutical compositions may be administered to a subject in need of treatment by systemic injection, for example by intravenous or subcutaneous, or by intramuscular injection; or by injection or application to a relevant site, for example by direct injection or direct application to the site when the site is exposed during surgery; or by topical application.

In one embodiment, the invention relates to a method for treating and/or preventing rheumatoid arthritis comprising administering to a mammal in need thereof a therapeutically effective amount of one of the provided adalimumab compositions.

The therapeutically effective amount of adalimumab in the provided compositions depends on the condition to be treated, the severity of the condition, prior therapy, and the patient's medical history, and the response to the therapeutic agent. An appropriate dose can be adjusted according to the judgment of the attending physician so that it can be administered to the patient at once or over multiple administrations.

In one embodiment, the effective amount of adalimumab per adult dose is from about 1 to 500 $mg/m^2$, or about 1 to 200 $mg/m^2$, or about 1 to 40 $mg/m^2$, or about 5 to 25 $mg/m^2$.

Alternatively, a flat dose can be administered, the amount of which can be in the range of 2 to 500 mg/dose, 2 to 100 mg/dose or about 10 to 80 mg/dose.

If the dose is to be administered more than once a week, the exemplary dose range is the same as the aforementioned dose ranges or lower, and it is preferably administered two or more times per week at a dose range of 25 to 100 mg/dose.

In another embodiment, an acceptable dose for administration by injection comprises 80 to 100 mg/dose or alternatively comprises 80 mg per dose.

The dose can be administered weekly, biweekly or separated by several weeks (for example, from 2 to 8).

In one embodiment, adalimumab is administered at the dose of 40 mg by a single subcutaneous (SC) injection.

In some cases, improvements in a patient's condition can be achieved by administering a dose of up to about 100 mg of the pharmaceutical composition one to three times a week for a period of at least three weeks. To achieve the desired level of improvement, treatment for longer periods may be necessary. For incurable chronic conditions, the treatment regimen can be continued indefinitely. For pediatric patients (age 4 to 17 years), a suitable treatment regimen may include administering a dose of 0.4 mg/kg to 4 mg/kg of adalimumab one or more times per week.

In another embodiment, the pharmaceutical formulations of the invention may be prepared in a bulk formulation, and as such, the components of the pharmaceutical composition are present in the amounts higher than it would be required for administration and are diluted appropriately prior to the administration.

The pharmaceutical compositions can be administered as a single therapeutic agent or in combination with additional therapeutic agents as needed. Thus, in one embodiment, the provided methods for treating and/or preventing are used in combination with administering a therapeutically effective amount of another active agent. The other active agent may be administered before, during or after administering the pharmaceutical compositions of the invention. The other active agent may be administered as part of the provided composition or, alternatively, as a separate formulation.

The administration of the provided pharmaceutical compositions can be carried out in various ways, including parenteral, oral, buccal, nasal, rectal, intraperitoneal, intradermal, transdermal, subcutaneous, intravenous, intraarterial, intracardiac, intraventricular, intracranial, intratracheal, intrathecal, intramuscular injection, intravitreal injection, and topical application.

The pharmaceutical compositions of the present invention are particularly useful for parenteral administration, *i.e.,* subcutaneously, intramuscularly, intravenously, intraperitoneally, intramedullary, intraarticulary, intrasynovialy and/or intrathecally. The parenteral administration can be carried out by bolus injection or continuous infusion. The pharmaceutical compositions for injection may be in a unit dosage form, for example, but is not limited to in ampoules, vials, prefilled syringes or in multi-dose containers with an added preservative. In addition, a number of recent drug delivery approaches have been developed, and the pharmaceutical compositions of the present invention are suitable for administration by these new methods, *e.g.,* Inject-ease®, Genject®, injectors such as GenPen®, and needleless devices such as MediJector® and BioJector®. The pharmaceutical composition of the present invention can also be adapted for yet to be discovered modes of administration. See also Langer, 1990, Science, 249:1527-1533.

The provided pharmaceutical compositions can also be formulated as a depot preparation. Such long-acting formulations can be administered by implantation (*e.g.,* subcutaneously or intramuscularly) or by intramuscular injection.

Thus, for example, the formulations can be modified using suitable polymeric or hydrophobic materials (*e.g.,* as an emulsion in an acceptable oil), or ion exchange resins, or as sparingly soluble derivatives, for example, as a sparingly soluble salt.

The pharmaceutical compositions may, if desired, be provided in a vial, package or dispenser device, which may contain one or more unit dosage forms comprising the active ingredient. In one embodiment, the dispenser device may comprise a syringe comprising a single dose of a ready for injection liquid formulation. The syringe can be accompanied by the instruction for administration.

In another embodiment, the present invention provides a kit or container containing an aqueous pharmaceutical composition of the invention. The polypeptide concentration in the aqueous pharmaceutical composition can vary over a wide range, but generally within the range of about 1 to about 200 mg/mL of the aqueous formulation. The kit can also be accompanied by the instruction for use.

Method for Producing

A method for producing the above compositions comprises the addition of acetate buffering agents to an aqueous phase followed by the addition, in any order, of the following components: an osmolyte and/or a stabilizing agent selected from the group of trehalose, proline or a combination thereof; a recombinant monoclonal anti-TNFα antibody; a surface-active substance selected from the group of polysorbate 20, polysorbate 80, poloxamer 188, or a combination thereof.

Techniques

1. Protein Concentration Determination in Test Samples.

The protein concentration was determined using UV spectrophotometry at a wavelength of 280 nm in UV-spectrophotometry plates.

Each sample was diluted with appropriate placebo solution to a concentration of ˜ 0.5 mg/mL. 150 μL of the diluted sample was placed into a well of the UV spectrophotometry plate. The optical density of the plate solutions was measured using a plate-reader at the wavelength of 280 nm. The appropriate placebo solution was used as the reference solution.

The protein concentration (C) at mg/mL was calculated by the following formula:

$$C = \frac{A(280) * b}{\varepsilon * l}$$

$A_{280}$ is optical density value at the wavelength of 280 nm;
$\varepsilon$ is extinction value for the study protein;
$b$ is total dilution ratio of the sample;
$l$ is layer thickness per plate well; for 150 μL, $l$ = 0.42 cm.

2. Buffer Replacement and Sample Concentration.

Diafiltration and concentration of the samples were performed in Stirred Cell (Millipore) concentration cells under pressure.

3. "PEG aggregation."

Preparation of PEG 6000 Solutions.

A PEG 6000 solution having a mass concentration of 20-25% in the study additive formulation was prepared. The resulting solutions were filtered through a 0.45 μm Durapore filter.

The calculated amount of the sample, the additive solution, and 20-25% PEG 6000 solution were transferred into 96-well UV-spectrophotometry plates such that the PEG6000 concentration per well was from 0 to 18%, and the protein concentration per well was 1 mg/mL. All the resulted solutions in wells were well mixed by pipetting.

Next, the solution degree of turbidity was assessed visually, and the optical density of the solutions was measured at a wavelength of 400 nm. The less stable the sample, the lower PEG 6000 concentration will form visible aggregates (opalescence). The solution degree of turbidity was also evaluated following one or more days after the solution preparation.

## 4. Protein Homogeneity and Aggregation Point Determination using Dynamic Light Scattering (DLS) Technique.

Study sample homogeneity determination was performed on a Zetasizer Nano ZSP in the Size measurement mode. For this purpose, 0.05 mL of the solution was placed into a dust-free disposable plastic cuvette.

- Analytical model: Protein analysis.
- Holding at the temperature for 30 seconds before measuring.
- At each point, an average of 13 measurements in 3 replications.

Determination of the study protein aggregation point was performed on the Zetasizer Nano ZSP. For this purpose, the solution was placed into a quartz dust-free cuvette, which was gradually heated in the instrument while continuously measuring the intensity of the scattered light in the Temperature trend measurement mode.

- Analytical model: Protein analysis.
- Temperature trend, mod: Protein aggregation point. From 50 to 85°C at the heating step of 1.5°C.
- Holding at the temperature for 30 seconds before measuring.
- At each point, an average of 15 measurements in 1 replication.

A temperature trend was plotted using the instrument software, which automatically calculates the protein aggregation point (Aggregation point).

## 5. Thermal Stability Determination by "Thermostress 50°C" Technique.

The test samples were divided into 2 parts and placed in separate tubes: 1 tube for each formulation was stored in a refrigerator at +4°C, the rest were placed into an incubator and incubated at 50°C for the specified time. On completing the warm-up period, the tubes were removed from the incubator, allowed to stand at room temperature for about 15 minutes, and submitted for analysis according to the specification.

## 6. Colloidal Stability Determination by "Shake-Test" Technique.

The test samples were divided into 2 parts of 200 $\mu$L each and placed in glass vials, 1 vial of each formulation was stored in the refrigerator at +4°C, the rest were placed into a thermo-shaker and shook at 800 rpm at 2 to 8°C for the specified time. On completing the stress, the tubes were removed from the thermo-shaker and submitted for analysis according to the specification.

## 7. Colloidal Stability Determination by Cryoconcentration Technique.

The test samples were divided into 2 parts and placed in polymeric tubes: 1 tube for each formulation was stored in a refrigerator at +4°C, the rest were placed into a freezing chamber and stored at minus 16 to 20°C for the specified time. On completing the stress, the tubes were removed from the freezing chamber, allowed to stand at room temperature until the contents completely thawed, the solutions were mixed using Vortex and submitted for analysis according to the specification.

## 8. Sample Purity Determination by Size Exclusion High-Performance Liquid Chromatography (SE-HPLC) Technique.

Column: Tosoh TSK-GelG3000SW$_{XL}$ 7.8 mm ID $\times$ 30 cm, cat. # 08541.
Column temperature: 25 $\underline{o}$C.
Mobile phase flow rate: 0.7 mL/min.
Injection volume : 20 $\mu$L.
Sample concentration : 5 mg/mL.
Detector wavelength: 220 nm.
Elution time: 23 min.
Mobile phase: Anhydrous disodium hydrophosphate: 7.1 mg/mL.
Sodium chloride: 17.54 mg/mL.
pH of the mobile phase was adjusted to 7.0 with orthophosphoric acid.

9. Sample Acid-Base Profile Determination using Caliper LabChip GX II Instrument.

9.1. Sample preparation.

Samples were diluted to a concentration of 1 mg/mL. 2 μL of 5 mg/mL carboxypeptidase B (CpB) solution was added to 200 μL of the resulting solution. It was stirred and incubated for 2 hours at 37°C. The test samples were dialyzed against three water changes in Amicon Ultra centrifuge tubes and concentrated to 2 mg/mL.

9.2. Work solution preparation.

Work solutions and a plate with the assay samples were prepared according to the manufacturer's procedure using the HT Protein Charge Variant Labeling Kit.

9.3. Chip preparation.

Preparation of the chip and tube with buffer was carried out according to the manufacturer's procedure using buffer solutions from the Protein Charge Variant Buffer Kit.
The assay initiation is a standard operation. Protein Charge Variant 68s assay.

10. Sample Purity Determination using Caliper Labchip GX II Instrument under Reducing and Non-Reducing Conditions.

10.1. Assay sample preparation.

700 μL of HT Protein Express Sample Buffer supplemented with 24.5 μL of 1M DTT for buffer under reducing conditions and 24.5 μL of 1M IAM for buffer under non-reducing conditions was used to prepare denaturing and reducing solutions. Samples were diluted to a concentration of 2 mg/mL. Two microtubes were prepared for each sample - 35 μL of denaturing buffer was added to one, 35 μL of reducing buffer was added to another. The samples were denatured at 100°C for 5 minutes. Tubes were vortexed, then 70 μL of water was added to each tube and mixed. 44 μL of each sample was transferred to a 96-well plate for analysis.

10.2. Work solution preparation and chip loading.

Work solution and chip preparation were carried out according to a standard procedure using the HT Protein Express Reagent Kit. The assay initiation is a standard operation. HT Protein Express 200 assay.

11. Sample Acid-Base Profile Determination using Ion-Exchange (IE) HPLC.

| | |
|---|---|
| Column: | DIONEX ProPack WCX-10 4 mm x 250 mm (Tosoh bioscience, Japan); |
| Pre-column: | Dionex ProPack WCX-10G |
| Mobile phase: | Eluent A: 0.01 M 2-(N-morpholino)ethanesulfonic acid (MESA), pH 6.0 |
| | Eluent B: 0.01 M MESA, 0.4 M sodium chloride, pH 5.5; |
| Injection volume: | 40 μL; |
| Flow rate: | 0.5 mL/min; |
| Column temperature: | 25°C; |
| Autosampler temperature: | 5°C; |
| Detection wavelength: | 280 nm. |
| Gradient: | Phase A 95 - 0 - 95 %. |

12. Purity Determination by Polyacrylamide Gel Electrophoresis (PAGE) under Reducing and Non-Reducing Conditions.

PAG was prepared in the presence of sodium dodecyl sulfate in glass plates consisting of a concentrating layer of 4% PAGE and a separating layer: 12.5% PAG for reducing conditions, 8% PAG for non-reducing conditions.
The electrophoresis chamber was assembled and installed according to the operational manual for the vertical electrophoresis device. Specimens were prepared by diluting the samples with purified water to a final concentration of 1 mg/mL. A volume equivalent to 40 μg was taken and the prepared specimens of the test sample were mixed in a ratio of 3:1 (v/v) with a sample application buffer solution containing 2-mercaptoethanol (reducing conditions) and containing no 2-mercaptoethanol (non-reducing conditions), mixed. The resulting solutions were incubated at a temperature of (99 ± 1)°C for 3 minutes (samples containing 2-mercaptoethanol) and at a temperature of (99 ± 1)°C for 1 minute (samples containing no 2-mercaptoethanol). The solutions were cooled to room temperature, mixed and transferred to the wells of PAG under the layer of running buffer solution.

Electrophoresis was run in a direct current mode using a water cooling system. The power source parameters were set as following: when the dye front was passing through the stacking gel, the voltage was 110 V. Once the dye front entered the lower running gel for 5 to 7 mm, the voltage was increased to 180 V. The power source was turned off when the dye front reached the lower limit of the gel.

Once the electrophoresis finished, the gels were separated from the glasses and proteins were fixed with the fixing solution for 16 to 18 hours at room temperature. Further, the gels were stained (in acid blue 83 solution) and washed until a clear view of the bands. The gels were scanned. The purity and impurities in the test samples were evaluated using the GelPro software.

13. Specific Activity Determination.

Sample-specific activity was assessed by the ability to bind specifically and neutralize TNFα, which in turn has a cytotoxic effect and causes the death of WEHI-13 var culture (fibrosarcoma, Mus *musculus*), a variant of the culture that has increased sensitivity to TNFα in the presence of actinomycin D. Sample preparation was performed using a TecanEvo 200 robotic station, RPMI1640, 2 mM Gln, 10% FBS, 2.5 μg/mL of actinomycin D, 5 μg/mL of gentamicin were used as the QM (quantification medium).

The test antibody sample was diluted to 50 μg/mL with a dilution step of no more than 20 and placed into the robotic station. Using the TecanEvo200, three independent dilutions of RS and IS in the range of 10,000 to 0.5 ng/mL were prepared in culture plates, 500 pg/mL of TNFα solution was added to prepared dilutions. The resulting mixture was stirred and incubated at room temperature for 1 hour.

After the incubation, $(0.5 \pm 0.1) \times 10^6$ cells/mL of WEHI-13 var cell suspension was added. The plates were placed in a $CO_2$ incubator and incubated at the temperature of (37 ± 1)ºC, 5% $CO_2$ in humidified air for 20-24 h.

At the end of the incubation period, the vital dye Alamar Blue was added to the culture plates and the plates were incubated under the same conditions until the color development. The fluorescence intensity was evaluated at an excitation/emission wavelength of 544/590 nm using an Infinite M200Pro reading device. Fluorescence intensity versus protein concentration curves were plotted using Magellan 7.2 software; the parallel alignment of the resulted curves was evaluated. The relative specific activity of the test samples was determined as the reference sample ED50 to test sample ED50 ratio, expressed as a percentage.

The following examples are provided for the best understanding of the invention. These examples are provided for illustrative purposes only and are not to be construed as limiting in any way the scope of the invention.

All publications, patents and patent applications cited in this specification are included herein by reference. While the above invention has been described in some detail by way of illustration and example for purpose of clarity, as will be appreciated by those skilled in the art based on the teaching disclosed herein, certain changes and modifications can be made without departing from the spirit and scope of the enclosed embodiments of the invention.

**Examples**

Example 1. Buffer Solution Nature Selection.

Study Formulations.

Four buffer solutions were selected for this study, and the original Humira preparation formulations (for formulations having protein content of 50 mg/mL and 100 mg/mL) were used as a control.

| Humira 1 | Disodium hydrophosphate dihydrate | 1.530 mg/mL |
| | Sodium dihydrophosphate dihydrate | 0.860 mg/mL |
| | Mannitol | 12.0 mg/mL |
| | Citric acid monohydrate | 1.305 mg/mL |
| | Polysorbate 80 | 1.0 mg/mL |
| | Sodium citrate | 0.305 mg/mL |
| | Sodium chloride | 6.165 mg/mL |
| | Sodium hydroxide | up to pH 5.2 |
| Humira 2 | Mannitol | 42.0 mg/mL |
| | Polysorbate 80 | 1.0 mg/mL |
| Acet, pH 5.0 | Sodium acetate trihydrate | 0.436 mg/mL |
| | Glacial acetic acid | up to pH 5.0 |
| Cit, pH 5.0 | Sodium citrate dihydrate | 0.932 mg/mL |
| | Citric acid anhydrous | 0.352 mg/mL |
| His, pH 6.0 | L-histidine | 0.40 mg/mL |
| | Histidine hydrochloride monohydrate | 0.40 mg/mL |
| PB, pH 6.0 | Sodium dihydrophosphate monohydrate | 1.21 mg/mL |
| | Sodium phosphate anhydrous | 0.17 mg/mL |

1.1. Colloidal Stability Determination by PEG Aggregation.

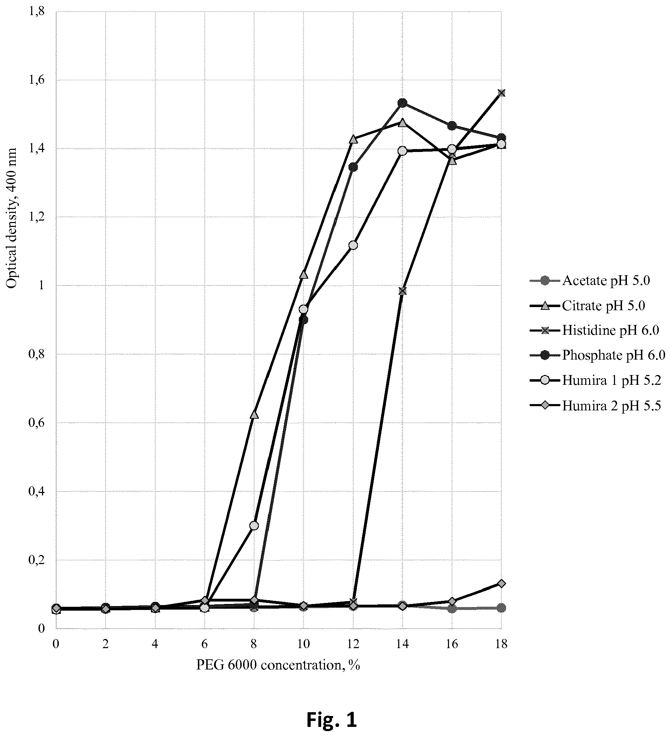

The "PEG aggregation" test was performed in triplicate for each sample. The analysis was performed according to the procedure 3. The data on the average optical density of the solutions are presented in Table 1. The results are also shown in Fig. 1.

Table 1. The average optical density of the solutions after preparation.

| PEG 6000, % | 0 % | 2 % | 4 % | 6 % | 8 % | 10 % | 12 % | 14 % | 16 % | 18 % |
|---|---|---|---|---|---|---|---|---|---|---|
| Acet, pH 5.0 | 0.0597 | 0.0519 | 0.0531 | 0.0593 | 0.0590 | 0.0598 | 0.0502 | 0.0513 | 0.0591 | 0.0608 |
| Cit, pH 5.0 | 0.0582 | 0.0620 | 0.0620 | 0.0639 | 0.6261 | 1.0340 | 1.4279 | 1.4765 | 1.3668 | 1.4133 |
| His, pH 6.0 | 0.0583 | 0.0615 | 0.0621 | 0.0634 | 0.0637 | 0.0664 | 0.0781 | 0.9847 | 1.3867 | 1.5626 |
| PB, pH 6.0 | 0.0598 | 0.0619 | 0.0654 | 0.0656 | 0.0712 | 0.9003 | 1.3453 | 1.5324 | 1.4662 | 1.4297 |
| Humira 1 | 0.0563 | 0.0583 | 0.0594 | 0.0614 | 0.3005 | 0.9315 | 1.1182 | 1.3925 | 1.3980 | 1.4120 |

| Humira 2 | 0.0605 | 0.0579 | 0.0608 | 0.0834 | 0.0845 | 0.0685 | 0.0673 | 0.0658 | 0.0803 | 0.1320 |

There is visible aggregation

1.2. Thermal Stability Determination by Protein Aggregation Point using Dynamic Light Scattering (DLS) Technique.

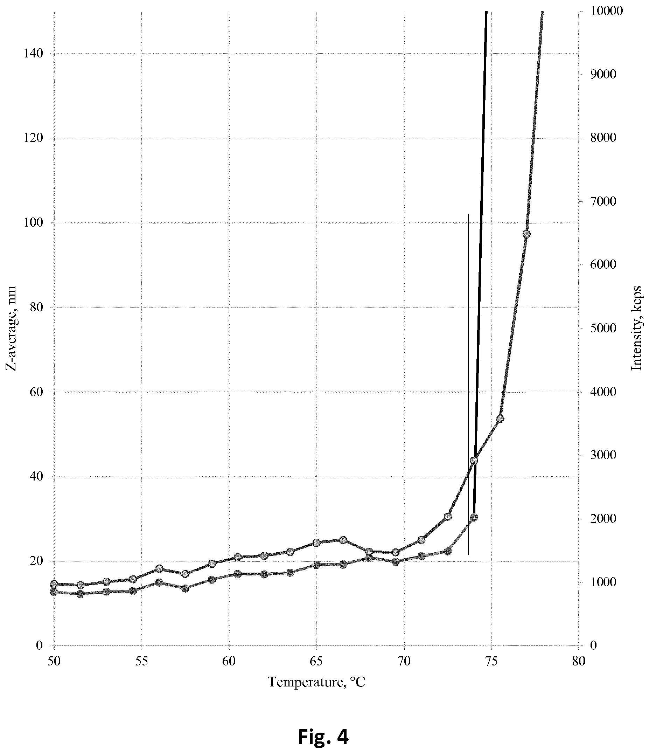

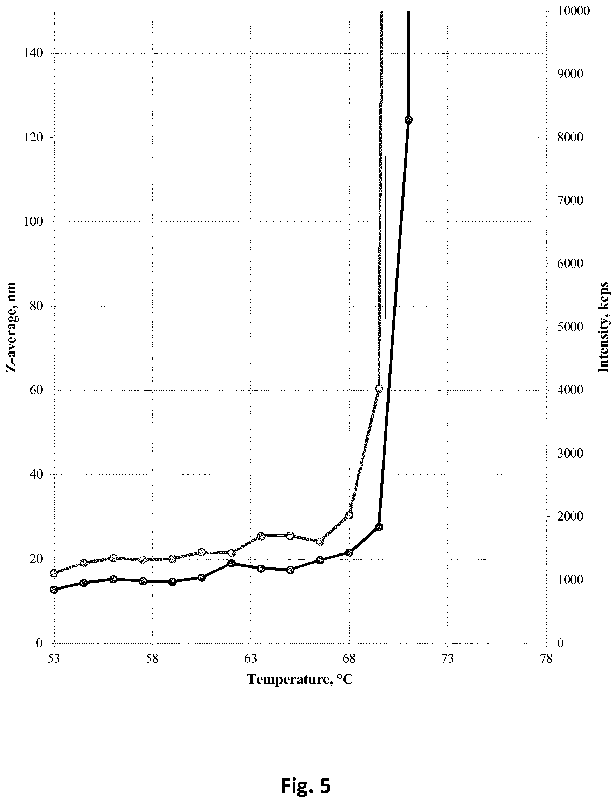

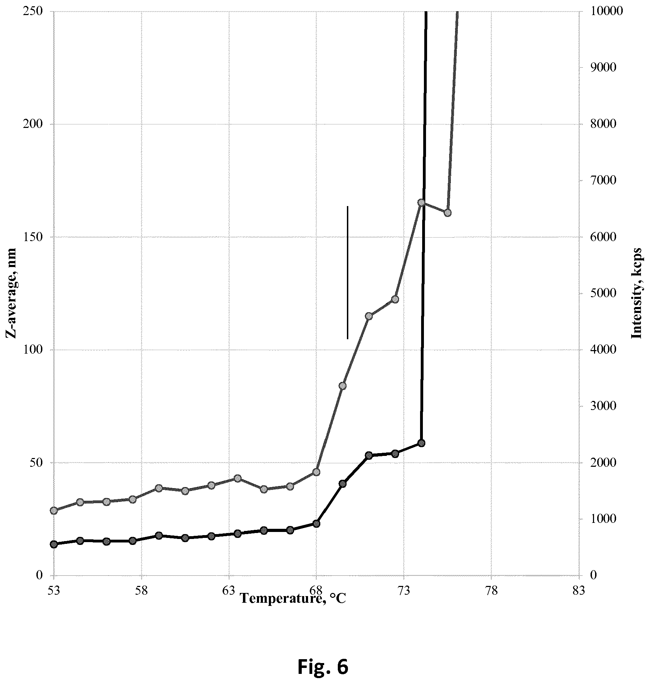

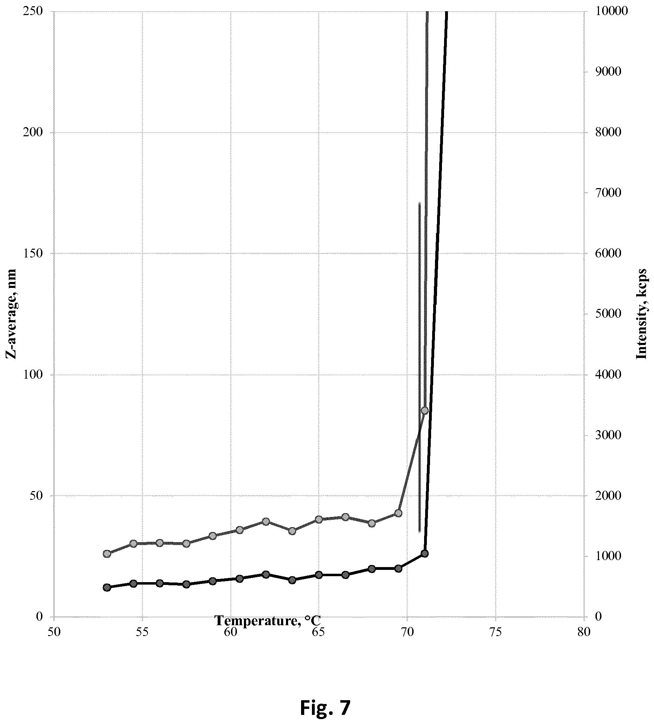

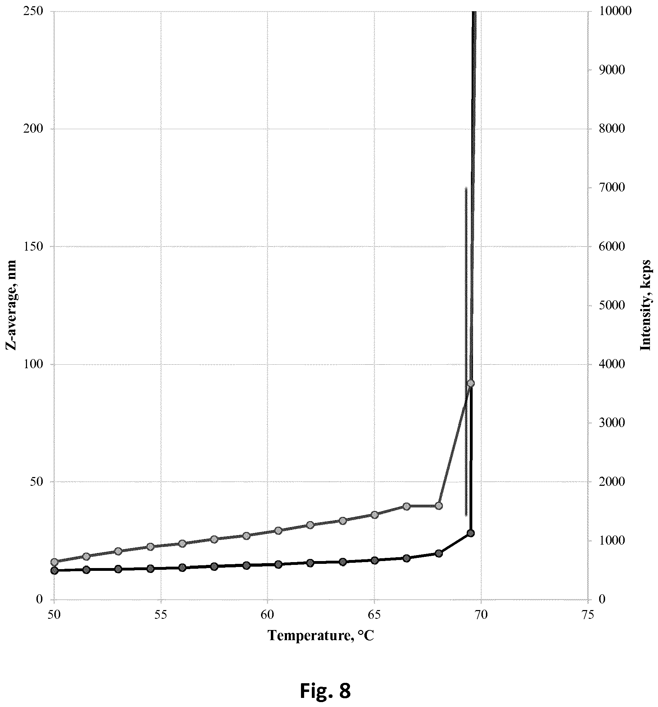

The analysis was performed according to the procedure 4. The results are shown in Table 2 and Fig. 4-8.

1.3. Thermal Stability Determination under Long-Term Exposure using Thermostress 50°C Method.

The analysis was performed according to the procedure 5. The results are shown in Table 2.

1.4. Results.

Table 2. Summary of the buffer solution nature selection.

| Formulation | Thermal stress 50°C, 24 h | | Visible aggregation at PEG 6000 % (SP type) | Aggregation point, °C (DLS) |
|---|---|---|---|---|
| | Increase in impurities, % (SE-HPLC) | Increase in fragments, % (SE-HPLC) | | |
| Protein concentration | 5 mg/mL | | 1 mg/mL | |
| Acet, pH 5.0 | 0.02 | 0.01 | > 18 | 74.0 |
| Cit, pH 5.0 | 1.50 | 0.08 | 8 | 69.5 |
| His, pH 6.0 | 0.75 | 0.20 | 14 | 69.5 |
| PB, pH 6.0 | 0.55 | 0.11 | 10 | 71.0 |
| Humira 1 | 0.38 | 0.15 | 8 | 69.5 |
| Humira 2 | 0.02 | 0.01 | > 18 | 72.5 |

▇ positive results        ▒ negative results        ☐ average results

1.5. Conclusions from Example 1.

Based on the results of this study, the recommended storage formulation (excluding additional additives) is:

| **Acet,** pH 5.0 | Sodium acetate trihydrate | 0.436 mg/mL |
|---|---|---|
| | Glacial acetic acid | up to pH 5.0 |

The present formulation showed the best stabilizing properties among all the test sample: the lowest increase in impurities during thermal stress (0.02% on a par with Humira 2 formulation), the absence of aggregation at 18% PEG 6000, and the highest aggregation temperature (74°C).

Adalimumab with Humira 1 formulation has a lower thermal stability than the recommended formulation. Minimal colloidal and thermal stability was noted for the formulation based on 5 mM citrate buffer solution with pH of 5.0.

Example 2. Composition pH and Buffer Capacity Optimization.

Based on the results of the first part of the study, adalimumab showed the best stability in acetate buffer solution with pH 5.0. According to the prior art, the most patents and patent applications for pharmaceutical compositions comprising adalimumab protect solutions with pH of 4.0 to 8.0. Thus, the purpose of the present section was to study the possibility for obtaining stable compositions comprising acetate ions with pH of less than 4.

Study Formulations.

| Abbreviation | Concentration, mM | pH |
|---|---|---|
| Acet 5 mM pH 6 | 5 | 6.0 |
| Acet 5 mM pH 5.5 | 5 | 5.5 |
| Acet 5 mM pH 5 | 5 | 5.0 |

(continued)

| Abbreviation | Concentration, mM | pH |
|---|---|---|
| Acet 5 mM pH 4 | 5 | 4.0 |
| Acet 5 mM pH 3.75 | 5 | 3.75 |
| Acet 5 mM pH 3.5 | 5 | 3.5 |
| Acet 10 mM pH 6 | 10 | 6.0 |
| Acet 10 mM pH 5.5 | 10 | 5.5 |
| Acet 10 mM pH 5 | 10 | 5.0 |
| Acet 10 mM pH 4 | 10 | 4.0 |
| Acet 10 mM pH 3.75 | 10 | 3.75 |
| Acet 10 mM pH 3.5 | 10 | 3.5 |
| Acet 20 mM pH 6 | 20 | 6.0 |
| Acet 20 mM pH 5.5 | 20 | 5.5 |
| Acet 20 mM pH 5 | 20 | 5.0 |
| Acet 20 mM pH 4 | 20 | 4.0 |
| Acet 20 mM pH 3.75 | 20 | 3.75 |
| Acet 20 mM pH 3.5 | 20 | 3.5 |

2.1. Colloidal Stability Determination by PEG Aggregation.

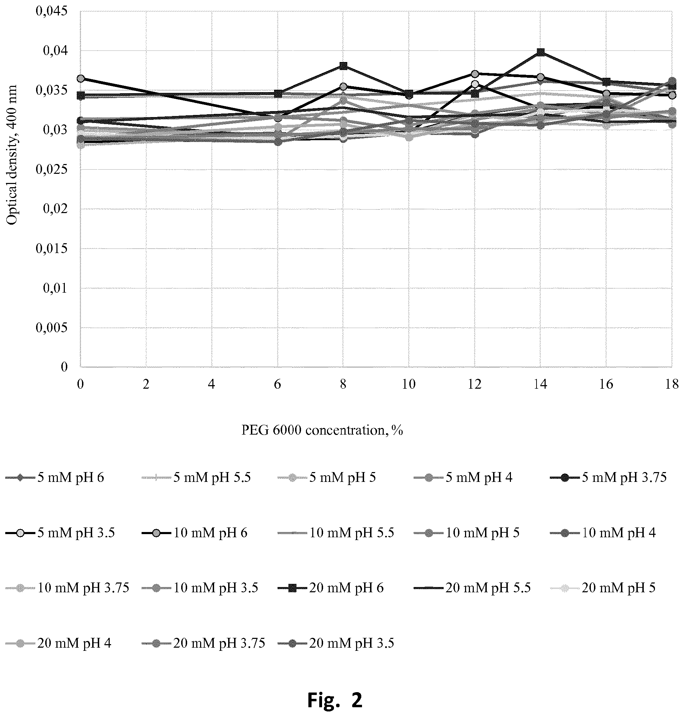

The "PEG aggregation" test was performed in triplicate for each sample. The analysis was performed according to the procedure 3. The data on the average optical density of the solutions are presented in Table 3. The results are also shown in Fig. 2.

Table 3. The average optical density (400 nm) of the solutions after preparation.

| PEG 6000, % | Acet 5 mM pH 6 | Acet 5 mM pH 5.5 | Acet 5 mM pH 5 | Acet 5 mM pH 4 | Acet 5 mM pH 3.75 | Acet 5 mM pH 3.5 | Acet 10 mM pH 6 | Acet 10 mM pH 5.5 | Acet 10 mM pH 5 | Acet 10 mM pH 4 |
|---|---|---|---|---|---|---|---|---|---|---|
| 0 % | 0.0341 | 0.0344 | 0.0299 | 0.0303 | 0.0312 | 0.0285 | 0.0365 | 0.0314 | 0.0289 | 0.0290 |
| 6 % | 0.0346 | 0.0341 | 0.0293 | 0.0294 | 0.0290 | 0.0288 | 0.0315 | 0.0316 | 0.0316 | 0.0296 |
| 8 % | 0.0344 | 0.0342 | 0.0290 | 0.0291 | 0.0295 | 0.0289 | 0.0355 | 0.0322 | 0.0312 | 0.0289 |
| 10 % | 0.0346 | 0.0331 | 0.0298 | 0.0298 | 0.0297 | 0.0296 | 0.0344 | 0.0331 | 0.0299 | 0.0296 |
| 12 % | 0.0348 | 0.0338 | 0.0302 | 0.0313 | 0.0319 | 0.0358 | 0.0371 | 0.0318 | 0.0303 | 0.0295 |
| 14 % | 0.0361 | 0.0346 | 0.0313 | 0.0327 | 0.0331 | 0.0327 | 0.0367 | 0.0316 | 0.0313 | 0.0326 |
| 16 % | 0.0359 | 0.0341 | 0.0341 | 0.0331 | 0.0333 | 0.0329 | 0.0346 | 0.0316 | 0.0339 | 0.0334 |
| 18 % | 0.0348 | 0.0350 | 0.0308 | 0.0308 | 0.0310 | 0.0310 | 0.0344 | 0.0355 | 0.0307 | 0.0314 |

| PEG 6000, % | Acet 10 mM pH 3.75 | Acet 10 mM pH 3.5 | Acet 10 mM pH 3.5 | Acet 20 mM pH 6 | Acet 20 mM pH 5.5 | Acet 20 mM pH 5 | Acet 20 mM pH 4 | Acet 20 mM pH 3.75 | Acet 20 mM pH 3.5 |
|---|---|---|---|---|---|---|---|---|---|
| 0 % | 0.0281 | 0.0292 | 0.0292 | 0.0344 | 0.0310 | 0.0295 | 0.0289 | 0.0289 | 0.0289 |
| 6 % | 0.0292 | 0.0288 | 0.0288 | 0.0346 | 0.0322 | 0.0289 | 0.0304 | 0.0294 | 0.0285 |
| 8 % | 0.0299 | 0.0337 | 0.0337 | 0.0381 | 0.0328 | 0.0292 | 0.0307 | 0.0296 | 0.0298 |
| 10 % | 0.0295 | 0.0306 | 0.0306 | 0.0346 | 0.0316 | 0.0295 | 0.0291 | 0.0302 | 0.0312 |
| 12 % | 0.0319 | 0.0321 | 0.0321 | 0.0346 | 0.0318 | 0.0300 | 0.0308 | 0.0301 | 0.0308 |
| 14 % | 0.0309 | 0.0331 | 0.0331 | 0.0398 | 0.0320 | 0.0322 | 0.0312 | 0.0315 | 0.0306 |
| 16 % | 0.0306 | 0.0320 | 0.0320 | 0.0361 | 0.0310 | 0.0325 | 0.0321 | 0.0315 | 0.0320 |
| 18 % | 0.0313 | 0.0313 | 0.0313 | 0.0356 | 0.0311 | 0.0322 | 0.0321 | 0.0324 | 0.0362 |

2.2. Thermal Stability Determination under Long-Term Exposure using Thermostress 50°C Method.

The analysis was performed according to the procedure 5.

2.3. Colloidal Stability Determination by "Shake-Test" Technique.

The analysis was performed according to the procedure 6.

2.4. Colloidal Stability Determination by Cryoconcentration Technique.

The analysis was performed according to the procedure 7.
Summary is provided in Table 4, where the sample quality measures before and after the thermal stress, shake test and cryoconcentration are demonstrated.

2.5. Results.

Table 4. Summary of the sample quality measures before and after stress.

| | Input control | | | | |
|---|---|---|---|---|---|
| | pH placebo | pH after dialysis | Δ pH after dialysis | Protein C, mg/mL | Turbidity, OD 400 nm |
| | | | | | SP-type |
| 5 mM acet pH 6.0 | 6.00 | 6.10 | 0.10 | 10 | 0.0506 |
| 10 mM acet pH 6.0 | 6.00 | 6.06 | 0.06 | 10 | 0.0531 |
| 20 mM acet pH 6.0 | 6.00 | 6.04 | 0.04 | 10 | 0.0511 |
| 5 mM acet pH 5.5 | 5.50 | 5.60 | 0.10 | 10 | 0.0513 |
| 10 mM acet pH 5.5 | 5.50 | 5.53 | 0.03 | 10 | 0.0531 |
| 20 mM acet pH 5.5 | 5.50 | 5.51 | 0.01 | 10 | 0.0514 |
| 5 mM acet pH 5.0 | 5.00 | 5.15 | 0.15 | 10 | 0.0504 |
| 10 mM acet pH 5.0 | 5.00 | 5.12 | 0.12 | 10 | 0.0501 |
| 20 mM acet pH 5.0 | 5.00 | 5.09 | 0.09 | 10 | 0.0488 |
| 5 mM acet pH 4.0 | 4.00 | 4.35 | 0.35 | 10 | 0.0446 |
| 10 mM acet pH 4.0 | 4.00 | 4.22 | 0.22 | 10 | 0.0471 |
| 20 mM acet pH 4.0 | 4.00 | 4.20 | 0.20 | 10 | 0.0489 |
| 5 mM acet pH 3.75 | 3.75 | 4.04 | 0.29 | 10 | 0.0504 |
| 10 mM acet pH 3.75 | 3.75 | 4.01 | 0.26 | 10 | 0.0501 |
| 20 mM acet pH 3.75 | 3.75 | 3.95 | 0.20 | 10 | 0.0473 |
| 5 mM acet pH 3.5 | 3.50 | 3.90 | 0.40 | 10 | 0.0511 |
| 10 mM acet pH 3.5 | 3.50 | 3.84 | 0.34 | 10 | 0.0476 |
| 20 mM acet pH 3.5 | 3.50 | 3.79 | 0.29 | 10 | 0.0492 |

positive results          negative results

average results/baseline data

**Table 5.**

| | Thermal stress 50°C, 96 h | | | | | | Shake test 800 rpm, 96 h | | | | | |
|---|---|---|---|---|---|---|---|---|---|---|---|---|
| | | Change in purity*, % | | | Turbidity, 400 nm | Acid-base profile Δ abs. %** | | Change in purity*, % | | | Turbidity, OD 400 nm | Acid-base profile Δ abs. %** |
| | Δ pH* | SE-HPLC | Caliper EP, red. | Caliper EP, non-red. | SP type | Caliper | Δ pH* | SE-HPLC | Caliper EP, red. | Caliper EP, non-red. | SP type | Caliper |
| 5 mM acet pH 6.0 | 0.03 | -0.58 | -1.88 | -0.81 | 0.0446 | 28.11 | 0.03 | -0.26 | -0.49 | +0.03 | 0.0490 | 2.66 |
| 10 mM acet pH 6.0 | 0.04 | -0.66 | -1.99 | -0.94 | 0.0479 | 32.34 | 0.05 | -0.31 | -0.87 | +0.19 | 0.0471 | 3.41 |
| 20 mM acet pH 6.0 | 0.01 | -0.74 | -2.09 | -1.59 | 0.0487 | 33.09 | 0.02 | -0.38 | -0.96 | +0.21 | 0.0468 | 3.98 |
| 5 mM acet pH 5.5 | 0.05 | -0.47 | -1.74 | -1.01 | 0.0455 | 25.41 | 0.04 | -0.22 | -0.99 | 0.55 | 0.0413 | 1.38 |
| 10 mM acet pH 5.5 | 0.06 | -0.76 | -1.61 | -0.76 | 0.0489 | 26.19 | 0.06 | -0.28 | -0.87 | +0.51 | 0.0482 | 1.55 |
| 20 mM acet pH 5.5 | 0.03 | -0.88 | -2.33 | -0.81 | 0.0494 | 28.88 | 0.09 | -0.31 | -0.79 | +0.63 | 0.0460 | 1.49 |
| 5 mM acet pH 5.0 | 0.00 | -0.55 | -1.99 | -1.13 | 0.0506 | 26.57 | 0.07 | -0.33 | -1.17 | +0.49 | 0.0480 | 2.45 |
| 10 mM acet pH 5.0 | 0.05 | -0.79 | -2.06 | -0.07 | 0.0517 | 22.04 | 0.06 | -0.50 | -1.07 | 0.55 | 0.0499 | 3.22 |
| 20 mM acet pH 5.0 | 0.03 | -0.91 | -0.65 | -1.90 | 0.0527 | 32.92 | 0.03 | -0.51 | +0.29 | -0.21 | 0.0520 | 7.02 |
| 5 mM acet pH 4.0 | -0.06 | -2.00 | -2.67 | -0.59 | 0.0494 | 33.07 | 0.05 | -0.24 | -1.49 | +2.05 | 0.0466 | 1.51 |
| 10 mM acet pH 4.0 | 0.03 | -2.57 | -2.55 | -1.63 | 0.0507 | 32.88 | 0.07 | -0.29 | -0.91 | +0.45 | 0.0505 | 3.44 |
| 20 mM acet pH 4.0 | -0.03 | -2.85 | -1.24 | -0.99 | 0.0511 | 30.75 | 0.01 | -0.40 | +1.34 | +0.85 | 0.0497 | 2.80 |

EP 3 563 867 A1

| | | | | | | | | | | | | |
|---|---|---|---|---|---|---|---|---|---|---|---|---|
| 5 mM acet pH 3.75 | -0.01 | -3.32 | -5.00 | -2.63 | 0.0492 | 29.11 | 0.07 | -0.42 | +1.33 | -1.41 | 0.0484 | 0.99 |
| 10 mM acet pH 3.75 | 0.02 | -3.09 | -2.62 | -2.50 | 0.0488 | 30.85 | 0.10 | -0.31 | -0.19 | +0.67 | 0.0452 | 1.29 |
| 20 mM acet pH 3.75 | 0.01 | -4.09 | -2.36 | -3.23 | 0.0535 | 30.95 | 0.06 | -0.51 | +12.11 | +0.37 | 0.0504 | 0.37 |
| 5 mM acet pH 3.5 | 0.00 | -4.28 | -5.50 | -2.72 | 0.0489 | 28.10 | 0.08 | -0.38 | -2.19 | +1.03 | 0.0468 | 0.76 |
| 10 mM acet pH 3.5 | 0.02 | -4.61 | -6.22 | -2.88 | 0.0494 | 32.07 | 0.10 | -0.38 | -0.01 | +0.50 | 0.0471 | 3.78 |
| 20 mM acet pH 3.5 | -0.05 | -6.87 | -7.32 | -3.11 | 0.0500 | 38.64 | 0.02 | -0.67 | +0.63 | +1.01 | 0.0498 | 2.67 |

**Table 5.** Continuation.

| | Freezing -20 ° C, defrosting + 25 ° C | | | | | |
| | | Change in purity*, % | | | Turbidity, OD 400 nm | Acid-base profile Δ abs. %** |
| | Δ pH* | SE-HPLC | Caliper EP, red. | Caliper EP, non-red. | SP type | Caliper |
| 5 mM acet pH 6.0 | -0.06 | -0.24 | -0.44 | -0.21 | 0.0487 | 3.11 |
| 10 mM acet pH 6.0 | -0.08 | -0.31 | -0.74 | -0.38 | 0.0493 | 3.54 |
| 20 mM acet pH 6.0 | -0.04 | -1.23 | -0.59 | -0.36 | 0.0511 | 4.11 |
| 5 mM acet pH 5.5 | -0.09 | -0.39 | -0.56 | -0.31 | 0.0486 | 2.66 |
| 10 mM acet pH 5.5 | -0.07 | -0.60 | -0.41 | -0.28 | 0.0473 | 2.19 |
| 20 mM acet pH 5.5 | -0.06 | -0.45 | -0.09 | -0.41 | 0.0480 | 4.01 |
| 5 mM acet pH 5.0 | -0.05 | -0.20 | -0.46 | -0.39 | 0.0494 | 2.67 |
| 10 mM acet pH 5.0 | -0.01 | -0.49 | -0.87 | +1.09 | 0.0466 | 4.46 |
| 20 mM acet pH 5.0 | 0.01 | -0.59 | -0.08 | -0.09 | 0.0536 | 3.69 |
| 5 mM acet pH 4.0 | -0.09 | -1.29 | -0.75 | +0.99 | 0.0475 | 2.06 |
| 10 mM acet pH 4.0 | 0.00 | -1.77 | -2.11 | -0.79 | 0.0440 | 3.18 |
| 20 mM acet pH 4.0 | -0.04 | -1.62 | +0.73 | +0.72 | 0.0513 | 4.54 |
| 5 mM acet pH 3.75 | -0.05 | -1.49 | -1.74 | -0.01 | 0.0463 | 3.07 |
| 10 mM acet pH 3.75 | -0.02 | -1.76 | -1.68 | +0.70 | 0.0473 | 3.02 |
| 20 mM acet pH 3.75 | 0.00 | -2.16 | +2.42 | +1.03 | 0.0544 | 4.70 |
| 5 mM acet pH 3.5 | -0.04 | -1.22 | -2.09 | +0.72 | 0.0479 | 2.60 |

| | | | | | |
|---|---|---|---|---|---|
| 10 mM acet pH 3.5 | 0.07 | -1.97 | -1.59 | +0.07 | 0.0492 | 6.83 |
| 20 mM acet pH 3.5 | -0.06 | -2.98 | -1.13 | +1.02 | 0.0427 | 4.88 |

* Change was calculated by the formula $\Delta =$ (Value after stress $-$ Value before stress) ** Absolute change was calculated by the formula $\Delta =$

|Content of acid fraction before stress $-$ Content of acid fraction after stress| + |Content of basic fraction before stress $-$

Content of basic fraction after stress| + |Content of dominant fraction before stress $-$ Content of dominant fraction after stress|

positive results      negative results      average results / baseline data

2.6. Conclusions from Example 2.

- The study showed that the presence of adalimumab in acid solutions of placebo leads to a significant increase in the pH level. In addition, compositions with pH of less than 5.0 demonstrated poor stability during dialysis (low purity on Labchip Caliper under reducing conditions) and thermal stress (low purity after stress on all assay methods used).

- The most stable sample among all studied is adalimumab in 5 mM acetate buffer solution with pH of 5.0 to 6.0. They demonstrated a minimal change in purity and in acid-base profile during the stresses. To create an adalimumab composition, however, it is possible to use solutions with a larger buffer capacity.

Example 3. Osmotic and Stabilizer Agent Selection.

Based on the results of the first part of the study, adalimumab showed the best stability in acetate buffer solution with pH 5.0-6.0. The formulation with pH 5.5 was used as a basis for the osmolyte and stabilizing agent selection.

Study Formulations.

| | | |
|---|---|---|
| Humira 1 | Disodium hydrophosphate dihydrate | 1.530 mg/mL |
| | Sodium dihydrophosphate dihydrate | 0.860 mg/mL |
| | Mannitol | 12.0 mg/mL |
| | Citric acid monohydrate | 1.305 mg/mL |
| | Polysorbate 80 | 1.0 mg/mL |
| | Sodium citrate dihydrate | 0.305 mg/mL |
| | Sodium chloride | 6.165 mg/mL |
| | Sodium hydroxide | up to pH 5.2 |
| Humira 2 | Mannitol | 42.0 mg/mL |
| | Polysorbate 80 | 1.0 mg/mL |
| Acet, pH 5.5 | Sodium acetate trihydrate | 0.436 mg/mL |
| | Glacial acetic acid | up to pH 5.5 |
| Acet + NaCl, pH 5.5 | Sodium acetate trihydrate | 0.436 mg/mL |
| | Glacial acetic acid | up to pH 5.5 |
| | Sodium chloride | 9 mg/mL |
| Acet + Tre, pH 5.5 | Sodium acetate trihydrate | 0.436 mg/mL |
| | Glacial acetic acid | up to pH 5.5 |
| | Trehalose dihydrate | 100 mg/mL |
| Acet + Suc, pH 5.5 | Sodium acetate trihydrate | 0.436 mg/mL |
| | Glacial acetic acid | up to pH 5.5 |
| | Sucrose | 100 mg/mL |
| Acet + Mannitol, pH 5.5 | Sodium acetate trihydrate | 0.436 mg/mL |
| | Glacial acetic acid | up to pH 5.5 |
| | Mannitol | 50 mg/mL |
| Acet + Sorb, pH 5.5 | Sodium acetate trihydrate | 0.436 mg/mL |
| | Glacial acetic acid | up to pH 5.5 |
| | Sorbitol | 50 mg/mL |
| Acet + 100mM Gly, pH 5.5 | Sodium acetate trihydrate | 0.436 mg/mL |
| | Glacial acetic acid | up to pH 5.5 |
| | Glycine | 7.5 mg/mL |
| Acet + 50 mM Arg, pH 5.5 | Sodium acetate trihydrate | 0.436 mg/mL |
| | Glacial acetic acid | up to pH 5.5 |
| | Arginine hydrochloride | 10.5 mg/mL |

(continued)

| | | |
|---|---|---|
| Acet + 10 mM Meth, pH 5.5 | Sodium acetate trihydrate<br>Glacial acetic acid<br>Methionine | 0.436 mg/mL<br>up to pH 5.5<br>1.5 mg/mL |
| Acet + 250 mM Prol, pH 5.5 | Sodium acetate trihydrate<br>Glacial acetic acid<br>L-Proline | 0.436 mg/mL<br>up to pH 5.5<br>27.0 mg/mL |
| Acet + 10 mM EDTA, pH 5.5 | Sodium acetate trihydrate<br>Glacial acetic acid<br>EDTA disodium salt dihydrate | 0.436 mg/mL<br>up to pH 5.5<br>3.72 mg/mL |
| Acet + 100mM Lys, pH 5.5 | Sodium acetate trihydrate<br>Glacial acetic acid<br>Lysine | 0.436 mg/mL<br>up to pH 5.5<br>14.6 mg/mL |
| Acet + 100mM Guan, pH 5.5 | Sodium acetate trihydrate<br>Glacial acetic acid<br>Guanidine hydrochloride | 0.436 mg/mL<br>up to pH 5.5<br>9.55 mg/mL |
| Acet + 100mM Glu, pH 5.5 | Sodium acetate trihydrate<br>Glacial acetic acid<br>Glucose monohydrate | 0.436 mg/mL<br>up to pH 5.5<br>19.82 mg/mL |
| Acet + 100mM Lact, pH 5.5 | Sodium acetate trihydrate<br>Glacial acetic acid<br>Lactose monohydrate | 0.436 mg/mL<br>up to pH 5.5<br>36.03 mg/mL |
| Acet + 100mM Mannose, pH 5.5 | Sodium acetate trihydrate<br>Glacial acetic acid<br>Mannose | 0.436 mg/mL<br>up to pH 5.5<br>18.02 mg/mL |
| Acet + Tre + 0. Cyst, pH 5.5 | Sodium acetate trihydrate<br>Glacial acetic acid<br>Trehalose dihydrate<br>Cysteine hydrochloride monohydrate | 0.436 mg<br>up to pH 5.5<br>100 mg<br>0.088 mg |
| Acet + Tre + 2. Cyst, pH 5.5 | Sodium acetate trihydrate<br>Glacial acetic acid<br>Trehalose dihydrate<br>Cysteine hydrochloride monohydrate | 0.436 mg<br>up to pH 5.5<br>100 mg<br>0.44 mg |
| Acet + 10mM Cyst, pH 5.5 | Sodium acetate trihydrate<br>Glacial acetic acid<br>Cysteine hydrochloride monohydrate | 0.436 mg/mL<br>up to pH 5.5<br>1.76 mg/mL |
| Acet + Tween 20 0.5, pH 5.5 | Sodium acetate trihydrate<br>Glacial acetic acid<br>Polysorbate 20 | 0.436 mg/mL<br>up to pH 5.5<br>0.5 mg/mL |
| Acet + Tween20 1.0, pH 5.5 | Sodium acetate trihydrate<br>Glacial acetic acid<br>Polysorbate 20 | 0.436 mg/mL<br>up to pH 5.5<br>1.0 mg/mL |
| Acet + Tween80 0.5, pH 5.5 | Sodium acetate trihydrate<br>Glacial acetic acid<br>Polysorbate 80 | 0.436 mg/mL<br>up to pH 5.5<br>0.5 mg/mL |

(continued)

| | Sodium acetate trihydrate | 0.436 mg/mL |
| Acet + Tween80 1.0, pH 5.5 | Glacial acetic acid | up to pH 5.5 |
| | Polysorbate 80 | 1.0 mg/mL |
| | Sodium acetate trihydrate | 0.436 mg/mL |
| Acet + Pol.188 0.5, pH 5.5 | Glacial acetic acid | up to pH 5.5 |
| | Poloxamer 188 | 0.5 mg/mL |
| | Sodium acetate trihydrate | 0.436 mg/mL |
| Acet + Pol.188 1.0, pH 5.5 | Glacial acetic acid | up to pH 5.5 |
| | Poloxamer 188 | 1.0 mg/mL |

3.1. Colloidal Stability Determination by PEG Aggregation.

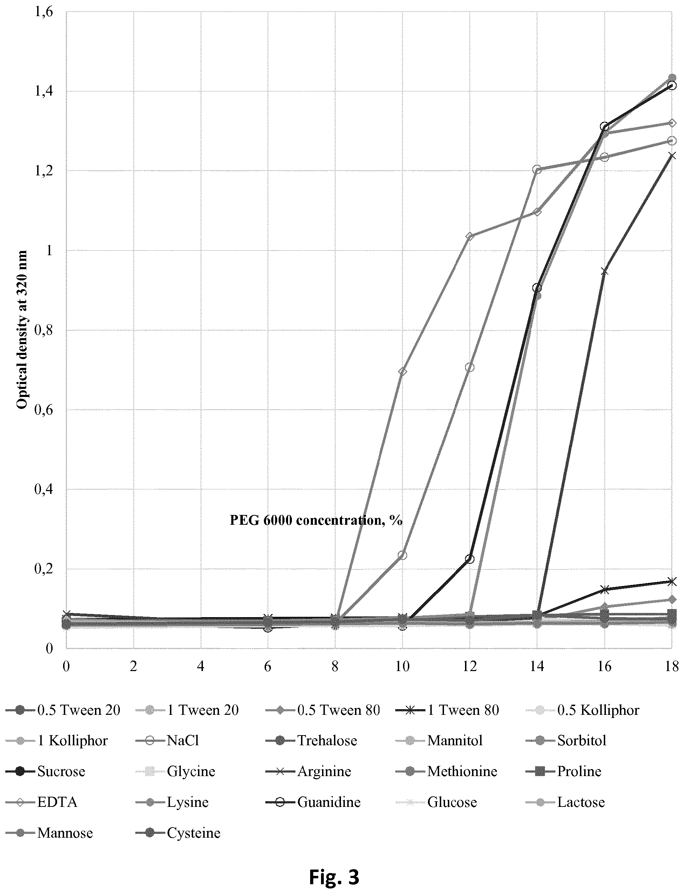

The "PEG aggregation" test was performed in triplicate for each sample. The analysis was performed according to the procedure 3. The data on the average optical density of the solutions are presented in Table 6. The results are also shown in Fig. 3.

Table 6. The average optical density of the adalimumab solutions after preparation.

| PEG% | 0.5 Tween 20 | 1 Tween 20 | 0.5 Tween 80 | 1 Tween 80 | 0.5 Pol.188 | 1 Pol.188 | NaCl | Tre | Mann | Sorb | Suc |
|---|---|---|---|---|---|---|---|---|---|---|---|
| 0 | 0.0648 | 0.0638 | 0.0714 | 0.0733 | 0.0588 | 0.0599 | 0.0737 | 0.0599 | 0.0699 | 0.0599 | 0.0621 |
| 6 | 0.0632 | 0.0654 | 0.0687 | 0.0755 | 0.0552 | 0.0566 | 0.0570 | 0.0638 | 0.0664 | 0.0614 | 0.0649 |
| 8 | 0.0620 | 0.0658 | 0.0654 | 0.0769 | 0.0590 | 0.0565 | 0.0622 | 0.0661 | 0.0652 | 0.0582 | 0.0646 |
| 10 | 0.0674 | 0.0670 | 0.0700 | 0.0776 | 0.0644 | 0.0669 | 0.2339 | 0.0630 | 0.0600 | 0.0615 | 0.0696 |
| 12 | 0.0649 | 0.0671 | 0.0704 | 0.0754 | 0.0650 | 0.0673 | 0.7066 | 0.0663 | 0.0637 | 0.0611 | 0.0700 |
| 14 | 0.0660 | 0.0709 | 0.0704 | 0.0819 | 0.0668 | 0.0710 | 1.2027 | 0.0733 | 0.0633 | 0.0618 | 0.0727 |
| 16 | 0.0663 | 0.0703 | 0.1049 | 0.1479 | 0.0687 | 0.0660 | 1.2339 | 0.0660 | 0.0620 | 0.0623 | 0.0733 |
| 18 | 0.0674 | 0.0707 | 0.1230 | 0.1681 | 0.0689 | 0.0773 | 1.2754 | 0.0621 | 0.0658 | 0.0620 | 0.0760 |

| PEG % | Gly | Arg | Meth | Prol | EDTA | Lys | Guan | Glu | Lact | Mannose | 10Cyst |
|---|---|---|---|---|---|---|---|---|---|---|---|
| 0 | 0.0611 | 0.0863 | 0.0581 | 0.0650 | 0.0561 | 0.0698 | 0.0636 | 0.0529 | 0.0693 | 0.0582 | 0.0616 |
| 6 | 0.0613 | 0.0613 | 0.0567 | 0.0696 | 0.0573 | 0.0697 | 0.0525 | 0.0565 | 0.0610 | 0.0601 | 0.0650 |
| 8 | 0.0646 | 0.0614 | 0.0620 | 0.0722 | 0.0584 | 0.0723 | 0.0606 | 0.0563 | 0.0623 | 0.0613 | 0.0670 |
| 10 | 0.0682 | 0.0700 | 0.0676 | 0.0734 | 0.6958 | 0.0765 | 0.0565 | 0.0573 | 0.0650 | 0.0633 | 0.0733 |
| 12 | 0.0710 | 0.0687 | 0.0645 | 0.0798 | 1.0355 | 0.0855 | 0.2242 | 0.0593 | 0.0663 | 0.0612 | 0.0708 |
| 14 | 0.0726 | 0.0769 | 0.0657 | 0.0837 | 1.0964 | 0.8867 | 0.9060 | 0.0611 | 0.0663 | 0.0638 | 0.0836 |
| 16 | 0.0739 | 0.9483 | 0.0676 | 0.0863 | 1.2935 | 1.2942 | 1.3112 | 0.0618 | 0.0668 | 0.0630 | 0.0758 |
| 18 | 0.0777 | 1.2383 | 0.0775 | 0.0869 | 1.3199 | 1.4338 | 1.4138 | 0.0585 | 0.0673 | 0.0657 | 0.0728 |

▦ There is visible aggregation

3.2. Thermal Stability Determination by Protein Aggregation Point using Dynamic Light Scattering (DLS) Technique.

The analysis was performed according to the procedure 4.

3.3. Thermal Stability Determination under Long-Term Exposure using Thermostress 50°C Method.

The analysis was performed according to the procedure 5.

3.4. Results.

**Table 7.** Summary on the sample quality measures before and after stress.

| | Protein C, mg/mL | Thermal stress 50°C, 72 h | | | | Aggregation temperature, °C | Aggreg. at % PEG 6000 | Conclusion |
|---|---|---|---|---|---|---|---|---|
| | | Change in purity, %* | Increase in fragments, %* | Turbidity, OD 400 nm | Acid-base profile Δ abs. %** | | | |
| | | SE-HPLC | | SP type | Labchip Caliper | | | |
| Humira 1 | 5 | -1.10 | 0.94 | 0.0544 | 21.44 | 69.5*** | 8 | |
| Humira 2 | 5 | -0.18 | 0.10 | 0.0487 | 18.38 | 72.5 | > 18 | |
| Acet | 5 | -0.46 | 0.43 | 0.0464 | 17.01 | 74.0 | 16 | |
| Acet + NaCl | 5 | -2.03 | 1.86 | 0.0457 | 22.05 | 66.5*** | 10 | |
| Acet + Tre | 5 | -0.28 | 0.15 | 0.0581 | 14.55 | 75.5 | > 18 | + |
| Acet + Suc | 5 | -0.46 | 0.34 | 0.0505 | 19.78 | 74.0 | > 18 | |
| Acet + Mannitol | 5 | -0.48 | 0.30 | 0.0521 | 19.03 | 75.5 | > 18 | |
| Acet + Sorb | 5 | -0.53 | 0.34 | 0.0574 | 20.78 | 75.5 | > 18 | |
| Acet + 100mM Gly | 5 | -0.39 | 0.20 | 0.0457 | 20.70 | 74.0 | > 18 | + |
| Acet + 50 mM Arg | 5 | -1.31 | 1.15 | 0.0497 | 14.90 | 69.5*** | 16 | + |
| Acet + 10 mM Meth | 5 | -0.42 | 0.3\ | 0.0432 | 16.27 | - | > 18 | + |
| Acet + 250 mM Prol | 5 | -0.38 | 0.27 | 0.0448 | 15.43 | 74.0 | > 18 | + |
| Acet + 10 mM EDTA | 5 | -1.24 | 0.72 | 0.0488 | 12.08 | 68.0*** | 10 | |
| Acet + 100mM Lys | 5 | -3.26 | 2.19 | 0.0424 | 17.67 | 68.0*** | 14 | |

| | Protein C, mg/mL | Thermal stress 50°C, 72 h | | | | Aggregation temperature, °C | Aggreg. at % PEG 6000 | Conclusion |
|---|---|---|---|---|---|---|---|---|
| | | Change in purity, %* | Increase in fragments, %* | Turbidity, OD 400 nm | Acid-base profile Δ abs. %** | | | |
| | | SE-HPLC | | SP type | Labchip Caliper | | | |
| Acet + 100mM Guan | 5 | -3.98 | 2.36 | 0.0480 | 17.51 | 66.5*** | 12 | |
| Acet + 100mM Glu | 5 | -1.60 | 1.54 | 0.0496 | 55.64 | 74.0 | > 18 | |
| Acet + 100mM Lact | 5 | -1.86 | 1.60 | 0.0457 | 29.43 | 74.0 | > 18 | |
| Acet + 100mM Mannose | 5 | -1.73 | 1.57 | 0.0450 | 44.16 | 74.0 | > 18 | |

| | Protein C, mg/mL | SE-HPLC | | Turbidity, OD 400 nm | Acid-base profile Δ abs. %** | Aggregation temperature, °C | Aggreg. at % PEG 6000 | Conclusion |
|---|---|---|---|---|---|---|---|---|
| **Thermal stress 50°C, 72 h** | | Change in purity, %* | Increase in fragments, %* | SP type | Labchip Caliper | | | |
| Acet + 0. Cyst | 5 | -5.08 | 3.55 | 0.0425 | - | - | - | |
| Acet + 2. Cyst | 5 | -8.99 | 5.78 | 0.0440 | - | - | - | |
| Acet + 10mM Cyst | 5 | -100 | 93.20 | 0.0467 | - | - | > 18 | |
| Acet + Tween20 0.5 | 5 | - | - | 0.0425 | 19.25 | 74.0*** | > 18 | + |
| Acet + Tween20 1.0 | 5 | -0.41 | 0.33 | 0.0452 | 20.72 | 74.0*** | > 18 | + |
| Acet + Tween80 0.5 | 5 | -0.46 | 0.39 | 0.0475 | 19.52 | 72.5*** | > 18 | + |
| Acet + Tween80 1.0 | 5 | -0.48 | 0.31 | 0.0436 | 17.36 | 72.5*** | > 18 | + |
| Acet + Koll 0.5 | 5 | -0.31 | 0.30 | 0.0452 | 17.65 | 74.0 | > 18 | + |
| Acet + Koll 1.0 | 5 | -0.40 | 0.44 | 0.0487 | 19.46 | 74.0 | > 18 | + |

* Change was calculated by the formula Δ = (Value after stress − Value before stress) ** Absolute change was calculated by the formula Δ = |Content of acid fraction before stress − Content of acid fraction after stress| + |Content of basic fraction before stress − Content of basic fraction after stress| + |Content of dominant fraction before stress − Content of dominant fraction after stress|
*** There is a marked aggregation upon heating (dramatic improvement in particle size and scattered light intensity > 10,000 kcps).

■ positive results    ▨ negative results    □ average results/baseline data

3.5. Conclusions from Example 3.

Based on the results of the present study, promising additives for adalimumab compositions are trehalose dihydrate, glycine, proline, methionine, and arginine hydrochloride. Polysorbate 80, Polysorbate 20, Poloxamer 188 were taken as surface-active substances for the final pharmaceutical composition screening.

Sodium chloride, arginine, EDTA, lysine, guanidine have an adverse impact on the colloidal stability of adalimumab.

Sodium chloride, lysine, guanidine have an adverse impact on the thermal stability of adalimumab. Cysteine-containing in the formulation leads to complete fragmentation of the antibody. A shift in the protein acid-base profile was noted when EDTA additive was added to the formulation.

Example 4. Final Pharmaceutical Composition Selection.

Based on the results of the third part of the study, trehalose dihydrate, glycine, proline, methionine, and arginine hydrochloride were chosen as promising additives. Polysorbate 80, Polysorbate 20, Poloxamer 188 were taken as surface-active substances for the final pharmaceutical composition screening.

Study formulation (mg/mL).

| # | Buffer solution | Protein conc. | Trehalose dihydrate | L-Proline | Glycine | Methionine | Polysorbate 80 | Polysorbate 20 | Poloxamer 188 | Arginine hydrochloride |
|---|---|---|---|---|---|---|---|---|---|---|
| 1 | | 15 | Humira formulation 1 (see item 1) | | | | | | | |
| 2 | | 15 | Humira formulation 2 (see item 1) | | | | | | | |
| 3 | | 15 | 80 | | | | | | | |
| 4 | | 15 | 80 | | | | 0.1 | | | |
| 5 | | 15 | 80 | | | | 0.5 | | | |
| 6 | | 15 | 80 | | | | 1.0 | | | |
| 7 | Acetate | 15 | 80 | | | | | 0.1 | | |
| 8 | buffer, pH | 15 | 80 | | | | | 1.0 | | |
| 9 | 5.5 | 15 | 80 | | | | | | 0.1 | |
| 10 | | 15 | 80 | | | | | | 0.5 | |
| 11 | | 15 | 80 | | | | | | 1.0 | |
| 12 | | 15 | 80 | | | | | | | 0.5 |
| 13 | | 15 | 80 | | | | 0.1 | | | 0.5 |
| 14 | Acetate | 15 | 80 | | | | 0.5 | | | 0.5 |
| 15 | buffer, pH | 15 | 80 | | | | 1.0 | | | 0.5 |
| 16 | 5.5 | 15 | 80 | | | | | 0.1 | | 0.5 |

| # | Buffer solution | Protein conc. | Trehalose dihydrate | L-Proline | Glycine | Methionine | Polysorbate 80 | Polysorbate 20 | Poloxamer 188 | Arginine hydrochloride |
|---|---|---|---|---|---|---|---|---|---|---|
| 17 | | 15 | 80 | | | | | 1.0 | | 0.5 |
| 18 | | 15 | 80 | | | | | | 0.1 | 0.5 |
| 19 | | 15 | 80 | | | | | | 0.5 | 0.5 |
| 20 | | 15 | 80 | | | | | | 1.0 | 0.5 |
| 21 | | 15 | | 27 | | | | | | |
| 22 | | 15 | | 27 | | | 0.1 | | | |
| 23 | | 15 | | 27 | | | 0.5 | | | |
| 24 | | 15 | | 27 | | | 1.0 | | | |
| 25 | | 15 | | 27 | | | | 0.1 | | |
| 26 | | 15 | | 27 | | | | 1.0 | | |
| 27 | | 15 | | 27 | | | | | 0.1 | |
| 28 | | 15 | | 27 | | | | | 0.5 | |
| 29 | | 15 | | 27 | | | | | 1.0 | |
| 30 | | 15 | | 8.0 | 7.5 | | | | | |
| 31 | | 15 | | 8.0 | 7.5 | | 0.1 | | | |
| 32 | | 15 | | 8.0 | 7.5 | | 0.5 | | | |
| 33 | | 15 | | 8.0 | 7.5 | | 1.0 | | | |
| 34 | | 15 | | 8.0 | 7.5 | | | 0.1 | | |
| 35 | | 15 | | 8.0 | 7.5 | | | 1.0 | | |
| 36 | | 15 | | 8.0 | 7.5 | | | | 0.1 | |
| 37 | | 15 | | 8.0 | 7.5 | | | | 0.5 | |
| 38 | | 15 | | 8.0 | 7.5 | | | | 1.0 | |
| 39 | | 15 | | 27 | | 1.5 | | | | |
| 40 | | 15 | | 27 | | 1.5 | 0.1 | | | |
| 41 | | 15 | | 27 | | 1.5 | 0.5 | | | |
| 42 | | 15 | | 27 | | 1.5 | 1.0 | | | |
| 43 | | 15 | | 27 | | 1.5 | | 0.1 | | |
| 44 | | 15 | | 27 | | 1.5 | | 1.0 | | |
| 45 | | 15 | | 27 | | 1.5 | | | 0.1 | |
| 46 | | 15 | | 27 | | 1.5 | | | 0.5 | |
| 47 | | 15 | | 27 | | 1.5 | | | 1.0 | |

| # | Buffer solution | Protein conc. | Trehalose dihydrate | L-Proline | Glycine | Methionine | Polysorbate 80 | Polysorbate 20 | Poloxamer 188 | Arginine hydrochloride |
|---|---|---|---|---|---|---|---|---|---|---|
| 48 | | 15 | 40 | 13.5 | | | | | | |
| 49 | | 15 | 53.3 | 9.0 | | | | | | |
| 50 | | 15 | 26.7 | 18 | | | | | | |

4.1. Thermal Stability Determination under Long-Term Exposure using Thermostress 50°C Method.

The analysis was performed according to the procedure 5.

4.2. Colloidal Stability Determination by "Shake-Test" Technique.

The analysis was performed according to the procedure 6.

4.3. Colloidal Stability Determination by Cryoconcentration Technique.

The analysis was performed according to the procedure 7.

Summary is provided in Tables 8 and 9, where the sample quality measures before and after the thermal stress, shake test and cryoconcentration are demonstrated.

4.4. Results.

Table 8. Summary on SE-HPLC and UV-spectrophotometry of the samples before and after stress.

| # | anti TNFα | Trehalose dihydrate | L-Proline | Glycine | Methionine | Polysorbate 80 | Polysorbate 20 | Poloxamer 188 | Arginine hydrochloride | Thermal stress, 50°C,120 h | Freezing -18°C, thawing +25°C | Shake test 120 h | Shake test 120 h | Freezing -18°C, thawing +25°C |
|---|---|---|---|---|---|---|---|---|---|---|---|---|---|---|
| | | | | | | | | | | Change in purity SE-HPLC, %* | | | Turbidity, OD 400 nm | |
| 1 | 15 | Humira formulation 1 (see item 1) | | | | | | | | -1.12 | 0.02 | 0.03 | 0.0410 | 0.0501 |
| 2 | 15 | Humira formulation 2 (see item 1) | | | | | | | | -0.38 | -7.49 | 0.02 | 0.0408 | 0.0503 |
| 3 | 15 | 80 | | | | | | | | -0.33 | 0.04 | 0.03 | 0.0409 | 0.0521 |
| 4 | 15 | 80 | | | | 0.1 | | | | -0.34 | -0.09 | -0.09 | 0.0446 | 0.0479 |
| 5 | 15 | 80 | | | | 0.5 | | | | -0.35 | -0.04 | -0.02 | 0.0406 | 0.0531 |
| 6 | 15 | 80 | | | | 1.0 | | | | -0.38 | -0.04 | -0.21 | 0.0407 | 0.0489 |
| 7 | 15 | 80 | | | | | 0.1 | | | -0.39 | 0.05 | 0.07 | 0.0413 | 0.0474 |
| 8 | 15 | 80 | | | | | 1.0 | | | -0.44 | 0.07 | -0.01 | 0.0454 | 0.0465 |
| 9 | 15 | 80 | | | | | | 0.1 | | -0.54 | 0.02 | -0.07 | 0.0465 | 0.0494 |
| 10 | 15 | 80 | | | | | | 0.5 | | -0.45 | -0.09 | -0.18 | 0.0465 | 0.0516 |
| 11 | 15 | 80 | | | | | | 1.0 | | -0.56 | -0.04 | 0.05 | 0.0412 | 0.0494 |
| 12 | 15 | 80 | | | | | | | 0.1 | -0.40 | -0.03 | 0.08 | 0.0448 | 0.0446 |
| 13 | 15 | 80 | | | | 0.1 | | | 0.1 | -0.44 | 0.03 | -0.07 | 0.0479 | 0.0468 |
| 14 | 15 | 80 | | | | 0.5 | | | 0.1 | -0.46 | -0.09 | -0.09 | 0.0445 | 0.0510 |
| 15 | 15 | 80 | | | | 1.0 | | | 0.1 | -0.49 | -0.01 | 0.07 | 0.0434 | 0.0512 |
| 16 | 15 | 80 | | | | | 0.1 | | 0.1 | -0.47 | 0.05 | 0.08 | 0.0445 | 0.0455 |

| # | Additive formulation, mg/mL | | | | | | | | | Change in purity SE-HPLC, %* | | | Turbidity, OD 400 nm | |
|---|-------|-------------------|----------|--------|-------------|----------------|----------------|----------------|------------------------|--------------------------|----------------------------------|------------------|------------------|----------------------------------|
| | anti TNFα | Trehalose dihydrate | L-Proline | Glycine | Methionine | Polysorbate 80 | Polysorbate 20 | Poloxamer 188 | Arginine hydrochloride | Thermal stress, 50°C,120 h | Freezing -18°C, thawing +25°C | Shake test 120 h | Shake test 120 h | Freezing -18°C, thawing +25°C |
| 17 | 15 | 80 | | | | | 1.0 | | 0.1 | -0.33 | 0.04 | 0.07 | 0.0478 | 0.0444 |
| 18 | 15 | 80 | | | | | | 0.1 | 0.1 | -0.46 | 0.09 | 0.05 | 0.0497 | 0.0447 |
| 19 | 15 | 80 | | | | | | 0.5 | 0.1 | -0.48 | -0.01 | 0.01 | 0.0441 | 0.0518 |
| 20 | 15 | 80 | | | | | | 1.0 | 0.1 | -0.40 | -0.01 | 0.03 | 0.0434 | 0.0470 |

| # | anti TNFα | Trehalose dihydrate | L-Proline | Glycine | Methionine | Polysorbate 80 | Polysorbate 20 | Poloxamer 188 | Arginine hydrochloride | Thermal stress, 50°C,120 h | Freezing -18°C, thawing +25°C | Shake test 120 h | Shake test 120 h | Freezing -18°C, thawing +25°C |
|---|---|---|---|---|---|---|---|---|---|---|---|---|---|---|
| | | | Additive formulation, mg/mL | | | | | | | Change in purity SE-HPLC, %* | | | Turbidity, OD 400 nm | |
| 21 | 15 | | 27 | | | | | | | -0.36 | 0.00 | 0.02 | 0.0400 | 0.0409 |
| 22 | 15 | | 27 | | | 0.1 | | | | -0.51 | -0.03 | -0.06 | 0.0440 | 0.0475 |
| 23 | 15 | | 27 | | | 0.5 | | | | -0.78 | -0.03 | -0.04 | 0.0464 | 0.0745 |
| 24 | 15 | | 27 | | | 1.0 | | | | -0.39 | 0.03 | 0.01 | 0.0445 | 0.0443 |
| 25 | 15 | | 27 | | | | 0.1 | | | -0.46 | -0.01 | 0.08 | 0.0412 | 0.0457 |
| 26 | 15 | | 27 | | | | 1.0 | | | -0.48 | -0.07 | -0.07 | 0.0434 | 0.0476 |
| 27 | 15 | | 27 | | | | | 0.1 | | -0.66 | -0.09 | 0.00 | 0.0445 | 0.0449 |
| 28 | 15 | | 27 | | | | | 0.5 | | -0.36 | -0.04 | 0.06 | 0.0410 | 0.0469 |
| 29 | 15 | | 27 | | | | | 1.0 | | -0.39 | 0.02 | 0.06 | 0.0409 | 0.0501 |
| 30 | 15 | | 8.0 | 7.5 | | | | | | -0.46 | -0.02 | 0.00 | 0.0402 | 0.0467 |
| 31 | 15 | | 8.0 | 7.5 | | 0.1 | | | | -0.48 | -0.05 | -0.01 | 0.0403 | 0.0439 |
| 32 | 15 | | 8.0 | 7.5 | | 0.5 | | | | -0.40 | -0.04 | -0.01 | 0.0412 | 0.0496 |
| 33 | 15 | | 8.0 | 7.5 | | 1.0 | | | | -0.20 | 0.02 | 0.08 | 0.0555 | 0.0474 |
| 34 | 15 | | 8.0 | 7.5 | | | 0.1 | | | -0.46 | -0.01 | -0.03 | 0.0585 | 0.0479 |
| 35 | 15 | | 8.0 | 7.5 | | | 1.0 | | | -0.37 | 0.01 | -0.02 | 0.0565 | 0.0443 |
| 36 | 15 | | 8.0 | 7.5 | | | | 0.1 | | -0.49 | -0.02 | 0.04 | 0.0408 | 0.0457 |
| 37 | 15 | | 8.0 | 7.5 | | | | 0.5 | | -0.36 | -0.01 | 0.06 | 0.0478 | 0.0464 |
| 38 | 15 | | 8.0 | 7.5 | | | | 1.0 | | -0.31 | 0.00 | 0.03 | 0.0432 | 0.0498 |

| # | Additive formulation, mg/mL | | | | | | | | | Change in purity SE-HPLC, %* | | | Turbidity, OD 400 nm | |
| | anti TNFα | Trehalose dihydrate | L-Proline | Glycine | Methionine | Polysorbate 80 | Polysorbate 20 | Poloxamer 188 | Arginine hydrochlorid | Thermal stress, 50°C,120 h | Freezing -18°C, thawing +25°C | Shake test 120 h | Shake test 120 h | Freezing -18°C, thawing +25°C |
| 39 | 15 | | 27 | | 1.5 | | | | | -0.36 | 0.01 | -0.01 | 0.0512 | 0.0463 |
| 40 | 15 | | 27 | | 1.5 | 0.1 | | | | -0.38 | 0.06 | 0.05 | 0.0509 | 0.0503 |
| 41 | 15 | | 27 | | 1.5 | 0.5 | | | | -0.36 | -0.01 | 0.08 | 0.0549 | 0.0417 |
| 42 | 15 | | 27 | | 1.5 | 1.0 | | | | -0.30 | 0.01 | 0.04 | 0.0520 | 0.0473 |

EP 3 563 867 A1

| # | Additive formulation, mg/mL | | | | | | | | | Change in purity SE-HPLC, %* | | | Turbidity, OD 400 nm | |
|---|---|---|---|---|---|---|---|---|---|---|---|---|---|---|
| | anti TNFα | Trehalose dihydrate | L-Proline | Glycine | Methionine | Polysorbate 80 | Polysorbate 20 | Poloxamer 188 | Arginine hydrochlorid | Thermal stress, 50°C,120 h | Freezing -18°C, thawing +25°C | Shake test 120 h | Shake test 120 h | Freezing -18°C, thawing +25°C |
| 43 | 15 | | 27 | | 1.5 | | 0.1 | | | -0.37 | 0.00 | -0.08 | 0.0512 | 0.0444 |
| 44 | 15 | | 27 | | 1.5 | | 1.0 | | | -0.32 | -0.03 | 0.03 | 0.0513 | 0.0465 |
| 45 | 15 | | 27 | | 1.5 | | | 0.1 | | -0.33 | -0.01 | -0.03 | 0.0510 | 0.0411 |
| 46 | 15 | | 27 | | 1.5 | | | 0.5 | | -0.36 | -0.02 | -0.02 | 0.0550 | 0.0477 |
| 47 | 15 | | 27 | | 1.5 | | | 1.0 | | -0.32 | 0.01 | -0.03 | 0.0552 | 0.0498 |
| 48 | 15 | 40 | 13.5 | | | | | | | -0.50 | -0.09 | -0.08 | 0.0494 | 0.0469 |
| 49 | 15 | 53.3 | 9.0 | | | | | | | -0.55 | -0.01 | 0.03 | 0.0414 | 0.0464 |
| 50 | 15 | 26.7 | 18 | | | | | | | -0.59 | -0.02 | -0.03 | 0.0410 | 0.0466 |

* Change was calculated by the formula $\Delta = $ (Value after stress $-$ Value before stress)

▮ positive results   ▯ negative results   ☐ average results/baseline data

**Table 9.** Summary on the acid-base profile of the samples on the LabChip instrument and the homogeneity using the dynamic light scattering technique before and after stress.

| # | anti TNFα | Trehalose dihydrate | L-Proline | Glycine | Methionine | Polysorbate 80 | Polysorbate 20 | Poloxamer 188 | Arginine | Homogenicity DLS, % (by intensity) | Thermal stress, 50°C, 120 h | Shake test 120 h | Freezing -18°C, thawing +25°C | Thermal stress, +50°C 120 h | Shake test 120 h | Freezing -18°C, thawing +25°C |
|---|---|---|---|---|---|---|---|---|---|---|---|---|---|---|---|---|
| | | | Additive formulation, mg/mL | | | | | | | | Homogenicity DLS after stresses, % | | | Total change in acid-base profile, in modulus**, % | | |
| 1 | 15 | Humira formulation 1 (see item 1) | | | | | | | | 100 | 99.6 | 100 | 100 | 33.16 | 1.13 | 1.84 |
| 2 | 15 | Humira formulation 2 (see item 1) | | | | | | | | 98.8 | 96.0 | 100 | 80.1 | 29.55 | 1.03 | 2.88 |
| 3 | 15 | 80 | | | | | | | | 100 | 100 | 93.3 | 100 | 24.56 | 2.88 | 2.46 |
| 4 | 15 | 80 | | | | 0.1 | | | | 100 | 100 | 93.6 | 100 | 27.26 | | |
| 5 | 15 | 80 | | | | 0.5 | | | | 100 | 100 | 94.8 | 100 | 28.27 | 1.09 | 2.82 |
| 6 | 15 | 80 | | | | 1.0 | | | | 100 | 100 | 96.9 | 100 | 27.64 | | |
| 7 | 15 | 80 | | | | | 0.1 | | | 100 | 100 | 97.0 | 100 | 24.10 | | |
| 8 | 15 | 80 | | | | | 1.0 | | | 100 | 98.5 | 98.8 | 100 | 26.17 | | |
| 9 | 15 | 80 | | | | | | 0.1 | | 100 | 100 | 98.6 | 100 | 26.26 | | |
| 10 | 15 | 80 | | | | | | 0.5 | | 100 | 100 | 95.1 | 100 | 26.98 | 1.44 | 3.55 |
| 11 | 15 | 80 | | | | | | 1.0 | | 100 | 97.6 | 98.9 | 100 | 26.13 | | |
| 12 | 15 | 80 | | | | | | | 0.1 | 100 | 100 | 99.0 | 100 | 24.88 | 0.88 | 3.10 |
| 13 | 15 | 80 | | | | 0.1 | | | 0.1 | 100 | 100 | 100 | 100 | 25.58 | 1.43 | 1.99 |
| 14 | 15 | 80 | | | | 0.5 | | | 0.1 | 100 | 100 | 100 | 100 | 26.16 | | |

EP 3 563 867 A1

| # | anti TNFα | Trehalose dihydrate | L-Proline | Glycine | Methionine | Polysorbate 80 | Polysorbate 20 | Poloxamer 188 | Arginine | Homogenicity DLS, % (by intensity) | Homogenicity DLS after stresses, % | | | Total change in acid-base profile, in modulus**, % | | |
|---|---|---|---|---|---|---|---|---|---|---|---|---|---|---|---|---|
| | | | | | | | | | | | Thermal stress, 50°C, 120 h | Shake test 120 h | Freezing -18°C, thawing +25°C | Thermal stress, +50°C 120 h | Shake test 120 h | Freezing -18°C, thawing +25°C |
| 15 | 15 | 80 | | | | 1.0 | | | 0.1 | 99.4 | 100 | 100 | 100 | 24.96 | | |
| 16 | 15 | 80 | | | | | 0.1 | | 0.1 | 100 | 100 | 100 | 100 | 28.10 | | |
| 17 | 15 | 80 | | | | | 1.0 | | 0.1 | 100 | 100 | 100 | 100 | 24.74 | | |

| # | anti TNFα | Trehalose dihydrate | L-Proline | Glycine | Methionine | Polysorbate 80 | Polysorbate 20 | Poloxamer 188 | Arginine | Homogenicity DLS, % (by intensity) | Homogenicity DLS after stresses, % | | | Total change in acid-base profile, in modulus**, % | | |
|---|---|---|---|---|---|---|---|---|---|---|---|---|---|---|---|---|
| | | | | | | | | | | | Thermal stress, 50°C, 120 h | Shake test 120 h | Freezing -18°C, thawing +25°C | Thermal stress, +50°C 120 h | Shake test 120 h | Freezing -18°C, thawing +25°C |
| 18 | 15 | 80 | | | | | | 0.1 | 0.1 | 100 | 100 | 100 | 100 | 24.50 | | |
| 19 | 15 | 80 | | | | | | 0.5 | 0.1 | 100 | 100 | 100 | 100 | 24.55 | 2.30 | 3.68 |
| 20 | 15 | 80 | | | | | | 1.0 | 0.1 | 100 | 100 | 90.0 | 100 | 24.64 | | |
| 21 | 15 | | 26.5 | | | | | | | 100 | 100 | 100 | 100 | 26.79 | 1.03 | 1.20 |
| 22 | 15 | | 26.5 | | | 0.1 | | | | 100 | 100 | 100 | 100 | 24.60 | | |
| 23 | 15 | | 26.5 | | | 0.5 | | | | 100 | 100 | 100 | 100 | 21.96 | 1.85 | 2.49 |
| 24 | 15 | | 26.5 | | | 1.0 | | | | 100 | 100 | 95.8 | 100 | 22.20 | | |
| 25 | 15 | | 26.5 | | | | 0.1 | | | 100 | 100 | 100 | 100 | 24.22 | | |
| 26 | 15 | | 26.5 | | | | 1.0 | | | 100 | 98.5 | 100 | 100 | 26.07 | | |
| 27 | 15 | | 26.5 | | | | | 0.1 | | 100 | 100 | 100 | 100 | 22.38 | | |
| 28 | 15 | | 26.5 | | | | | 0.5 | | 100 | 100 | 100 | 100 | 21.70 | 2.74 | 4.67 |
| 29 | 15 | | 26.5 | | | | | 1.0 | | 100 | 100 | 100 | 100 | 21.16 | | |
| 30 | 15 | | 8.0 | 7.5 | | | | | | 100 | 100 | 100 | 100 | 24.87 | 2.90 | 2.91 |
| 31 | 15 | | 8.0 | 7.5 | 0.1 | | | | | 100 | 100 | 100 | 100 | 29.76 | | |
| 32 | 15 | | 8.0 | 7.5 | 0.5 | | | | | 100 | 100 | 100 | 100 | 31.09 | 2.78 | 0.18 |
| 33 | 15 | | 8.0 | 7.5 | 1.0 | | | | | 100 | 100 | 100 | 100 | 29.96 | | |

44

| # | anti TNFα | Trehalose dihydrate | L-Proline | Glycine | Methionine | Polysorbate 80 | Polysorbate 20 | Poloxamer 188 | Arginine | Homogenicity DLS, % (by intensity) | Thermal stress, 50°C, 120 h | Shake test 120 h | Freezing -18°C, thawing +25°C | Thermal stress, +50°C 120 h | Shake test 120 h | Freezing -18°C, thawing +25°C |
|---|---|---|---|---|---|---|---|---|---|---|---|---|---|---|---|---|
| | | | | | | | Additive formulation, mg/mL | | | Homogenicity DLS, % (by intensity) | Homogenicity DLS after stresses, % | | | Total change in acid-base profile, in modulus**, % | | |
| 34 | 15 | | 8.0 | 7.5 | | | 0.1 | | | 100 | 100 | 100 | 100 | 27.30 | | |
| 35 | 15 | | 8.0 | 7.5 | | | 1.0 | | | 100 | 100 | 100 | 100 | 32.39 | | |
| 36 | 15 | | 8.0 | 7.5 | | | | 0.1 | | 100 | 100 | 100 | 100 | 31.69 | | |
| 37 | 15 | | 8.0 | 7.5 | | | | 0.5 | | 100 | 100 | 100 | 100 | 29.55 | 2.49 | 0.49 |
| 38 | 15 | | 8.0 | 7.5 | | | | 1.0 | | 100 | 100 | 100 | 100 | 30.00 | | |
| 39 | 15 | 26.5 | 1.5 | | | | | | | 100 | 100 | 100 | 100 | 28.52 | 1.44 | 1.62 |
| 40 | 15 | 26.5 | 1.5 | | 0.1 | | | | | 100 | 100 | 100 | 100 | 28.41 | | |
| 41 | 15 | 26.5 | 1.5 | | 0.5 | | | | | 100 | 100 | 100 | 100 | 28.16 | 1.09 | 0.79 |
| 42 | 15 | 26.5 | 1.5 | | 1.0 | | | | | 100 | 100 | 94.6 | 100 | 28.56 | | |
| 43 | 15 | 26.5 | 1.5 | | | | 0.1 | | | 100 | 100 | 92.0 | 100 | 33.88 | | |
| 44 | 15 | 26.5 | 1.5 | | | | 1.0 | | | 100 | 100 | 88.4 | 100 | 30.46 | | |
| 45 | 15 | 26.5 | 1.5 | | | | | 0.1 | | 100 | 100 | 95.4 | 100 | 26.82 | | |
| 46 | 15 | 26.5 | 1.5 | | | | | 0.5 | | 100 | 100 | 92.7 | 100 | 24.57 | 0.33 | 0.81 |
| 47 | 15 | 26.5 | 1.5 | | | | | 1.0 | | 100 | 100 | 100 | 100 | 28.22 | | |
| 48 | 15 | 40 | 13.5 | | | | | | | 100 | 100 | 90.7 | 100 | 26.85 | 1.74 | 0.77 |
| 49 | 15 | 53.3 | 9.0 | | | | | | | 100 | 100 | 100 | 100 | 26.58 | 1.33 | 4.37 |

| # | Additive formulation, mg/mL | | | | | | | | | Homogenicity DLS, % (by intensity) | Homogenicity DLS after stresses, % | | | Total change in acid-base profile, in modulus**, % | | |
|---|---|---|---|---|---|---|---|---|---|---|---|---|---|---|---|---|
| | anti TNFα | Trehalose dihydrate | L-Proline | Glycine | Methionine | Polysorbate 80 | Polysorbate 20 | Poloxamer 188 | Arginine | | Thermal stress, 50°C, 120 h | Shake test 120 h | Freezing -18°C, thawing +25°C | Thermal stress, +50°C 120 h | Shake test 120 h | Freezing -18°C, thawing +25°C |
| 50 | 15 | 26.7 | 18 | | | | | | | 100 | 100 | 99.9 | 100 | 26.73 | 0.42 | 5.87 |

* Change was calculated by the formula $\Delta = $ (Value after stress $-$ Value before stress)  ** Absolute change was calculated by the formula $\Delta = $ |Content of acid fraction before stress $-$ Content of acid fraction after stress| $+$ |Content of basic fraction before stress $-$ Content of basic fraction after stress| $+$ |Content of dominant fraction before stress $-$ Content of dominant fraction after stress|

▓ positive results          ▒ negative results          ☐ average results / baseline data

4.4. Conclusions from Example 4.

The study showed that adalimumab in the Humira formulation 1 is the most thermolabile among all the test sample. Thermostress at 50°C for 120 hours leads to the maximum increase in impurities (1.2%) on SE-HPLC, and to the maximum change in the acid-base profile (the total absolute change of all fractions is more than 33% on the LabChip Caliper instrument). The colloidal stability of the sample in the Humira formulation 1 is comparable to the alternative formulations.

The Humira formulation 2 has a thermal stability comparable with one of the alternative formulations (loss of purity during thermal stress 0.38%). However, upon freeze-thawing of adalimumab in the present formulation, there is a marked aggregation of the protein: the increase in aggregates in the Size Exclusion HPLC is more than 7%, which makes the preparation unusable in case of its accidental freezing. A marked increase in impurities after freezing was also noted using DLS.

Alternative formulations based on the acetate buffer solution with the addition of a number of additives showed good thermal and colloidal stability during stresses. Size Exclusion HPLC showed no difference in impurity increase in the present formulations. Also, there was no significant effect of surface-active substances on the stability of adalimumab at the concentration of 15 mg/mL.

Analysis of the stressed samples using DLS showed that the presence of arginine hydrochloride in the formulation reduces the impurity increase during the shake-test, and the presence of methionine increases the impurity accumulation during shaking.

Minimal change in the acid-base profile was noted for formulations 12-29 (formulations containing trehalose and arginine hydrochloride, and also containing L-proline). Based on the results of the present study, promising formulations for adalimumab are:

| | | | |
|---|---|---|---|
| Sodium acetate trihydrate | 0.436 mg/mL | Sodium acetate trihydrate | 0.436 mg/mL |
| Glacial acetic acid | up to pH 5.0-6.0 | Glacial acetic acid | up to pH 5.0-6.0 |
| Trehalose dihydrate | 80 mg/mL | Trehalose dihydrate | 80 mg/mL |
| | | Polysorbate 20 / Polysorbate 80 / Poloxamer 188 | 0.5 - 1.0 mg/mL |
| Sodium acetate trihydrate | 0.436 mg/mL | Sodium acetate trihydrate | 0.436 mg/mL |
| Glacial acetic acid | up to pH 5.0-6.0 | Glacial acetic acid | up to pH 5.0-6.0 |
| Trehalose dihydrate | 80 mg/mL | Trehalose dihydrate | 80 mg/mL |
| Arginine hydrochloride | 0.1 mg/mL | Arginine hydrochloride | 0.1 mg/mL |
| | | Polysorbate 20 / Polysorbate 80 / Poloxamer 188 | 0.5 - 1.0 mg/mL |
| Sodium acetate trihydrate | 0.436 mg/mL | Sodium acetate trihydrate | 0.436 mg/mL |
| Glacial acetic acid | up to pH 5.0-6.0 | Glacial acetic acid | up to pH 5.0-6.0 |
| L-Proline | 27 mg/mL | L-Proline | 27 mg/mL |
| | | Polysorbate 20 / Polysorbate 80 / Poloxamer 188 | 0.5 -1.0 mg/mL |

Example 5. Final High Concentrated Adalimumab Pharmaceutical Composition Confirmation.

To confirm the stability of the recommended pharmaceutical compositions, adalimumab at the concentration of 50 mg/mL, 100 mg/mL, and 150 mg/mL was exposed to a thermal stress at 50°C for 6 days.

Study Formulations.

| # | Buffer solution | Protein conc. | Trehalose dihydrate | L-Proline | Polysorbate 80 | Polysorbate 20 | Poloxamer 188 | Arginine hydrochlorid |
|---|---|---|---|---|---|---|---|---|
| 1 | | | Humira formulation 1 (see item 1) | | | | | |
| 2 | | | Humira formulation 2 (see item 1) | | | | | |
| 3 | | | 80 | | 0.5 | | | 0.1 |
| 4 | | | 80 | | 1.0 | | | 0.1 |
| 5 | | | 80 | | | 0.5 | | 0.1 |
| 6 | | | 80 | | | 1.0 | | 0.1 |
| 7 | | | 80 | | | | 0.5 | 0.1 |
| 8 | | 50 | 80 | | | | 1.0 | 0.1 |
| 9 | | | 80 | | 0.5 | | 0.5 | 0.1 |
| 10 | | | | 27 | 0.5 | | | |
| 11 | Acetate | | | 27 | 1.0 | | | |
| 12 | buffer, | | | 27 | | 0.5 | | |
| 13 | pH 5.0-6.0 | | | 27 | | 1.0 | | |
| 14 | | | | 27 | | | 0.5 | |
| 15 | | | | 27 | | | 1.0 | |
| 16 | | | | 27 | 0.5 | | 0.5 | |
| 17 | | | Humira formulation 1 (see item 1) | | | | | |
| 18 | | | Humira formulation 2 (see item 1) | | | | | |
| 19 | | | 80 | | 0.5 | | | 0.1 |
| 20 | | 100 | 80 | | 1.0 | | | 0.1 |
| 21 | | | 80 | | | 0.5 | | 0.1 |
| 22 | | | 80 | | | 1.0 | | 0.1 |
| 23 | | | 80 | | | | 0.5 | 0.1 |

| # | Buffer solution | Protein conc. | Trehalose dihydrate | L-Proline | Polysorbate 80 | Polysorbate 20 | Poloxamer 188 | Arginine hydrochlorid |
|---|---|---|---|---|---|---|---|---|
| 24 | | | 80 | | | | 1.0 | 0.1 |
| 25 | | | 80 | | 0.5 | | 0.5 | 0.1 |
| 26 | | | | 27 | 0.5 | | | |
| 27 | | | | 27 | 1.0 | | | |
| 28 | | | | 27 | | 0.5 | | |
| 29 | | | | 27 | | 1.0 | | |
| 30 | | | | 27 | | | 0.5 | |
| 31 | | | | 27 | | | 1.0 | |
| 32 | | | | 27 | 0.5 | | 0.5 | |

| # | Buffer solution | Protein conc. | Trehalose dihydrate | L-Proline | Polysorbate 80 | Polysorbate 20 | Poloxamer 188 | Arginine hydrochlorid |
|---|---|---|---|---|---|---|---|---|
| 33 | | | Humira formulation 1 (see item 1) | | | | | |
| 34 | | | Humira formulation 2 (see item 1) | | | | | |
| 35 | | | 80 | | 0.5 | | | 0.1 |
| 37 | | | 80 | | 1.0 | | | 0.1 |
| 38 | | | 80 | | | 0.5 | | 0.1 |
| 39 | | | 80 | | | 1.0 | | 0.1 |
| 40 | | | 80 | | | | 0.5 | 0.1 |
| 41 | Acetate buffer, pH 5.0-6.0 | 150 | 80 | | | | 1.0 | 0.1 |
| 42 | | | 80 | | 0.5 | | 0.5 | 0.1 |
| 43 | | | | 27 | 0.5 | | | |
| 44 | | | | 27 | 1.0 | | | |
| 45 | | | | 27 | | 0.5 | | |
| 46 | | | | 27 | | 1.0 | | |
| 47 | | | | 27 | | | 0.5 | |
| 48 | | | | 27 | | | 1.0 | |
| 49 | | | | 27 | 0.5 | | 0.5 | |

5.1. Thermal Stability Determination under Long-Term Exposure using Thermostress 50°C Method.

The analysis was performed according to the procedure 5.

5.2. Results.

**Table 10.** Summary on the concentrated samples with pH 5.0 before and after stresses.

| # | Protein conc. | Trehalose | L-Proline | Polysorbate 80 | Polysorbate 20 | Poloxamer | Arginine hydrochlorid | SE-HPLC Monomer content, % | SE-HPLC Change in purity after thermal stress, 50°C 144 h* | IE-HPLC acid | IE-HPLC base | IE-HPLC alkali | Total change in acid-base profile, in modulus **, % | Monomer content, % Red. | Monomer content, % Non-red. | Change in purity after thermal stress, 50°C, 144 h* Red. | Change in purity after thermal stress, 50°C, 144 h* Non-red. | Activity before stress | Value after thermal stress, 50°C, 144 h |
|---|---|---|---|---|---|---|---|---|---|---|---|---|---|---|---|---|---|---|---|
| 1 | | Humira formulation 1 (see item 1) | | | | | | 99.85 | 2.67 | 11.30 | 86.90 | 1.80 | 61.87 | 98.46 | 94.64 | -1.68 | -6.46 | 96 | 92 |
| 2 | | Humira formulation 2 (see item 1) | | | | | | 99.85 | 1.65 | 11.19 | 87.16 | 1.66 | 61.66 | 98.73 | 94.89 | -1.46 | -3.48 | 97 | 95 |
| 3 | 50 | 80 | | 0.5 | | | 0.1 | 99.30 | 1.55 | 11.48 | 86.88 | 1.65 | 64.49 | 97.99 | 94.16 | -1.46 | -2.69 | 94 | 92 |
| 4 | | 80 | | 1.0 | | | 0.1 | 99.04 | 1.62 | 9.59 | 88.92 | 1.49 | 68.05 | | | -0.99 | -2.46 | | 93 |
| 5 | | 80 | | | 0.5 | | 0.1 | 99.38 | 1.79 | 11.46 | 86.85 | 1.69 | 62.11 | | | -1.41 | -3.19 | | 91 |

| | | Formulation | | | | | | SE-HPLC | | IE-HPLC | | | | Polyacrylamide gel electrophoresis | | | | Spec. activity | |
|---|---|---|---|---|---|---|---|---|---|---|---|---|---|---|---|---|---|---|---|
| # | Protein conc. | Trehalose | L-Proline | Polysorbate 80 | Polysorbate 20 | Poloxamer | Arginine hydrochlorid | Monomer content, % | Change in purity after thermal stress, 50°C 144 h* | acid | base | alkali | Total change in acid-base profile, in modulus **, % | Monomer content, % Red. | Non-red. | Change in purity after thermal stress, 50°C, 144 h* Red. | Non-red. | Activity before stress | Value after thermal stress, 50°C, 144 h |
| 6 | | 80 | | | 1.0 | | 0.1 | 99.34 | 1.72 | 11.89 | 86.80 | 1.31 | 63.01 | | | -1.37 | -3.49 | | 90 |
| 7 | | 80 | | | | 0.5 | 0.1 | 99.48 | 1.54 | 11.02 | 87.38 | 1.60 | 63.66 | | | -1.31 | -3.10 | | 91 |
| 8 | | 80 | | | | 1.0 | 0.1 | 99.45 | 1.62 | 11.02 | 87.58 | 1.40 | 64.68 | | | -1.67 | -3.79 | | 91 |
| 9 | | 80 | 0.5 | | 0.5 | | 0.1 | 99.35 | 1.66 | 11.09 | 86.97 | 1.94 | 63.79 | | | -1.78 | -3.91 | | 90 |
| 10 | | | 27 | 0.5 | | | | 99.29 | 1.76 | 11.27 | 87.19 | 1.55 | 62.47 | 98.10 | 94.78 | -1.46 | -3.49 | 96 | 97 |
| 11 | | | 27 | 1.0 | | | | 99.93 | 1.81 | 11.43 | 87.03 | 1.54 | 62.11 | | | -1.78 | -2.41 | | 96 |

53

EP 3 563 867 A1

| # | Protein conc. | Trehalose | L-Proline | Polysorbate 80 | Polysorbate 20 | Poloxamer | Arginine hydrochloride | SE-HPLC | | IE-HPLC | | | | Polyacrylamide gel electrophoresis | | | | Spec. activity | |
|---|---|---|---|---|---|---|---|---|---|---|---|---|---|---|---|---|---|---|---|
| | | | | | | | | Monomer content, % | Change in purity after thermal stress, 50°C 144 h* | Fraction content, % | | | Total change in acid-base profile, in modulus **, % | Monomer content, % | | Change in purity after thermal stress, 50°C, 144 h* | | Activity before stress | Value after thermal stress, 50°C, 144 h |
| | | | | | | | | | | acid | base | alkali | | Red. | Non-red. | Red. | Non-red. | | |
| 12 | | | 27 | | 0.5 | | | 99.44 | 1.69 | 11.41 | 87.18 | 1.41 | 62.46 | | | -1.41 | -2.46 | | 94 |
| 13 | | | 27 | | 1.0 | | | 99.65 | 1.79 | 11.98 | 86.53 | 1.49 | 62.41 | | | -1.29 | -3.01 | | 93 |
| 14 | | | 27 | | | 0.5 | | 99.41 | 1.72 | 11.95 | 86.42 | 1.63 | 60.36 | | | -1.29 | -2.64 | | 94 |
| 15 | | | 27 | | | 1.0 | | 99.22 | 1.58 | 11.40 | 87.15 | 1.44 | 62.77 | | | -1.38 | -2.04 | | 95 |
| 16 | | | 27 | 0.5 | | 0.5 | | 99.03 | 1.79 | 11.46 | 87.00 | 1.54 | 64.66 | | | -1.43 | -3.14 | | 93 |

| # | Protein conc. | Trehalose | L-Proline | Polysorbate 80 | Polysorbate 20 | Poloxamer | Arginine hydrochloride | SE-HPLC Monomer content, % | SE-HPLC Change in purity after thermal stress, 50°C 144 h* | IE-HPLC Fraction content, % acid | base | alkali | IE-HPLC Total change in acid-base profile, in modulus **, % | Polyacrylamide gel electrophoresis Monomer content, % Red. | Non-red. | Change in purity after thermal stress, 50°C, 144 h* Red. | Non-red. | Spec. activity Activity before stress | Value after thermal stress, 50°C, 144 h |
|---|---|---|---|---|---|---|---|---|---|---|---|---|---|---|---|---|---|---|---|
| 17 | | Humira formulation 1 (see item 1) | | | | | | 99.75 | 2.99 | 11.63 | 86.41 | 1.96 | 60.84 | 97.95 | 94.10 | -1.46 | -7.23 | 98 | 99 |
| 18 | | Humira formulation 2 (see item 1) | | | | | | 99.84 | 2.26 | 11.20 | 87.10 | 1.70 | 61.30 | 98.26 | 94.74 | -1.68 | -3.15 | 99 | 75 |
| 19 | 100 | 80 | | 0.5 | | | 0.1 | 99.26 | 2.01 | 10.63 | 87.77 | 1.60 | 64.94 | 98.38 | 94.11 | -1.54 | -2.86 | 98 | 91 |
| 20 | | 80 | | 1.0 | | | 0.1 | 99.90 | 2.48 | 9.55 | 89.06 | 1.40 | 69.09 | | | -1.82 | -4.52 | | 84 |
| 21 | | 80 | | | 0.5 | | 0.1 | 99.30 | 2.06 | 11.46 | 87.04 | 1.50 | 65.03 | | | -2.06 | -4.61 | | 89 |
| 22 | | 80 | | | 1.0 | | 0.1 | 99.10 | 2.09 | 11.98 | 86.39 | 1.63 | 65.87 | | | -2.21 | -4.13 | | 84 |

| # | Protein conc. | Trehalose | L-Proline | Polysorbate 80 | Polysorbate 20 | Poloxamer | Arginine hydrochloride | SE-HPLC | | IE-HPLC | | | | Polyacrylamide gel electrophoresis | | | | Spec. activity | |
|---|---|---|---|---|---|---|---|---|---|---|---|---|---|---|---|---|---|---|---|
| | | | | Formulation | | | | Monomer content, % | Change in purity after thermal stress, 50°C 144 h* | Fraction content, % | | | Total change in acid-base profile, in modulus **, % | Monomer content, % | | Change in purity after thermal stress, 50°C, 144 h* | | Activity before stress | Value after thermal stress, 50°C, 144 h |
| | | | | | | | | | | acid | base | alkali | | Red. | Non-red. | Red. | Non-red. | | |
| 23 | | 80 | | | | 0.5 | 0.1 | 99.38 | 2.01 | 11.15 | 87.25 | 1.61 | 63.18 | | | -2.27 | -4.06 | | 81 |
| 24 | | 80 | | | | 1.0 | 0.1 | 99.29 | 2.36 | 10.85 | 87.57 | 1.58 | 65.63 | | | -2.57 | -4.28 | | 86 |
| 25 | | 80 | 0.5 | | | 0.5 | 0.1 | 99.41 | 2.46 | 11.86 | 86.98 | 1.16 | 63.64 | | | -2.66 | -4.49 | | 88 |
| 26 | | 27 | 0.5 | | | | | 99.08 | 2.21 | 11.21 | 87.31 | 1.48 | 62.43 | | | -3.09 | -4.25 | | 91 |
| 27 | | 27 | 1.0 | | | | | 99.95 | 1.87 | 10.62 | 87.82 | 1.57 | 60.90 | 98.35 | 94.09 | -2.68 | -3.44 | 97 | 87 |
| 28 | | 27 | | | 0.5 | | | 99.43 | 2.62 | 11.41 | 86.93 | 1.66 | 64.13 | | | -2.64 | -3.46 | | 89 |

| # | Formulation Protein conc. | Trehalose | L-Proline | Polysorbate 80 | Polysorbate 20 | Poloxamer 188 | Arginine hydrochloride | SE-HPLC Monomer content, % | SE-HPLC Change in purity after thermal stress, 50°C 144 h* | IE-HPLC Fraction content, % acid | base | alkali | IE-HPLC Total change in acid-base profile, in modulus **, % | PAGE Monomer content, % Red. | Non-red. | PAGE Change in purity after thermal stress, 50°C, 144 h* Red. | Non-red. | Spec. activity Activity before stress | Value after thermal stress, 50°C, 144 h |
|---|---|---|---|---|---|---|---|---|---|---|---|---|---|---|---|---|---|---|---|
| 29 | | | 27 | | 1.0 | | | 99.46 | 2.49 | 11.32 | 86.90 | 1.78 | 63.89 | | | -1.89 | -3.49 | | 88 |
| 30 | | | 27 | | | 0.5 | | 99.28 | 2.48 | 10.99 | 87.31 | 1.71 | 62.47 | | | -1.44 | 0.89 | | 89 |
| 31 | | | 27 | | | 1.0 | | 99.33 | 1.92 | 11.52 | 87.08 | 1.40 | 60.28 | | | -0.84 | -2.09 | | 89 |
| 32 | | | 27 | 0.5 | | 0.5 | | 99.38 | 2.31 | 10.46 | 87.79 | 1.75 | 62.48 | | | -1.98 | -3.01 | | 86 |

57

| # | Protein conc. | Trehalose | L-Proline | Polysorbate 80 | Polysorbate 20 | Poloxamer | Arginine hydrochlorid | SE-HPLC Monomer content, % | Change in purity after thermal stress, 50°C 144 h* | IE-HPLC Fraction content, % acid | base | alkali | Total change in acid-base profile, in modulus **, % | Polyacrylamide gel electrophoresis Monomer content, % Red. | Non-red. | Change in purity after thermal stress, 50°C, 144 h* Red. | Non-red. | Spec. activity Activity before stress | Value after thermal stress, 50°C, 144 h |
|---|---|---|---|---|---|---|---|---|---|---|---|---|---|---|---|---|---|---|---|
| 33 | | Humira formulation 1 (see item 1) | | | | | | 99.88 | 3.22 | 11.45 | 86.73 | 1.82 | 59.91 | 98.29 | 94.64 | -1.92 | -6.61 | 99 | 98 |
| 34 | | Humira formulation 2 (see item 1) | | | | | | 99.72 | 2.81 | 11.19 | 87.16 | 1.65 | 60.64 | 98.43 | 94.06 | -1.75 | -7.52 | 99 | 101 |
| 35 | 150 | 80 | | 0.5 | | | 0.1 | 99.09 | 2.40 | 10.75 | 87.64 | 1.61 | 63.48 | 98.32 | 94.85 | -1.82 | -4.13 | 98 | 113 |
| 37 | | 80 | | 1.0 | | | 0.1 | 99.92 | 2.67 | 11.28 | 87.01 | 1.71 | 62.90 | | | -2.00 | -6.09 | | 98 |
| 38 | | 80 | | | 0.5 | | 0.1 | 99.24 | 2.45 | 11.28 | 87.25 | 1.47 | 62.30 | | | -2.28 | -4.34 | | 99 |
| 39 | | 80 | | | 1.0 | | 0.1 | 99.16 | 2.80 | 10.73 | 87.75 | 1.52 | 64.32 | | | -2.70 | -4.24 | | 97 |

58

| # | Protein conc. | Formulation | | | | | | SE-HPLC | | IE-HPLC | | | | Polyacrylamide gel electrophoresis | | | | Spec. activity | |
|---|---|---|---|---|---|---|---|---|---|---|---|---|---|---|---|---|---|---|---|
| | | Trehalose | L-Proline | Polysorbate 80 | Polysorbate 20 | Poloxamer | Arginine hydrochlorid | Monomer content, % | Change in purity after thermal stress, 50°C 144 h* | Fraction content, % | | | Total change in acid-base profile, in modulus **, % | Monomer content, % | | Change in purity after thermal stress, 50°C, 144 h* | | Activity before stress | Value after thermal stress, 50°C, 144 h |
| | | | | | | | | | | acid | base | alkali | | Red. | Non-red. | Red. | Non-red. | | |
| 40 | | 80 | | | | 0.5 | 0.1 | 99.74 | 2.89 | 11.39 | 86.92 | 1.69 | 64.11 | | | -2.20 | -4.11 | | 94 |
| 41 | | 80 | | | | 1.0 | 0.1 | 99.12 | 2.80 | 11.74 | 86.80 | 1.46 | 63.79 | | | -2.67 | -4.06 | | 93 |
| 42 | | 80 | 0.5 | | | 0.5 | 0.1 | 99.20 | 2.79 | 11.79 | 86.52 | 1.69 | 64.39 | | | -2.78 | -4.08 | | 96 |
| 43 | | | 27 | 0.5 | | | | 99.88 | 2.48 | 11.28 | 87.10 | 1.63 | 62.63 | | | -3.13 | -2.37 | | 86 |
| 44 | | | 27 | 1.0 | | | | 99.61 | 2.64 | 11.35 | 87.07 | 1.58 | 61.45 | 98.45 | 94.14 | -2.92 | -2.50 | 97 | 88 |
| 45 | | | 27 | | 0.5 | | | 99.46 | 2.71 | 11.49 | 86.78 | 1.73 | 63.44 | | | -2.01 | -2.03 | | 89 |

EP 3 563 867 A1

| # | Formulation | | | | | | | SE-HPLC | | IE-HPLC | | | | Polyacrylamide gel electrophoresis | | | | Spec. activity | |
|---|---|---|---|---|---|---|---|---|---|---|---|---|---|---|---|---|---|---|---|
| | Protein conc. | Trehalose | L-Proline | Polysorbate 80 | Polysorbate 20 | Poloxamer | Arginine hydrochlorid | Monomer content, % | Change in purity after thermal stress, 50°C 144 h* | Fraction content, % | | | Total change in acid-base profile, in modulus **, % | Monomer content, % | | Change in purity after thermal stress, 50°C, 144 h* | | Activity before stress | Value after thermal stress, 50°C, 144 h |
| | | | | | | | | | | acid | base | alkali | | Red. | Non-red. | Red. | Non-red. | | |
| 46 | | | 27 | | 1.0 | | | 99.42 | 2.83 | 11.13 | 86.99 | 1.88 | 63.79 | | | -2.03 | -1.95 | | 87 |
| 47 | | | 27 | | | 0.5 | | 99.12 | 2.36 | 10.91 | 87.27 | 1.82 | 62.17 | | | -1.45 | 0.52 | | 86 |
| 48 | | | 27 | | | 1.0 | | 99.12 | 2.52 | 11.30 | 87.14 | 1.57 | 61.01 | | | -2.08 | -1.73 | | 92 |
| 49 | | | 27 | 0.5 | | 0.5 | | 99.42 | 2.80 | 11.98 | 86.56 | 1.46 | 61.09 | | | -2.35 | -1.79 | | 91 |

* Change was calculated by the formula $\Delta = $ (Value after stress − Value before stress) ** Absolute change was calculated by the formula $\Delta = $ |Content of acid fraction before stress − Content of acid fraction after stress| + |Content of basic fraction before stress − Content of basic fraction after stress| + |Content of dominant fraction before stress − Content of dominant fraction after stress|

average results / baseline data

negative results

positive results

**Table 11.** Summary on the concentrated samples with pH 6.0 before and after stresses.

| # | Formulation | Protein conc. | Trehalose | L-Proline | Polysorbate 80 | Polysorbate 20 | Poloxamer 188 | Arginine hydrochlorid | SE-HPLC Monomer content, % | SE-HPLC Change in purity after thermal stress, 50°C 144 h* | IE-HPLC acid | IE-HPLC base | IE-HPLC alkali | Total change in acid-base profile, in modulus**, % | PAGE Monomer content, % Red. | Non-red. | Change in purity after thermal stress, 50°C, 144 h* Red. | Non-red. | Spec. activity Activity before stress | Value after thermal stress, 50°C, 144 h |
|---|---|---|---|---|---|---|---|---|---|---|---|---|---|---|---|---|---|---|---|---|
| 1 | Humira formulation 1 (see item 1) | | | | | | | | 99.85 | 2.67 | 11.30 | 86.90 | 1.80 | 61.87 | 98.46 | 94.64 | -1.68 | -6.46 | 96 | 92 |
| 2 | Humira formulation 2 (see item 1) | | | | | | | | 99.85 | 1.65 | 11.19 | 87.16 | 1.66 | 61.66 | 98.73 | 94.89 | -1.46 | -3.48 | 97 | 95 |
| 50 | | 50 | 80 | | 0.5 | | | 0.1 | 99.31 | 1.66 | 11.38 | 86.47 | 2.15 | 61.44 | | | -1.66 | -2.41 | 94 | 92 |
| 51 | | | 80 | | 1.0 | | | 0.1 | 99.41 | 1.74 | 11.46 | 86.99 | 1.55 | 62.01 | 97.66 | 94.06 | -1.45 | -3.08 | | 88 |
| 52 | | | 80 | | | 0.5 | | 0.1 | 99.64 | 1.61 | 11.84 | 87.01 | 1.15 | 64.21 | | | -1.11 | -2.49 | | 94 |

| | Formulation | | | | | | | SE-HPLC | | IE-HPLC | | | | Polyacrylamide gel electrophoresis | | | | Spec. activity | |
|---|---|---|---|---|---|---|---|---|---|---|---|---|---|---|---|---|---|---|---|
| | | | | | | | | | | Fraction content, % | | | | Monomer content, % | | Change in purity after thermal stress, 50°C, 144 h* | | | |
| # | Protein conc. | Trehalose | L-Proline | Polysorbate 80 | Polysorbate 20 | Poloxamer | Arginine hydrochloride | Monomer content, % | Change in purity after thermal stress, 50°C 144 h* | acid | base | alkali | Total change in acid-base profile, in modulus**, % | Red. | Non-red. | Red. | Non-red. | Activity before stress | Value after thermal stress, 50°C, 144 h |
| 53 | | 80 | | | 1.0 | | 0.1 | 99.41 | 1.45 | 11.35 | 86.86 | 1.79 | 64.26 | | | -1.65 | -2.77 | | 96 |
| 54 | | 80 | | | | 0.5 | 0.1 | 99.29 | 1.56 | 11.65 | 86.88 | 1.47 | 61.87 | | | -1.82 | -2.09 | | 89 |
| 55 | | 80 | | | | 1.0 | 0.1 | 99.56 | 1.59 | 11.98 | 86.79 | 1.23 | 61.94 | | | -1.97 | -2.67 | | 91 |
| 56 | | 80 | | | 0.5 | 0.5 | 0.1 | 99.16 | 1.67 | 11.71 | 87.16 | 1.13 | 61.81 | | | -1.19 | -2.48 | | 96 |
| 57 | | | 27 | 0.5 | | | | 99.64 | 1.47 | 11.22 | 87.19 | 1.59 | 60.20 | 98.02 | 94.41 | -0.88 | -2.37 | 95 | 95 |
| 58 | | | 27 | 1.0 | | | | 99.67 | 1.88 | 11.64 | 86.68 | 1.68 | 62.11 | | | -0.91 | -2.48 | | 94 |

| # | Protein conc. | Trehalose | L-Proline | Polysorbate 80 | Polysorbate 20 | Poloxamer 188 | Arginine hydrochlorid | SE-HPLC Monomer content, % | SE-HPLC Change in purity after thermal stress, 50°C 144 h* | IE-HPLC Fraction content, % acid | base | alkali | IE-HPLC Total change in acid-base profile, in modulus**, % | Polyacrylamide gel electrophoresis Monomer content, % Red. | Non-red. | Change in purity after thermal stress, 50°C, 144 h* Red. | Non-red. | Spec. activity Activity before stress | Value after thermal stress, 50°C, 144 h |
|---|---|---|---|---|---|---|---|---|---|---|---|---|---|---|---|---|---|---|---|
| 59 | | | 27 | | 0.5 | | | 99.73 | 1.62 | 11.58 | 86.63 | 1.79 | 63.53 | | | -1.19 | -2.61 | | 89 |
| 60 | | | 27 | | 1.0 | | | 99.48 | 1.40 | 11.27 | 87.15 | 1.58 | 60.84 | | | -1.41 | -2.00 | | 96 |
| 61 | | | 27 | | | 0.5 | | 99.35 | 1.89 | 11.34 | 86.81 | 1.85 | 60.84 | | | -1.06 | -3.09 | | 91 |
| 62 | | | 27 | | | 1.0 | | 99.47 | 1.85 | 11.65 | 87.09 | 1.26 | 61.01 | | | -0.94 | -2.14 | | 90 |
| 63 | | | 27 | 0.5 | | 0.5 | | 99.58 | 1.74 | 11.82 | 87.14 | 1.04 | 60.06 | | | -1.23 | -2.90 | | 93 |

64

EP 3 563 867 A1

| # | Protein conc. | Trehalose | L-Proline | Polysorbate 80 | Polysorbate 20 | Poloxamer | Arginine hydrochloride | SE-HPLC | | IE-HPLC | | | | Polyacrylamide gel electrophoresis | | | | Spec. activity | |
|---|---|---|---|---|---|---|---|---|---|---|---|---|---|---|---|---|---|---|---|
| | | | | | | | | Monomer content, % | Change in purity after thermal stress, 50°C 144 h* | Fraction content, % | | | Total change in acid-base profile, in modulus**, % | Monomer content, % | | Change in purity after thermal stress, 50°C, 144 h* | | Activity before stress | Value after thermal stress, 50°C, 144 h |
| | | | | | | | | | | acid | base | alkali | | Red. | Non-red. | Red. | Non-red. | | |
| 17 | | Humira formulation 1 (see item 1) | | | | | | 99.75 | 2.99 | 11.63 | 86.41 | 1.96 | 60.84 | 97.95 | 94.10 | -1.46 | -7.23 | 98 | 99 |
| 18 | | Humira formulation 2 (see item 1) | | | | | | 99.84 | 2.26 | 11.20 | 87.10 | 1.70 | 61.30 | 98.26 | 94.74 | -1.68 | -3.15 | 99 | 75 |
| 64 | 100 | 80 | | 0.5 | | | 0.1 | 99.31 | 2.34 | 11.55 | 86.65 | 1.80 | 69.84 | 98.12 | 94.66 | -1.35 | -2.06 | 98 | 94 |
| 65 | | 80 | | 1.0 | | | 0.1 | 99.26 | 2.41 | 11.64 | 86.84 | 1.52 | 66.44 | | | -1.82 | -3.49 | | 96 |
| 66 | | 80 | | | 0.5 | | 0.1 | 99.64 | 2.43 | 11.20 | 86.69 | 2.11 | 62.59 | | | -2.31 | -4.11 | | 89 |
| 67 | | 80 | | | 1.0 | | 0.1 | 99.53 | 1.89 | 11.68 | 86.72 | 1.60 | 61.49 | | | -2.48 | -3.69 | | 90 |

| # | Protein conc. | Trehalose | L-Proline | Polysorbate 80 | Polysorbate 20 | Poloxamer 188 | Arginine hydrochloride | SE-HPLC Monomer content, % | SE-HPLC Change in purity after thermal stress, 50°C 144 h* | IE-HPLC acid | IE-HPLC base | IE-HPLC alkali | Total change in acid-base profile, in modulus**, % | Gel Monomer content Red. | Gel Monomer content Non-red. | Change in purity after thermal stress 50°C,144 h* Red. | Non-red. | Activity before stress | Value after thermal stress, 50°C, 144 h |
|---|---|---|---|---|---|---|---|---|---|---|---|---|---|---|---|---|---|---|---|
| 68 | | 80 | | | | 0.5 | 0.1 | 99.16 | 1.84 | 11.54 | 86.69 | 1.77 | 61.48 | | | -2.64 | -3.17 | | 91 |
| 69 | | 80 | | | | 1.0 | 0.1 | 99.60 | 1.91 | 11.20 | 86.51 | 2.29 | 62.03 | | | -2.41 | -3.76 | | 92 |
| 70 | | 80 | | | 0.5 | 0.5 | 0.1 | 99.22 | 2.06 | 11.28 | 87.12 | 1.60 | 62.07 | | | -2.37 | -3.19 | | 93 |
| 71 | | | 27 | 0.5 | | | | 99.18 | 2.24 | 11.62 | 87.30 | 1.08 | 61.34 | | | -3.11 | -2.22 | | 94 |
| 72 | | | 27 | 1.0 | | | | 99.67 | 2.16 | 11.59 | 86.88 | 1.53 | 61.55 | 98.32 | 94.21 | -2.46 | -3.49 | 102 | 96 |
| 73 | | | 27 | | 0.5 | | | 99.48 | 2.19 | 11.41 | 86.84 | 1.75 | 60.48 | | | -2.80 | -3.85 | | 88 |

| # | Protein conc. | Trehalose | L-Proline | Polysorbate 80 | Polysorbate 20 | Poloxamer | Arginine hydrochlorid | Monomer content, % | Change in purity after thermal stress, 50°C 144 h* | acid | base | alkali | Total change in acid-base profile, in modulus**, % | Red. | Non-red. | Red. | Non-red. | Activity before stress | Value after thermal stress, 50°C, 144 h |
|---|---|---|---|---|---|---|---|---|---|---|---|---|---|---|---|---|---|---|---|
| 74 | | | 27 | | 1.0 | | | 99.49 | 1.99 | 11.60 | 86.70 | 1.70 | 60.68 | | | -1.42 | -3.41 | | 89 |
| 75 | | | 27 | | | 0.5 | | 99.56 | 2.09 | 11.26 | 86.90 | 1.84 | 61.89 | | | -0.84 | -2.18 | | 90 |
| 76 | | | 27 | | | 1.0 | | 99.62 | 2.22 | 11.41 | 86.66 | 1.93 | 60.76 | | | -0.98 | -2.14 | | 93 |
| 77 | | | 27 | 0.5 | | 0.5 | | 99.44 | 2.37 | 11.73 | 86.58 | 1.69 | 63.18 | | | -1.49 | -3.11 | | 98 |

| # | Protein conc. | Trehalose | L-Proline | Polysorbate 80 | Polysorbate 20 | Poloxamer | Arginine hydrochloride | SE-HPLC Monomer content, % | SE-HPLC Change in purity after thermal stress, 50°C 144 h* | IE-HPLC Fraction content, % acid | base | alkali | Total change in acid-base profile, in modulus**, % | Monomer content, % Red. | Non-red. | Change in purity after thermal stress, 50°C, 144 h* Red. | Non-red. | Activity before stress | Value after thermal stress, 50°C, 144 h |
|---|---|---|---|---|---|---|---|---|---|---|---|---|---|---|---|---|---|---|---|
| 33 | | Humira formulation 1 (see item 1) | | | | | | 99.88 | 3.22 | 11.45 | 86.73 | 1.82 | 59.91 | 98.29 | 94.64 | -1.92 | -6.61 | 99 | 98 |
| 34 | | Humira formulation 2 (see item 1) | | | | | | 99.72 | 2.81 | 11.19 | 87.16 | 1.65 | 60.64 | 98.43 | 94.06 | -1.75 | -7.52 | 99 | 101 |
| 78 | 150 | 80 | | 0.5 | | | 0.1 | 99.18 | 2.87 | 11.44 | 86.88 | 1.68 | 59.22 | 98.13 | 94.54 | -0.82 | -4.41 | 99 | 94 |
| 79 | | 80 | | 1.0 | | | 0.1 | 99.25 | 2.66 | 11.52 | 86.57 | 1.91 | 60.16 | | | -2.08 | -4.09 | | 92 |
| 80 | | 80 | | | 0.5 | | 0.1 | 99.35 | 2.28 | 11.68 | 86.69 | 1.63 | 63.11 | | | -2.14 | -4.44 | | 92 |
| 81 | | 80 | | | 1.0 | | 0.1 | 99.41 | 1.87 | 11.97 | 86.81 | 1.22 | 62.59 | | | -2.62 | -4.14 | | 93 |

| # | Protein conc. | Trehalose | L-Proline | Polysorbate 80 | Polysorbate 20 | Poloxamer 188 | Arginine hydrochloride | SE-HPLC Monomer content, % | SE-HPLC Change in purity after thermal stress, 50°C 144 h* | IE-HPLC Fraction content, % acid | IE-HPLC base | IE-HPLC alkali | IE-HPLC Total change in acid-base profile, in modulus**, % | PAGE Monomer content, % Red. | PAGE Monomer content, % Non-red. | PAGE Change in purity after thermal stress, 50°C, 144 h* Red. | PAGE Change in purity Non-red. | Spec. activity Activity before stress | Spec. activity Value after thermal stress, 50°C, 144 h |
|---|---|---|---|---|---|---|---|---|---|---|---|---|---|---|---|---|---|---|---|
| 8-2 | | 80 | | | | 0.5 | 0.1 | 99.64 | 2.44 | 11.44 | 86.76 | 1.80 | 63.00 | | | -2.33 | -4.16 | | 93 |
| 8-3 | | 80 | | 0.5 | | 1.0 | 0.1 | 99.12 | 2.57 | 11.50 | 86.58 | 1.92 | 62.41 | | | -2.57 | -4.90 | | 94 |
| 8-4 | | 80 | | 0.5 | | 0.5 | 0.1 | 99.16 | 2.14 | 11.64 | 86.59 | 1.77 | 63.49 | | | -2.60 | -4.16 | | 91 |
| 8-5 | | | 27 | 1.0 | | | | 99.26 | 5.49 | 11.63 | 86.74 | 1.63 | 61.18 | | | -2.13 | -3.37 | | 94 |
| 8-6 | | | 27 | | | | | 99.61 | 1.88 | 11.80 | 86.59 | 1.61 | 60.66 | 98.04 | 94.10 | -1.87 | -2.11 | 97 | 96 |
| 8-7 | | | 27 | | 0.5 | | | 99.34 | 2.91 | 11.68 | 86.44 | 1.88 | 60.49 | | | -2.06 | -2.18 | | 92 |

EP 3 563 867 A1

| # | Protein conc. | Trehalose | L-Proline | Polysorbate 80 | Polysorbate 20 | Poloxamer | Arginine hydrochloride | SE-HPLC | | IE-HPLC | | | | Polyacrylamide gel electrophoresis | | | | Spec. activity | |
|---|---|---|---|---|---|---|---|---|---|---|---|---|---|---|---|---|---|---|---|
| | | | | | | | | Monomer content, % | Change in purity after thermal stress, 50°C 144 h* | Fraction content, % | | | Total change in acid-base profile, in modulus**, % | Monomer content, % | | Change in purity after thermal stress, 50°C, 144 h* | | Activity before stress | Value after thermal stress, 50°C, 144 h |
| | | | | | | | | | | acid | base | alkali | | Red. | Non-red. | Red. | Non-red. | | |
| 88 | | | 27 | | 1.0 | | | 99.55 | 2.60 | 11.91 | 86.58 | 1.51 | 61.27 | | | -2.74 | -2.95 | | 88 |
| 89 | | | 27 | | | 0.5 | | 99.16 | 2.58 | 11.11 | 86.93 | 1.96 | 61.08 | | | -1.73 | -1.99 | | 89 |
| 90 | | | 27 | | | 1.0 | | 99.20 | 2.78 | 11.54 | 86.97 | 1.49 | 62.54 | | | -2.13 | -2.60 | | 90 |
| 91 | | | 27 | 0.5 | | 0.5 | | 99.44 | 2.43 | 11.67 | 86.77 | 1.56 | 60.20 | | | -2.01 | -2.06 | | 93 |

* Change was calculated by the formula $\Delta = (\text{Value after stress} - \text{Value before stress})$  ** Absolute change was calculated by the formula $\Delta = |\text{Content of acid fraction before stress} - \text{Content of acid fraction after stress}| + |\text{Content of basic fraction before stress} - \text{Content of basic fraction after stress}| + |\text{Content of dominant fraction before stress} - \text{Content of dominant fraction after stress}|$

positive results  negative results  average results / baseline data

General conclusions

1. Screening for the stable adalimumab pharmaceutical composition was carried out in several stages: selection of the buffer solution nature, selection of pH and buffer capacity of the solution, selection of the osmolyte and the stabilizing agent, selection of the final pharmaceutical composition and confirmation of the final high-concentrated adalimumab pharmaceutical composition.

2. During the investigation, the adalimumab thermal and colloidal stability in more than 90 formulations were studied using the following methods: PEG aggregation, shake-test, freeze-thawing, thermal stress with subsequent analysis of the degree of turbidity (UV-spectrophotometry), purity (exclusion HPLC, dynamic light scattering and gel electrophoresis, including using the LabChip, Caliper system), and acid-base profile (LabChip, Caliper and ion-exchange HPLC).

3. The study showed a low thermal stability of the original Humira preparation formulation (Humira 1), used in the commercial preparation with adalimumab concentration of 50 mg/mL, compared to the formulations of the present invention.

4. The experiment showed the significant impact of the freezing and thawing processes on adalimumab aggregation in the original Humira preparation formulation (Humira 2), used in the commercial preparation of adalimumab 100 mg/mL, compared to the formulations of the present invention.

5. Adalimumab pharmaceutical compositions based on acetate buffer solution having pH 5.0 to 6.0 supplemented with trehalose dihydrate, arginine hydrochloride, proline, and surface-active substances from the group of Polysorbate 20, Polysorbate 80 and Poloxamer 188 exhibit a greater thermal and colloid stability at concentrations of 50-150 mg/mL compared to the original formulations and are the subject matters of the present invention.

The optimal additive formulation of adalimumab was selected for solutions having pH 5.5. The stability of adalimumab in the formulations with a pH range of 5.0 to 6.0 was confirmed on the samples at protein concentrations of 50, 100 and 150 mg/mL.

6. The preparation process of the pharmaceutical compositions of the present invention with the adalimumab concentration of 150 mg/mL enables concentrating up to 200 mg/mL without loss of protein quality for subsequent dilution upon adding SAS and washing the ultrafiltration device after concentrating.

## Claims

1. An aqueous pharmaceutical composition for intravenous or subcutaneous administration comprising:

   a) a recombinant monoclonal anti-TNF$\alpha$ antibody;
   b) an acetate ion based buffer agent (or buffer system);
   c) trehalose and/or proline, or
   trehalose and arginine;
   d) polysorbate 20, polysorbate 80 or poloxamer 188, or a combination thereof.

2. The composition of claim 1 wherein the recombinant monoclonal anti-TNFa antibody is adalimumab.

3. The composition of claims 1-2 wherein the monoclonal antibody concentration is from 50 to 200 mg/mL.

4. The composition of claim 1 wherein the acetate buffer solution concentration is from 1 to 100 mM.

5. The composition of claim 1 wherein pH of the composition is from 4 to 7.

6. The composition of claim 1 wherein the trehalose concentration is from 25 to 150 mg/mL.

7. The composition of claim 1 wherein the proline concentration is from 5 to 40 mg/mL.

8. The composition of claim 1 wherein the trehalose concentration is from 25 to 150 mg/mL, and the arginine concentration is from 0.05 to 0.5 mg/mL.

9. The composition of claim 1 wherein the polysorbate 20 concentration is from 0.05 mg/mL to 10 mg/mL.

**10.** The composition of claim 1 wherein the polysorbate 80 concentration is from 0.05 mg/mL to 10 mg/mL.

**11.** The composition of claim 1 wherein the poloxamer 188 concentration is from 0.05 mg/mL to 10 mg/mL.

**12.** The composition of claim 1 comprising:

a) from 50 to 200 mg/mL of the recombinant monoclonal anti-TNFa antibody;
b) from 0.4 to 1.2 mg/mL of sodium acetate trihydrate;
c) from 25 to 150 mg/mL of trehalose and/or from 5 to 40 mg/mL of proline, or
from 25 to 150 mg/mL of trehalose and from 0.05 to 0.5 mg/mL of arginine;
d) glacial acetic acid until pH 5.0 to 6.0;
e) from 0.1 mg/mL to 1 mg/mL of Polysorbate 80.

**13.** The composition of claim 12 wherein the recombinant monoclonal anti-TNFa antibody is adalimumab.

**14.** The composition of claim 12 comprising:

a) from 50 to 150 mg/mL of adalimumab;
b) 0.436 mg/mL of sodium acetate trihydrate;
c) 80 mg/mL of trehalose dihydrate;
d) glacial acetic acid until pH 5.5;
e) 0.5 mg/mL of polysorbate 80.

**15.** The composition of claim 12 comprising:

f) from 50 to 150 mg/mL of adalimumab;
g) 0.436 mg/mL of sodium acetate trihydrate;
h) 80 mg/mL of trehalose dihydrate and 0.1 mg/mL of arginine hydrochloride;
i) glacial acetic acid until pH 5.5;
j) 0.5 mg/mL of Polysorbate 80.

**16.** The composition of claim 12 comprising:

a) from 50 to 150 mg/mL of adalimumab;
b) 0.436 mg/mL of sodium acetate trihydrate;
c) 27 mg/mL of proline;
d) glacial acetic acid until pH 5.5;
e) 0.5 mg/mL of polysorbate 80.

**17.** The composition of claim 12 comprising:

a) from 50 to 150 mg/mL of adalimumab;
b) 0.436 mg/mL of sodium acetate trihydrate;
c) 40 mg/mL of trehalose dihydrate;
d) 13.5 mg/mL of proline;
e) glacial acetic acid until pH 5.5;
f) 0.5 mg/mL of polysorbate 80.

**18.** The composition of claim 1 comprising:

a) from 50 to 200 mg/mL of the recombinant monoclonal anti-TNFa antibody;
b) from 0.4 to 1.2 mg/mL of sodium acetate trihydrate;
c) from 25 to 150 mg/mL of trehalose and/or from 5 to 40 mg/mL of proline, or from 25 to 150 mg/mL of trehalose
and from 0.05 to 0.5 mg/mL of arginine;
d) glacial acetic acid until pH 5.0 to 6.0;
e) from 0.1 mg/mL to 1 mg/mL of polysorbate 20.

**19.** The composition of claim 18 wherein the recombinant monoclonal anti-TNFa antibody is adalimumab.

**20.** The composition of claim 18 comprising:

a) from 50 to 150 mg/mL of adalimumab;
b) 0.436 mg/mL of sodium acetate trihydrate;
c) 80 mg/mL of trehalose dihydrate;
d) glacial acetic acid until pH 5.5;
e) 0.5 mg/mL of polysorbate 20.

**21.** The composition of claim 18 comprising:

a) from 50 to 150 mg/mL of adalimumab;
b) 0.436 mg/mL of sodium acetate trihydrate;
c) 80 mg/mL of trehalose dihydrate and 0.1 mg/mL of arginine hydrochloride;
d) glacial acetic acid until pH 5.5;
e) 0.5 mg/mL of Polysorbate 20.

**22.** The composition of claim 18 comprising:

a) from 50 to 150 mg/mL of adalimumab;
b) 0.436 mg/mL of sodium acetate trihydrate;
c) 27 mg/mL of proline;
d) glacial acetic acid until pH 5.5;
e) 0.5 mg/mL of polysorbate 20.

**23.** The composition of claim 18 comprising:

a) from 50 to 150 mg/mL of adalimumab;
b) 0.436 mg/mL of sodium acetate trihydrate;
c) 40 mg/mL of trehalose dihydrate;
d) 13.5 mg/mL of proline;
e) glacial acetic acid until pH 5.5;
f) 0.5 mg/mL of Polysorbate 20.

**24.** The composition of claim 1 comprising:

a) from 50 to 200 mg/mL of the recombinant monoclonal anti-TNFa antibody;
b) from 0.4 to 1.2 mg/mL of sodium acetate trihydrate;
c) from 25 to 150 mg/mL of trehalose and/or 5 to 40 mg/mL of proline, or
from 25 to 150 mg/mL of trehalose and from 0.05 to 0.5 mg/mL of arginine;
d) glacial acetic acid until pH 5.0 to 6.0;
e) from 0.1 mg/mL to 1 mg/mL of Poloxamer 188.

**25.** The composition of claim 24 wherein the recombinant monoclonal anti-TNFa antibody is adalimumab.

**26.** The composition of claim 24 comprising:

a) from 50 to 150 mg/mL of adalimumab;
b) 0.436 mg/mL of sodium acetate trihydrate;
c) 80 mg/mL of trehalose dihydrate;
d) glacial acetic acid until pH 5.5;
e) 1.0 mg/mL of Poloxamer 188.

**27.** The composition of claim 24 comprising:

a) from 50 to 150 mg/mL of adalimumab;
b) 0.436 mg/mL of sodium acetate trihydrate;
c) 80 mg/mL of trehalose dihydrate and 0.1 mg/mL of arginine hydrochloride;
d) glacial acetic acid until pH 5.5;

e) 1.0 mg/mL of poloxamer 188.

**28.** The composition of claim 24 comprising:

a) from 50 to 150 mg/mL of adalimumab;
b) 0.436 mg/mL of sodium acetate trihydrate;
c) 27 mg/mL of proline;
d) glacial acetic acid until pH 5.5;
e) 1.0 mg/mL of poloxamer 188.

**29.** The composition of claim 24 comprising:

a) from 50 to 150 mg/mL of adalimumab;
b) 0.436 mg/mL of sodium acetate trihydrate;
c) 40 mg/mL of trehalose dihydrate;
d) 13.5 mg/mL of proline;
e) glacial acetic acid until pH 5.5;
f) 1.0 mg/mL of Poloxamer 188.

**30.** A method for treating TNF$\alpha$ mediated diseases comprising administering an effective amount of the composition of claims 1-29.

**31.** The method of claim 30 wherein the TNF$\alpha$ mediated disease is selected from the group of:

a) active (moderate to severe) rheumatoid arthritis,
b) active psoriatic arthritis,
c) active ankylosing spondylitis,
d) chronic (moderate to severe) plaque psoriasis,
e) (moderate to severe) ulcerative colitis,
f) axial spondylarthritis,
g) active hidradenitis suppurativa,
h) juvenile idiopathic arthritis,
i) (moderate or severe) Crohn's disease,
j) uveitis,
k) active enthesitis-associated arthritis.

**32.** Use of the composition of claims 1-29 for treating TNF$\alpha$ mediated diseases.

**33.** Use of claim 32 wherein the disease is selected from the group of:

a) active (moderate to severe) rheumatoid arthritis,
b) active psoriatic arthritis,
c) active ankylosing spondylitis,
d) chronic (moderate to severe) plaque psoriasis,
e) (moderate to severe) ulcerative colitis,
f) axial spondylarthritis,
g) active hidradenitis suppurativa,
h) juvenile idiopathic arthritis,
i) (moderate or severe) Crohn's disease,
j) uveitis,
k) active enthesitis-associated arthritis.

**34.** A method for producing the composition of any one of claims 1-29 comprising the addition of acetate buffering agents to an aqueous phase followed by the addition, in any order, of the following components:

a) an osmolyte and/or a stabilizing agent selected from the group of trehalose, proline, arginine or a combination thereof;
b) a recombinant monoclonal anti-TNF$\alpha$ antibody;

c) a surface-active substance selected from the group of polysorbate 20, polysorbate 80, poloxamer 188, or a combination thereof.

**Fig. 1**

PEG 6000 concentration, %

5 mM pH 6    5 mM pH 5.5    5 mM pH 5    5 mM pH 4    5 mM pH 3.75

5 mM pH 3.5    10 mM pH 6    10 mM pH 5.5    10 mM pH 5    10 mM pH 4

10 mM pH 3.75    10 mM pH 3.5    20 mM pH 6    20 mM pH 5.5    20 mM pH 5

20 mM pH 4    20 mM pH 3.75    20 mM pH 3.5

# Fig. 2

**Fig. 3**

**Fig. 4**

**Fig. 5**

**Fig. 6**

**Fig. 7**

**Fig. 8**

## INTERNATIONAL SEARCH REPORT

| | International application No. |
|---|---|
| | PCT/RU 2017/050133 |

**A. CLASSIFICATION OF SUBJECT MATTER**

A61K 39/395 (2006.01)    A61P 37/00 (2006.01)

According to International Patent Classification (IPC) or to both national classification and IPC

**B. FIELDS SEARCHED**

Minimum documentation searched (classification system followed by classification symbols)

A61K 39/00, 39/395, A61P 37/00

Documentation searched other than minimum documentation to the extent that such documents are included in the fields searched

Electronic data base consulted during the international search (name of data base and, where practicable, search terms used)

PatSearch (RUPTO internal), Esp@cenet, PAJ, USPTO, Information Retrieval System of FIPS

**C. DOCUMENTS CONSIDERED TO BE RELEVANT**

| Category* | Citation of document, with indication, where appropriate, of the relevant passages | Relevant to claim No. |
|---|---|---|
| X<br>Y | WO 2010/121140 A1 (FACET BIOTECH CORPORATION et al.)<br>21.10.2010, paragraphs [0010], [0156] - [0168], [0173] | 1, 2, 4-11, 30-34<br>3, 12-29 |
| D, Y | WO 2012/065072 A2 (ABBOTT BIOTECHNOLOGY LTD et al.)<br>18.05.2012, p. 3, lines 2-5 | 3, 12-29 |
| Y | DOSON P. et al. Spravochnik biokhimika. Moscow "Mir" 1991, p. 357 | 3, 12-29 |

☐ Further documents are listed in the continuation of Box C.          ☐ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 10 April 2018 (10.04.2018) | 19 April 2018 (19.04.2018) |

| Name and mailing address of the ISA/ | Authorized officer |
|---|---|
| | |
| Facsimile No. | Telephone No. |

Form PCT/ISA/210 (second sheet) (July 1998)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6090382 A
- WO 9729131 A
- WO 2004016286 A
- WO 2012065072 A
- US 6258562 B
- US 8216583 B
- US 6509015 B
- US 7223394 B
- US 5656272 A
- WO 0212502 A
- US 521206 A
- US 7250165 B
- US 6593458 B
- US 6498237 B
- US 6451983 B
- US 6448380 B
- WO 9103553 A
- WO 09406476 A
- US 5231024 A
- EP 260610 B1
- WO 9216221 A
- WO 9217583 PCT
- WO 9311793 A
- EP 374510 A
- WO 9520978 A, Daum, L.
- WO 9408609 A
- WO 9319751 A
- EP 585705 A
- EP 230574 A
- WO 9104054 A
- EP 453898 A
- WO 9420139 A

**Non-patent literature cited in the description**

- **GRELL, M. et al.** *Cell,* 1995, vol. 83, 793-802
- **KRIEGLER et al.** *Cell,* 1988, vol. 53, 45-53
- **SMITH et al.** *J. Biol. Chem.,* 1987, vol. 262, 6951-6954
- **BUTLER et al.** *Nature,* 1986, vol. 320, 584
- **OLD.** *Science,* 1986, vol. 230, 630
- **CERAMI et al.** *Immunol. Today,* 1988, vol. 9, 28
- **PENNICA, D. et al.** *Nature,* 1984, vol. 312, 724-729
- **DAVIS, J. M. et al.** *Biochemistry,* 1987, vol. 26, 1322-1326
- **JONES, E. Y. et al.** *Nature,* 1989, vol. 338, 225-228
- **KIPRIYANOV, S. M. et al.** *Human Antibodies and Hybridomas,* 1995, vol. 6, 93-101
- **KIPRIYANOV, S. M. et al.** *Mol. Immunol.,* 1994, vol. 31, 1047-1058
- **MOELLER A. et al.** *Cytokine,* 1990, vol. 2, 162-169
- **MOELLER A. ; TRACEY K.J. ; CERAMI A.** *Annu. Rev. Med.,* 1994, vol. 45, 491-503
- **RUSSEL D. ; THOMPSON R.C.** *Curr. Opin. Biotech.,* 1993, vol. 4, 714-721
- **END W.P. ; DAYER J.M.** *ath. Rheum.,* 1995, vol. 38, 151-160
- **FAVA R.A. et al.** *Clin. Exp. Immunol.,* 1993, vol. 94, 261-266
- **ELLIOT M.J. et al.** *Lancet,* 1994, vol. 344, 1125-1127
- **ELLIOT M.J. et al.** *Lancet,* 1994, vol. 344, 1105-1110
- **RANKIN E.C. et al.** *Br. J. Rheumatol.,* 1995, vol. 34, 334-342
- **FIETZE, E. et al.** *Transplantation,* 1994, vol. 58, 675-680
- **EASON J.D. et al.** *Transplantation,* 1995, vol. 59, 300-305
- **SUTHANTHIRAN M. ; STROM T. B.** *New Engl. J. Med.,* 1994, vol. 331, 365-375
- **TRACY K.J. et al.** *Science,* 1986, vol. 234, 470-474
- **SUN X-M. et al.** *J. Clin. Invest.,* 1988, vol. 81, 1328-1331
- **MACDONALD T.T. et al.** *Clin. Exp. Immunol.,* 1990, vol. 81, 301-305
- **VAN DULLEMEN H.M. et al.** *Gastroenterology,* 1995, vol. 109, 129-135
- **BERTOLINI D.R. et al.** *Nature,* 1986, vol. 319, 516-518
- **KONIG A. et al.** *J. Bone Miner. Res.,* 1988, vol. 3, 621-627
- **LERNER U.H. ; OHIIN A.** *J. Bone Miner. Res.,* 1993, vol. 8, 147-155
- **SHANKA G. ; STERN P.H.** *Bone,* 1993, vol. 14, 871-876

- **MCCLAIN C.J. ; COHEN D.A.** *Hepatology,* 1989, vol. 9, 349-351
- **FELVER M.E. et al.** *Alcohol Clin. Exp. Res.,* 1990, vol. 14, 255-259
- **HANSEN J. et al.** *Hepatology,* 1994, vol. 20, 461-474
- **SHERON N. et al.** *J. Hepatol.,* 1991, vol. 12, 241-245
- **HUSSAIN M.J. et al.** *J. Clin. Pathol.,* 1994, vol. 47, 1112-1115
- **VAN DER POLL T. et al.** *N. Engl. J. Med.,* 1990, vol. 322, 1622-1627
- **VAN DER POLL T. et al.** *Prog. Clin. Biol. Res.,* 1991, vol. 367, 55-60
- **GIROIR B.P. et al.** *Am. J. Physiol.,* 1994, vol. 267, H118-124
- **LIU X.S. et al.** *Burns,* 1994, vol. 20, 40-44
- **SCALES W.E. et al.** *Am. J. Physiol.,* 1994, vol. 26, 1122-1127
- **SERRICK C. et al.** *Transplantation,* 1994, vol. 58, 1158-1162
- **YAO Y.M. et al.** *Resuscitation,* 1995, vol. 29, 157-168
- **MCCAULEY R. L et al.** *J. Clin. Immunol.,* 1992, vol. 12, 300-308
- **LANGER.** *Science,* 1990, vol. 249, 1527-1533